# EUROPEAN PATENT SPECIFICATION

(11) **EP 3 443 099 B1**
(45) Date of publication and mention of the grant of the patent: **06.01.2021**
(21) Application number: 17716850.7
(22) Date of filing: 07.04.2017
(51) Int. Cl.: C12N 15/82

(54) **SEED- AND FUNICULUS-PREFERENTIAL PROMOTERS AND USES THEREOF**
PROMOTOREN MIT BEVORZUGUNG VON SAMEN UND SAMENSTIELCHEN UND VERWENDUNGEN DAVON
PROMOTEURS PRÉFÉRENTIELS DES GRAINES ET DU FUNICULE ET LEURS UTILISATIONS

(30) Priority: 13.04.2016 WO PCT/EP2016/165100
(43) Date of publication of application: 20.02.2019
(73) Proprietor: BASF Agricultural Solutions Seed US LLC, Florham Park, NJ 07932 (US)
(72) Inventor: DENOLF, Peter, 9620 Velzeke (BE); VAN AUDENHOVE, Katrien, 9000 Gent (BE); TESKE, John, 9052 Gent (BE)
(74) Representative: BASF IP Association
(86) International application number: PCT/EP2017/058388
(87) International publication number: WO 2017/178367

(56) References cited:
- WO-A2-2008/122980
- US-A1- 2010 281 570
- DATABASE EMBL [Online] 9 October 2002 (2002-10-09), "oei86f11.b1 B.oleracea002 Brassica oleracea genomic, DNA sequence.", XP002770413, retrieved from EBI accession no. EM_GSS:BH988060 Database accession no. BH988060
- TOMOHIRO UEMURA ET AL: "Systematic Analysis of SNARE Molecules in <i>Arabidopsis</i>: Dissection of the post-Golgi Network in Plant Cells", CELL STRUCTURE AND FUNCTION., vol. 29, no. 2, 1 January 2004 (2004-01-01), pages 49-65, XP055375122, JP ISSN: 0386-7196, DOI: 10.1247/csf.29.49
- SANG-JIN KIM ET AL: "News and Views into the SNARE Complexity in Arabidopsis", FRONTIERS IN PLANT SCIENCE, vol. 3, 1 January 2012 (2012-01-01), pages 28/1-28/6, XP055375124, DOI: 10.3389/fpls.2012.00028

## Description

### FIELD OF THE INVENTION

The present invention relates to materials and methods for the expression of a gene of interest preferentially in seeds and in the funiculus of plants. In particular, the invention provides an expression cassette for regulating seed- and funiculus-preferential expression in plants.

### BACKGROUND

Modification of plants to alter and/or improve phenotypic characteristics (such as productivity or quality) requires the overexpression or down-regulation of endogenous genes or the expression of heterologous genes in plant tissues. Such genetic modification relies on the availability of a means to drive and to control gene expression as required. Indeed, genetic modification relies on the availability and use of suitable promoters which are effective in plants and which regulate gene expression so as to give the desired effect(s) in the transgenic plant.

For numerous applications in plant biotechnology a tissue-specific or a tissue-preferential expression profile is advantageous, since beneficial effects of expression in one tissue may have disadvantages in others.

Seed-preferential or seed-specific promoters are useful for expressing or down-regulating genes specifically in the seeds to get the desired function or effect, such as improving disease resistance, herbicide resistance, modifying seed or grain composition or quality, such as modifying starch quality or quantity, modifying oil quality or quantity, modifying amino-acid or protein composition, improving tolerance to biotic or abiotic stress, increasing yield, or altering metabolic pathways in the seeds.

Examples of seed-preferential or seed-specific promoters include the Tonoplast Intrinsic Protein alpha promoter from *Arabidopsis thaliana* (US patent application US2009/0241230), the KNAT411 promoter from *Arabidopsis thaliana* (US Pat. No. 6,342,657), an oleosin promoter, a 2S storage protein promoter or a legumin-like seed storage protein promoter from *Linum usitatissimum* (US Pat. No. 7,642,346), the acyl carrier protein promoter from *Brassica napus* (US Pat. Application No. 1994/0129129), the β-amylase promoter of barley (US Pat. Application No. 1997/0793599), and the Ha ds10 G1 promoter of sunflower (US Pat. No. 6,759,570).

There remains thus an interest in the isolation of novel seed- and funiculus-preferential promoters having moderate promoter activity. It is thus an objective of the present invention to provide *Brassica* promoters having moderate seed- and funiculus-preferential activity. This objective is solved by the present invention as herein further explained.

### SUMMARY

In one aspect, the invention provides an isolated nucleic acid comprising seed- and funiculus-preferential promoter activity selected from the group consisting of (a) a nucleic acid comprising the nucleotide sequence selected from the group: (i) the nucleotide sequence from position 1 to position 1414 of SEQ ID NO: 3, or a functional fragment thereof comprising at least the nucleotide sequence from position 1014 to position 1414 of SEQ ID NO: 3, (ii) the nucleotide sequence from position 1 to position 1364 of SEQ ID NO: 4, or a functional fragment thereof comprising at least the nucleotide sequence from position 964 to position 1364 of SEQ ID NO: 4, (iii) the nucleotide sequence from position 1 to position 1365 of SEQ ID NO: 5, or a functional fragment thereof comprising at least the nucleotide sequence from position 965 to position 1365 of SEQ ID NO: 5, (iv) the nucleotide sequence from position 1 to position 1358 of SEQ ID NO: 6, or a functional fragment thereof comprising at least the nucleotide sequence from position 958 to position 1358 of SEQ ID NO: 6, and (v) the nucleotide sequence from position 1 to position 1363 of SEQ ID NO: 7, or a functional fragment thereof comprising at least the nucleotide sequence from position 963 to position 1363 of SEQ ID NO: 7; and (b) a nucleic acid comprising a nucleotide sequence having at least 80% sequence identity to any one of (a).

In a further embodiment, the nucleic acid described above comprises the nucleotide sequence of SEQ ID NO: 18; the nucleotide sequence of SEQ ID NO: 19; the nucleotide sequence of SEQ ID NO: 20; and the nucleotide sequence of SEQ ID NO: 21.

In another embodiment, said nucleic acid further comprises an intron, the nucleotide sequence of which is selected from the group of: (a) a nucleotide sequence selected from the group: (i) the nucleotide sequence from position 1418 to position 2055 of SEQ ID NO: 3, or a functional fragment thereof, (ii) the nucleotide sequence from position 1368 to position 1650 of SEQ ID NO: 4, or a functional fragment thereof, (iii) the nucleotide sequence from position 1369 to position 2001 of SEQ ID NO: 5, or a functional fragment thereof, (iv) the nucleotide sequence from position 1362 to position 1647 of SEQ ID NO: 6, or a functional fragment thereof, and (v) the nucleotide sequence from position 1367 to position 2001 of SEQ ID NO: 7,or a functional fragment thereof; and (b) a nucleic acid comprising a nucleotide sequence having at least 80% sequence identity to any one of (a), or a functional fragment thereof. The nucleic acid sequence of said intron comprises the nucleotide sequence of SEQ ID NO: 22, the nucleotide sequence of SEQ ID NO: 23, and the nucleotide sequence of SEQ ID NO: 24. In a further embodiment, said nucleic acid comprising the intron has higher seed- and funiculus- preferential promoter activity than the nucleic acid not comprising the intron.

In yet another embodiment the above described nucleic acid is selected from the group: (a) a nucleic acid comprising a nucleotide sequence of any one of SEQ ID NOs: 3 to 7 or a functional fragment thereof; and (b) a nucleic acid comprising a nucleotide sequence having at least 80% sequence identity to any one of SEQ ID NOs: 3 to 7 or a functional fragment thereof.

A further embodiment provides a recombinant gene comprising the nucleic acid according to the invention operably linked to a heterologous nucleic acid sequence encoding an expression product of interest, and optionally a transcription termination and polyadenylation sequence, preferably a transcription termination and polyadenylation region functional in plant cells. In a further embodiment, said expression product of interest is an RNA capable of modulating the expression of a gene or is a protein.

Yet another embodiment provides a host cell or a plant cell, comprising the isolated nucleic acid according to the invention, or the recombinant gene according to the invention.

In further embodiments, a plant and a plant cell are provided comprising the recombinant gene according to the invention.

Yet another embodiment provides a method of producing a transgenic plant comprising the steps of (a) introducing or providing the recombinant gene according to the invention to a plant cell to create transgenic cells; and (b) regenerating transgenic plants from said transgenic cell.

Further provided is a method of effecting seed- and funiculus-preferential expression of a nucleic acid comprising introducing the recombinant gene according to the invention into the genome of a plant, or providing the plant according to the invention. Also provided is a method for altering seed properties of a plant or to produce a commercially relevant product in a plant, said method comprising introducing the recombinant gene according to the invention into the genome of a plant, or providing the plant according to the invention.

Also provided is the use of the isolated nucleic acid according to the invention to regulate expression of an operably linked nucleic acid in a plant, and the use of the isolated nucleic acid according to the invention, or the recombinant gene according to the invention to alter seed properties of a plant or to produce a commercially relevant product in a plant.

Yet another embodiment provides a method of producing food, feed, or an industrial product comprising (a) obtaining the plant or a part thereof, according to the invention; and (b) preparing the food, feed or industrial product from the plant or part thereof. Said food or feed is oil, meal, grain, starch, flour or protein, or said industrial product is biofuel, fiber, industrial chemicals, a pharmaceutical or a nutraceutical.

### BRIEF DESCRIPTION OF THE DRAWINGS

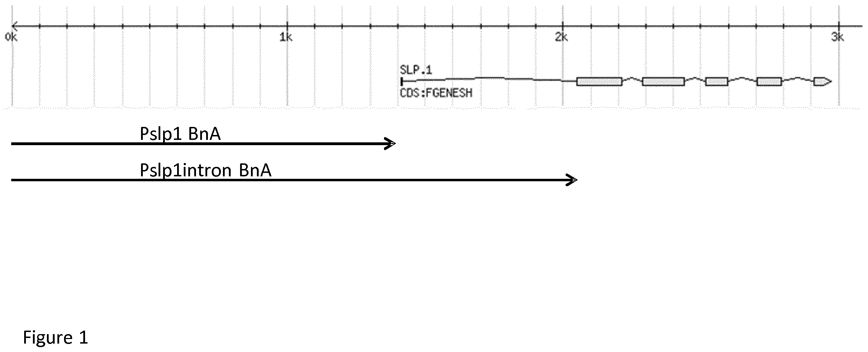

Figure **1**: Graphical display of the gene prediction for SLP1 BnA, using the FGeneSH prediction tool.

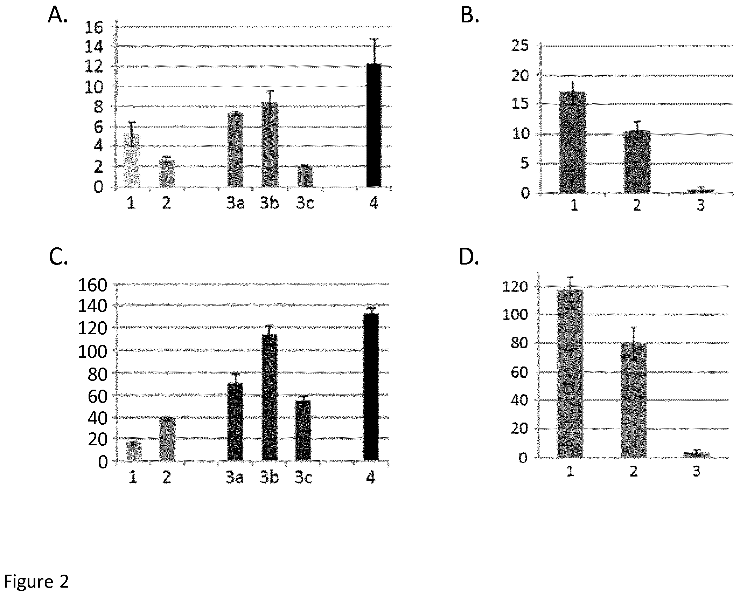

Figure **2**: Semi quantitative assessment of GUS labelling (µU*mg⁻¹ fresh weight*h) in transgenic lines carrying a Pslp1BnA::GUS T-DNA (A and B) or a Pslp1BnAintron::GUS T-DNA (C and D). Embryos (A and C) and seed coats (B and D) were stained at the following stages: 1: 15 to 18 DAF, 2: 20 to 23 DAF, 3: 27 to 32 DAF, 4: 35 to 40 DAF. For the stages 1, 2 and 4 embryos, the stainings are assessed for the whole embryos while for the stage 3 embryos, the staining was quantified in the outer cotyledon (a), in the inner cotyledon (b) and in the hypocotyl (c) separately.

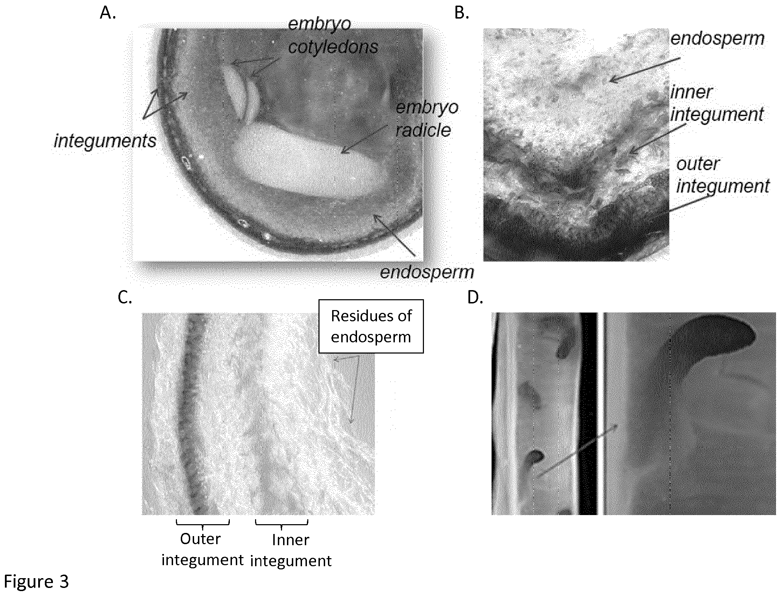

Figure **3**: Expression profile analysis in seeds carrying the T-DNA Pslp1 intron BnA::GUS. GUS labelling in the seed coat and endosperm at early stage (A, B) and at late stage (C), as well as in the funiculus (D). The right panel of D represent a close-up view of one of the funiculi shown in the left panel of D.

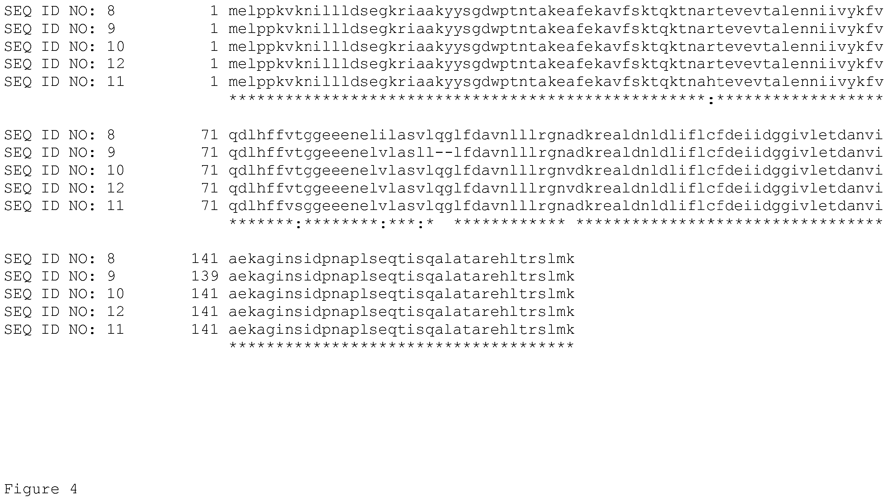

Figure **4**: Alignment of the amino acid sequence of different Brassica SLP1 proteins. Amino acid residues conserved in all proteins are indicated by an asterisk, conserved amino acid substitutions are indicated by a colon. The lowest sequence identity between any two SPL1 protein is about 97%.

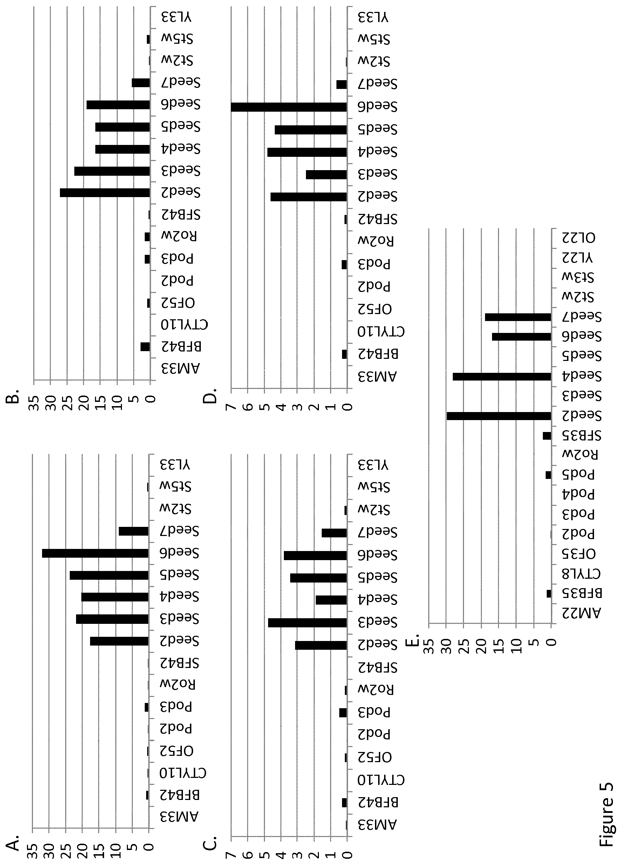

Figure **5**: Relative expression levels of different Brassica SLP1 transcripts in different plant tissues. A: SLP1 BnA; B: SLP1BnC; C: SPL1 Br; D: SLP1 Bo; E: SLP1 BjA. Different tissues for A to D: AM33: Apical meristem 33 days after sowing (DAS); BFB42: Big flower buds 42 DAS; CTYL10: Cotyledons 10 DAS; OF52: Open flowers 52 DAS; Pod2: Pods 14-20 DAS; Pod3: Pods 21-25 DAS; Ro2w: Roots 14 DAS; SFB42: Small flower buds 42 DAS; Seed2: Seeds 14-20 days after flowering (DAF); Seed3: Seeds 21-25 DAF; Seed4: Seeds 26-30 DAF; Seed5: Seeds 31-35 DAF; Seed6: Seeds 42 DAF; Seed7: Seeds 49 DAF; St2w: Stem 14 DAS; St5w: Stem 33 DAS; YL33: Young leaf 33 DAS. Different tissues for E: AM22: Apical meristem 22 days after sowing (DAS); BFB35: Big flower buds 35 DAS; CTYL8: Cotyledons 8 DAS; OF35: Open flowers 35 DAS; Pod2: Pods 14-20 DAS; Pod3: Pods 21-25 DAS; Pod4: Pods 26-30 DAS; Pod5: Pods 31-35 DAS; Ro2w: Roots 14 DAS; SFB35: Small flower buds 35 DAS; Seed2: Seeds 14-20 days after flowering (DAF); Seed3: Seeds 21-25 DAF; Seed4: Seeds 26-30 DAF; Seed5: Seeds 31-35 DAF; Seed6: Seeds 42 DAF; Seed7: Seeds 49 DAF; St2w: Stem 14 DAS; St3w: Stem 22 DAS; YL22: Young leaf 22 DAS; OL22: Old leaf 22 DAS.

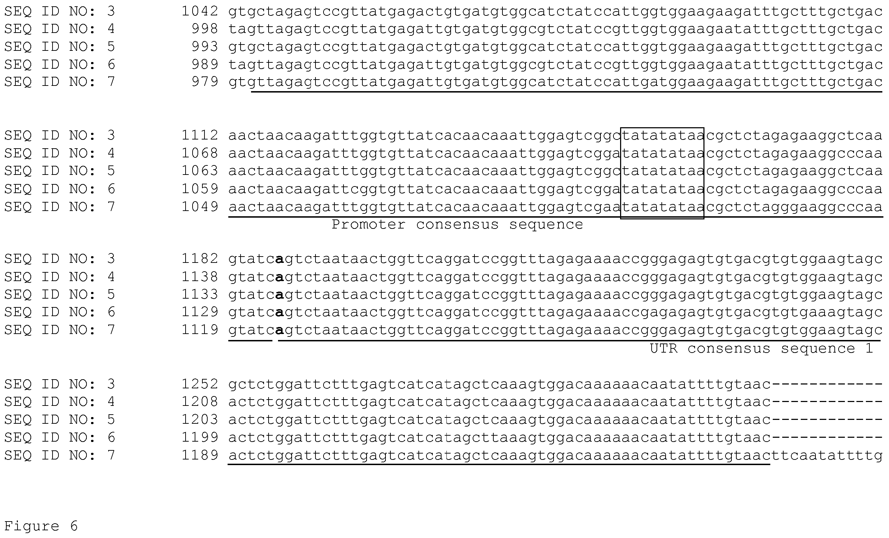

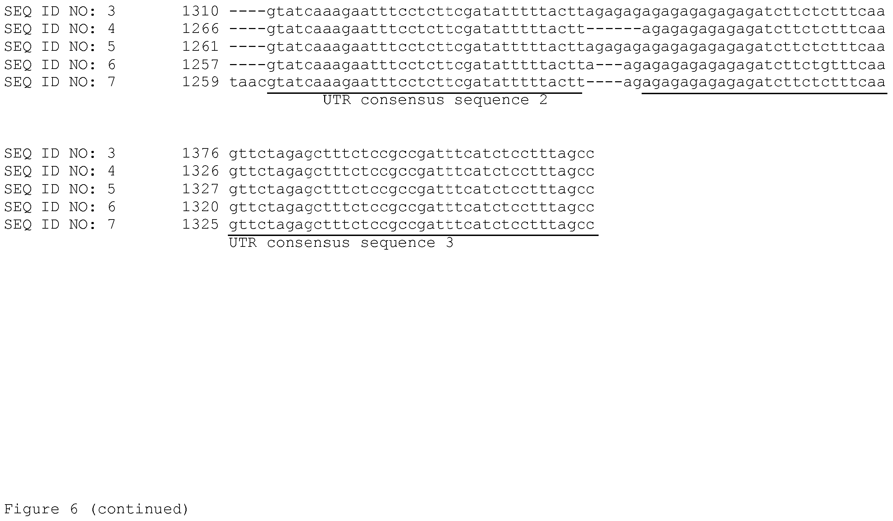

Figure **6**: Alignment of the about 400 nucleotides upstream of the translation start of the nucleotide sequence of the Brassica Pslp1 promoters from *Brassica napus*, *Brassica juncea*, *Brassica oleracea* and *Brassica rapa.* The TATA Box is indicated by a frame. The transcription initiation start is indicated in bold. The consensus sequences are underlined and named. Promoter consensus sequence has the sequence of SEQ ID NO: 18, UTR consensus sequence 1 has the sequence of SEQ ID NO: 19, UTR consensus sequence 2 has the sequence of SEQ ID NO: 20, UTR consensus sequence 3 has the sequence of SEQ ID NO: 21.

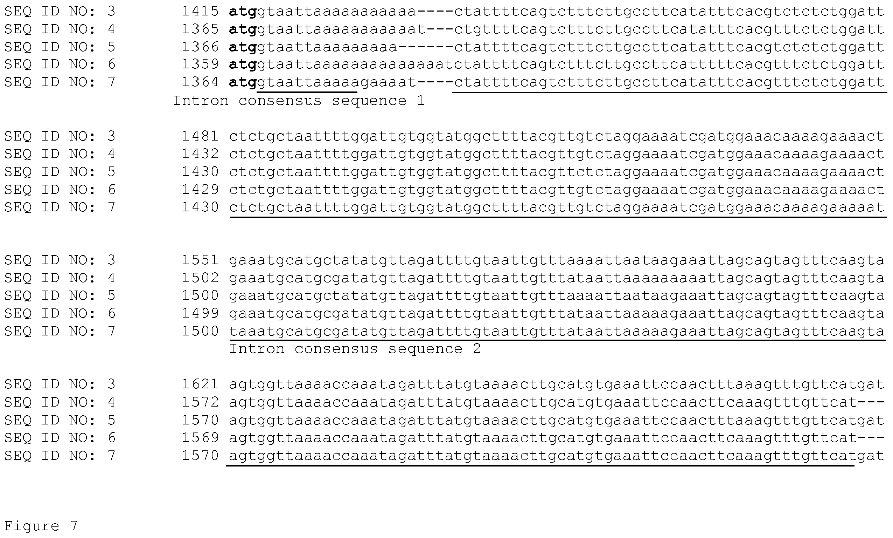

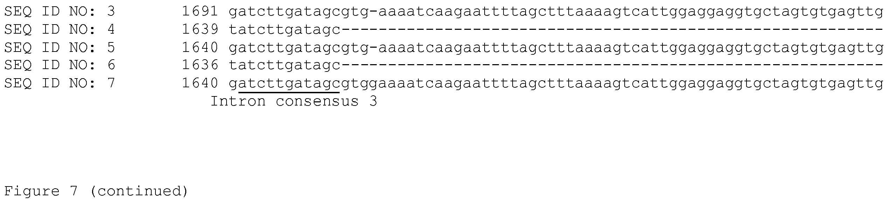

Figure **7**: Alignment of the translation start codons and of the introns of the nucleotide sequence of the Brassica Pslp1 promoters from *Brassica napus*, *Brassica juncea*, *Brassica oleracea* and *Brassica rapa.* The translation start codon is indicated in bold. The consensus sequences are underlined and named. Intron consensus sequence 1 has the sequence of SEQ ID NO: 22, intron consensus sequence 2 has the sequence of SEQ ID NO: 23, intron consensus sequence 3 has the sequence of SEQ ID NO: 24.

### DETAILED DESCRIPTION

The present invention is based on the observation that SEQ ID NOs: 3 to 7 have seed- and funiculus-preferential promoter activity in *Brassica.*

SEQ ID NOs: 3 to 7 depicts the region upstream (i.e. located 5' upstream of) from the first ATG start codon plus the first intron following the ATG of the SLP1 BnA, SLP1 BnC, SLP1 Br, SLP1 Bo, SLP1 BjA genes respectively.

SLP1 BnA and SLP1 BnC are the two copies present in *Brassica napus* of the orthologous gene to the *Arabidopsis thaliana* SLP1 gene At1g60970, which encodes a SNARE-like superfamily protein. SLP1 Br represents the two identical copies present in *Brassica rapa* of the orthologous gene to the *Arabidopsis thaliana* SLP1 gene At1g60970. SLP1 Bo is the only copy present in *Brassica oleracea* of the orthologous gene to the *Arabidopsis thaliana* SLP1 gene At1g60970. SLP1 BjA is one of the two copies present in *Brassica juncea* of the orthologous gene to the *Arabidopsis thaliana* SLP1 gene At1g60970. The SNARE (N-ethylmaleimide-sensitive factor adaptor protein receptor) gene family is a large family of genes encoding membrane associated proteins identified as key players in vesicle trafficking and vesicle fusion. Different members of this family are involved in diverse biological processes in plants, like for example cytokinesis, stress response and shoot gravitropism. Little is known about the expression pattern of the different members of this super family; however, *in silico* expression analysis in *Arabidopsis* indicated that the different members have different profiles, with most being either ubiquitously expressed, or preferentially expressed in the pollen (reviewed in Lipka et al. 2007).

In one aspect, the invention provides an isolated nucleic acid comprising seed- and funiculus-preferential promoter activity selected from the group consisting of (a) a nucleic acid comprising the nucleotide sequence selected from the group: (i) the nucleotide sequence from position 1 to position 1414 of SEQ ID NO: 3, or a functional fragment thereof comprising at least the nucleotide sequence from position 1014 to position 1414 of SEQ ID NO: 3, (ii) the nucleotide sequence from position 1 to position 1364 of SEQ ID NO: 4, or a functional fragment thereof comprising at least the nucleotide sequence from position 964 to position 1364 of SEQ ID NO: 4, (iii) the nucleotide sequence from position 1 to position 1365 of SEQ ID NO: 5, or a functional fragment thereof comprising at least the nucleotide sequence from position 965 to position 1365 of SEQ ID NO: 5, (iv) the nucleotide sequence from position 1 to position 1358 of SEQ ID NO: 6, or a functional fragment thereof comprising at least the nucleotide sequence from position 958 to position 1358 of SEQ ID NO: 6, and (v) the nucleotide sequence from position 1 to position 1363 of SEQ ID NO: 7, or a functional fragment thereof comprising at least the nucleotide sequence from position 963 to position 1363 of SEQ ID NO: 7; and (b) a nucleic acid comprising a nucleotide sequence having at least 80% sequence identity to any one of (a).

In a further embodiment, the nucleic acid described above comprises the nucleotide sequence of SEQ ID NO: 18; the nucleotide sequence of SEQ ID NO: 19; the nucleotide sequence of SEQ ID NO: 20; and the nucleotide sequence of SEQ ID NO: 21.

In another embodiment, said nucleic acid further comprises an intron, the nucleotide sequence of which is selected from the group of: (a) a nucleotide sequence selected from the group: (i) the nucleotide sequence from position 1418 to position 2055 of SEQ ID NO: 3, or a functional fragment thereof, (ii) the nucleotide sequence from position 1368 to position 1650 of SEQ ID NO: 4, or a functional fragment thereof, (iii) the nucleotide sequence from position 1369 to position 2001 of SEQ ID NO: 5, or a functional fragment thereof, (iv) the nucleotide sequence from position 1362 to position 1647 of SEQ ID NO: 6, or a functional fragment thereof, and (v) the nucleotide sequence from position 1367 to position 2001 of SEQ ID NO: 7,or a functional fragment thereof; and (b) a nucleic acid comprising a nucleotide sequence having at least 80% sequence identity to any one of (a), or a functional fragment thereof. The nucleic acid sequence of said intron comprises the nucleotide sequence of SEQ ID NO: 22, the nucleotide sequence of SEQ ID NO: 23, and the nucleotide sequence of SEQ ID NO: 24. In a further embodiment, said nucleic acid comprising the intron has higher seed- and funiculus- preferential promoter activity than the nucleic acid not comprising the intron.

In yet another embodiment the above described nucleic acid is selected from the group: (a) a nucleic acid comprising a nucleotide sequence of any one of SEQ ID NOs: 3 to 7 or a functional fragment thereof; and (b) a nucleic acid comprising a nucleotide sequence having at least 80% sequence identity to any one of SEQ ID NOs: 3 to 7 or a functional fragment thereof.

The nucleic acid comprising the seed- and funiculus-preferential promoter activity according to the invention may also be comprised in a larger DNA molecule.

"Seed- and funiculus-preferential promoter activity" in the context of this invention means the promoter activity is at least 2 times, or at least 5 times, or at least 10 times, or at least 20 times, or at least 50 times, or even at least 100 times higher in seeds and in the funiculus than in other tissues. In other words, in seed- and funiculus-preferential promoter activity, transcription of the nucleic acid operably linked to the promoter of the invention in the seeds and the funiculus is at least 2 times, or at least 5 times, or at least 10 times, or at least 20 times, or at least 50 times or even at least 100 times higher than in other tissues. In other words, the seed- and funiculus-preferential promoter drives seed- and funiculus-preferential expression of the nucleic acid operably linked to the seed- and funiculus-preferential promoter.

The phrase "operably linked" refers to the functional spatial arrangement of two or more nucleic acid regions or nucleic acid sequences. For example, a promoter region may be positioned relative to a nucleic acid sequence such that transcription of a nucleic acid sequence is directed by the promoter region. Thus, a promoter region is "operably linked" to the nucleic acid sequence. "Functionally linked" is an equivalent term.

The phrases "DNA", "DNA sequence," "nucleic acid sequence," "nucleic acid molecule" "nucleotide sequence" and "nucleic acid" refer to a physical structure comprising an orderly arrangement of nucleotides. The DNA sequence or nucleotide sequence may be contained within a larger nucleotide molecule, vector, or the like. In addition, the orderly arrangement of nucleic acids in these sequences may be depicted in the form of a sequence listing, figure, table, electronic medium, or the like.

As used herein, "promoter" means a region of DNA sequence that is essential for the initiation of transcription of DNA, resulting in the generation of an RNA molecule that is complementary to the transcribed DNA; this region may also be referred to as a "5' regulatory region." Promoters are usually located upstream of the coding sequence to be transcribed and have regions that act as binding sites for RNA polymerase II and other proteins such as transcription factors (trans-acting protein factors that regulate transcription) to initiate transcription of an operably linked gene. Promoters may themselves contain sub-elements (i.e. promoter motifs) such as cis-elements or enhancer domains that regulate the transcription of operably linked genes. The promoters of this invention may be altered to contain "enhancer DNA" to assist in elevating gene expression. As is known in the art, certain DNA elements can be used to enhance the transcription of DNA. These enhancers often are found 5' to the start of transcription in a promoter that functions in eukaryotic cells, but can often be inserted upstream (5') or downstream (3') to the coding sequence. In some instances, these 5' enhancer DNA elements are introns. Among the introns that are useful as enhancer DNA are the 5' introns from the rice actin 1 gene (see US5641876), the rice actin 2 gene, the maize alcohol dehydrogenase gene, the maize heat shock protein 70 gene (see US5593874), the maize shrunken 1 gene, the light sensitive 1 gene of *Solanum tuberosum,* the Arabidopsis histon 4 intron and the heat shock protein 70 gene of *Petunia hybrida* (see US5659122). Thus, as contemplated herein, a promoter or promoter region includes variations of promoters derived by inserting or deleting regulatory regions, subjecting the promoter to random or site-directed mutagenesis, etc. The activity or strength of a promoter may be measured in terms of the amounts of RNA it produces, or the amount of protein accumulation in a cell or tissue, relative to a promoter whose transcriptional activity has been previously assessed or relative to a promoter driving the expression of a housekeeping gene.

A promoter as used herein may thus include sequences downstream of the transcription start, such as sequences coding the 5' untranslated region (5' UTR) of the RNA, introns located downstream of the transcription start, or even sequences encoding the protein. A functional promoter fragment according to the invention may comprise its own 5'UTR comprising the nucleotide sequence of SEQ ID NO: 3 from nucleotide 1187 to nucleotide 1414, or comprising the nucleotide sequence of SEQ ID No: 4 from nucleotide 1143 to nucleotide 1364, or comprising the nucleotide sequence of SEQ ID No: 5 from nucleotide 1138 to nucleotide 1365, or comprising the nucleotide sequence of SEQ ID No: 6 from nucleotide 1134 to nucleotide 1358, or comprising the nucleotide sequence of SEQ ID No: 7 from nucleotide 1124 to nucleotide 1363. Alternatively, 5'UTR fragments from other Brassica SLP1 genes may be used. For example, a promoter fragment of SEQ ID NO: 3 may have the nucleotide sequence of said sequence from position 1187 to 1414 replaced by the nucleotide sequence of SEQ ID NO: 4 from nucleotide 1143 to nucleotide 1364. A promoter fragment of SEQ ID NO: 3 may have the nucleotide sequence of said sequence from position 1187 to 1414 replaced by the nucleotide sequence of SEQ ID NO: 5 from nucleotide 1138 to nucleotide 1365. A promoter fragment of SEQ ID NO: 3 may have the nucleotide sequence of said sequence from position 1187 to 1414 replaced by the nucleotide sequence of SEQ ID NO: 6 from nucleotide 1134 to nucleotide 1358. A promoter fragment of SEQ ID NO: 3 may have the nucleotide sequence of said sequence from position 1187 to 1414 replaced by the nucleotide sequence of SEQ ID NO: 7 from nucleotide 1124 to nucleotide 1363. As another example, a promoter fragment of SEQ ID NO: 4 may have the nucleotide sequence of said sequence from position 1143 to position 1364 replaced by the nucleotide sequence of SEQ ID NO: 3 from nucleotide 1187 to nucleotide 1414. A promoter fragment of SEQ ID NO: 4 may have the nucleotide sequence of said sequence from position 1143 to position 1364 replaced by the nucleotide sequence of SEQ ID NO: 5 from nucleotide 1138 to nucleotide 1365. A promoter fragment of SEQ ID NO: 4 may have the nucleotide sequence of said sequence from position 1143 to position 1364 replaced by the nucleotide sequence of SEQ ID NO: 6 from nucleotide 1134 to nucleotide 1358. A promoter fragment of SEQ ID NO: 4 may have the nucleotide sequence of said sequence from position 1143 to position 1364 replaced by the nucleotide sequence of SEQ ID NO: 7 from nucleotide 1124 to nucleotide 1363. As yet another example, a promoter fragment of SEQ ID NO: 5 may have the nucleotide sequence of said sequence from position 1138 to position 1365 replaced by the nucleotide sequence of SEQ ID NO: 3 from nucleotide 1187 to nucleotide 1414. A promoter fragment of SEQ ID NO: 5 may have the nucleotide sequence of said sequence from position 1138 to position 1365 replaced by the nucleotide sequence of SEQ ID NO: 4 from nucleotide 1143 to nucleotide 1364. A promoter fragment of SEQ ID NO: 5 may have the nucleotide sequence of said sequence from position 1138 to position 1365 replaced by the nucleotide sequence of SEQ ID NO: 6 from nucleotide 1134 to nucleotide 1358. A promoter fragment of SEQ ID NO: 5 may have the nucleotide sequence of said sequence from position 1138 to position 1365 replaced by the nucleotide sequence of SEQ ID NO: 7 from nucleotide 1124 to nucleotide 1363. As another example, a promoter fragment of SEQ ID NO: 6 may have the nucleotide sequence of said sequence from position 1134 to position 1358 replaced by the nucleotide sequence of SEQ ID NO: 3 from nucleotide 1187 to nucleotide 1414. A promoter fragment of SEQ ID NO: 6 may have the nucleotide sequence of said sequence from position 1138 to position 1365 replaced by the nucleotide sequence of SEQ ID NO: 4 from nucleotide 1143 to nucleotide 1364. A promoter fragment of SEQ ID NO: 6 may have the nucleotide sequence of said sequence from position 1138 to position 1365 replaced by the nucleotide sequence of SEQ ID NO: 5 from nucleotide 1138 to nucleotide 1365. A promoter fragment of SEQ ID NO: 6 may have the nucleotide sequence of said sequence from position 1138 to position 1365 replaced by the nucleotide sequence of SEQ ID NO: 7 from nucleotide 1124 to nucleotide 1363. As another example, a promoter fragment of SEQ ID NO: 7 may have the nucleotide sequence of said sequence from position 1124 to position 1363 replaced by the nucleotide sequence of SEQ ID NO: 3 from nucleotide 1187 to nucleotide 1414. A promoter fragment of SEQ ID NO: 7 may have the nucleotide sequence of said sequence from position 1124 to position 1363 replaced by the nucleotide sequence of SEQ ID NO: 4 from nucleotide 1143 to nucleotide 1364. A promoter fragment of SEQ ID NO: 7 may have the nucleotide sequence of said sequence from position 1124 to position 1363 replaced by the nucleotide sequence of SEQ ID NO: 5 from nucleotide 1138 to nucleotide 1365. A promoter fragment of SEQ ID NO: 7 may have the nucleotide sequence of said sequence from position 1124 to position 1363 replaced by the nucleotide sequence of SEQ ID NO: 6 from nucleotide 1134 to nucleotide 1358.

A promoter fragment according to the invention may comprise its own intron comprising the nucleotide sequence of SEQ ID NO: 3 from nucleotide 1418 to nucleotide 2055, or comprising the nucleotide sequence of SEQ ID No: 4 from nucleotide 1368 to nucleotide 1650, or comprising the nucleotide sequence of SEQ ID No: 5 from nucleotide 1369 to nucleotide 2001, or comprising the nucleotide sequence of SEQ ID No: 6 from nucleotide 1362 to nucleotide 1647, or comprising the nucleotide sequence of SEQ ID No: 7 from nucleotide 1367 to nucleotide 2001. Alternatively, intron fragments from other Brassica SLP1 genes may be used. For example, a promoter fragment of SEQ ID NO: 3 may have the nucleotide sequence of said sequence from position 1418 to position 2055 replaced by the nucleotide sequence of SEQ ID NO: 4 from nucleotide 1368 to nucleotide 1650. A promoter fragment of SEQ ID NO: 3 may have the nucleotide sequence of said sequence from position 1418 to position 2055 replaced by the nucleotide sequence of SEQ ID NO: 5 from nucleotide 1369 to nucleotide 2001. A promoter fragment of SEQ ID NO: 3 may have the nucleotide sequence of said sequence from position 1418 to position 2055 replaced by the nucleotide sequence of SEQ ID NO: 6 from nucleotide 1362 to nucleotide 1647. A promoter fragment of SEQ ID NO: 3 may have the nucleotide sequence of said sequence from position 1418 to position 2055 replaced by the nucleotide sequence of SEQ ID NO: 7 from nucleotide 1367 to nucleotide 2001. As another example, a promoter fragment of SEQ ID NO: 4 may have the nucleotide sequence of said sequence from position 1368 to position 1650 replaced by the nucleotide sequence of SEQ ID NO: 3 from nucleotide 1418 to nucleotide 2055. A promoter fragment of SEQ ID NO: 4 may have the nucleotide sequence of said sequence from position 1368 to position 1650 replaced by the nucleotide sequence of SEQ ID NO: 5 from nucleotide 1369 to nucleotide 2001. A promoter fragment of SEQ ID NO: 4 may have the nucleotide sequence of said sequence from position 1368 to position 1650 replaced by the nucleotide sequence of SEQ ID NO: 6 from nucleotide 1362 to nucleotide 1647. A promoter fragment of SEQ ID NO: 4 may have the nucleotide sequence of said sequence from position 1368 to position 1650 replaced by the nucleotide sequence of SEQ ID NO: 7 from nucleotide 1367 to nucleotide 2001. As yet another example, a promoter fragment of SEQ ID NO: 5 may have the nucleotide sequence of said sequence from position 1369 to position 2001 replaced by the nucleotide sequence of SEQ ID NO: 3 from nucleotide 1418 to nucleotide 2055. A promoter fragment of SEQ ID NO: 5 may have the nucleotide sequence of said sequence from position 1369 to position 2001 replaced by the nucleotide sequence of SEQ ID NO: 4 from nucleotide 1368 to nucleotide 1650. A promoter fragment of SEQ ID NO: 5 may have the nucleotide sequence of said sequence from position 1369 to position 2001 replaced by the nucleotide sequence of SEQ ID NO: 6 from nucleotide 1362 to nucleotide 1647. A promoter fragment of SEQ ID NO: 5 may have the nucleotide sequence of said sequence from position 1369 to position 2001 replaced by the nucleotide sequence of SEQ ID NO: 7 from nucleotide 1367 to nucleotide 2001. As another example, a promoter fragment of SEQ ID NO: 6 may have the nucleotide sequence of said sequence from position 1362 to position 1647 replaced by the nucleotide sequence of SEQ ID NO: 3 from nucleotide 1418 to nucleotide 2055. A promoter fragment of SEQ ID NO: 6 may have the nucleotide sequence of said sequence from position 1362 to position 1647 replaced by the nucleotide sequence of SEQ ID NO: 4 from nucleotide 1368 to nucleotide 1650. A promoter fragment of SEQ ID NO: 6 may have the nucleotide sequence of said sequence from position 1362 to position 1647 replaced by the nucleotide sequence of SEQ ID NO: 5 from nucleotide 1369 to nucleotide 2001. A promoter fragment of SEQ ID NO: 6 may have the nucleotide sequence of said sequence from position 1362 to position 1647 replaced by the nucleotide sequence of SEQ ID NO: 7 from nucleotide 1367 to nucleotide 2001. As another example, a promoter fragment of SEQ ID NO: 7 may have the nucleotide sequence of said sequence from position 1367 to position 2001 replaced by the nucleotide sequence of SEQ ID NO: 3 from nucleotide 1418 to nucleotide 2055. A promoter fragment of SEQ ID NO: 7 may have the nucleotide sequence of said sequence from position 1367 to position 2001 replaced by the nucleotide sequence of SEQ ID NO: 4 from nucleotide 1368 to nucleotide 1650. A promoter fragment of SEQ ID NO: 7 may have the nucleotide sequence of said sequence from position 1367 to position 2001 replaced by the nucleotide sequence of SEQ ID NO: 5 from nucleotide 1369 to nucleotide 2001. A promoter fragment of SEQ ID NO: 7 may have the nucleotide sequence of said sequence from position 1367 to position 2001 replaced by the nucleotide sequence of SEQ ID NO: 6 from nucleotide 1362 to nucleotide 1647.

Such a promoter fragment may be at least about 400 bp, at least about 500 bp, at least about 600 bp, at least about 700 bp, at least about 800 bp, at least about 900 bp, at least about 1000 bp, at least about 1100 bp, at least about 1200 bp, or at least about 1300 bp upstream of the first ATG start codon of the SLP1 transcripts and have seed- and funiculus-preferential promoter activity. In combination with the above described promoter fragments, a promoter fragment according to the invention may thus comprise the nucleotide sequence of SEQ ID NO: 3 from the nucleotide at position 1014 to the nucleotide at position 1414, the nucleotide sequence of SEQ ID NO: 3 from the nucleotide at position 914 to the nucleotide at position 1414, the nucleotide sequence of SEQ ID NO: 3 from the nucleotide at position 814 to the nucleotide at position 1414, the nucleotide sequence of SEQ ID NO: 3 from the nucleotide at position 714 to the nucleotide at position 1414, the nucleotide sequence of SEQ ID NO: 3 from the nucleotide at position 614 to the nucleotide at position 1414, the nucleotide sequence of SEQ ID NO: 3 from the nucleotide at position 514 to the nucleotide at position 1414, the nucleotide sequence of SEQ ID NO: 3 from the nucleotide at position 414 to the nucleotide at position 1414, the nucleotide sequence of SEQ ID NO: 3 from the nucleotide at position 314 to the nucleotide at position 1414, the nucleotide sequence of SEQ ID NO: 3 from the nucleotide at position 214 to the nucleotide at position 1414, the nucleotide sequence of SEQ ID NO: 3 from the nucleotide at position 114 to the nucleotide at position 1414, the nucleotide sequence of SEQ ID NO: 3 from the nucleotide at position 14 to the nucleotide at position 1414, or the nucleotide sequence of SEQ ID NO: 3 from the nucleotide at position 1 to the nucleotide at position 1414. A promoter fragment according to the invention may also comprise the nucleotide sequence of SEQ ID No: 4 from the nucleotide at position 964 to the nucleotide at position 1364, the nucleotide sequence of SEQ ID No: 4 from the nucleotide at position 864 to the nucleotide at position 1364, the nucleotide sequence of SEQ ID No: 4 from the nucleotide at position 764 to the nucleotide at position 1364, the nucleotide sequence of SEQ ID No: 4 from the nucleotide at position 664 to the nucleotide at position 1364, the nucleotide sequence of SEQ ID No: 4 from the nucleotide at position 564 to the nucleotide at position 1364, the nucleotide sequence of SEQ ID No: 4 from the nucleotide at position 464 to the nucleotide at position 1364, the nucleotide sequence of SEQ ID No: 4 from the nucleotide at position 364 to the nucleotide at position 1364, the nucleotide sequence of SEQ ID No: 4 from the nucleotide at position 264 to the nucleotide at position 1364, the nucleotide sequence of SEQ ID No: 4 from the nucleotide at position 164 to the nucleotide at position 1364, the nucleotide sequence of SEQ ID No: 4 from the nucleotide at position 64 to the nucleotide at position 1364, or the nucleotide sequence of SEQ ID No: 4 from the nucleotide at position 1 to the nucleotide at position 1364. A promoter fragment according to the invention may also comprise the nucleotide sequence of SEQ ID No: 5 from the nucleotide at position 965 to the nucleotide at position 1365, the nucleotide sequence of SEQ ID No: 5 from the nucleotide at position 865 to the nucleotide at position 1365, the nucleotide sequence of SEQ ID No: 5 from the nucleotide at position 765 to the nucleotide at position 1365, the nucleotide sequence of SEQ ID No: 5 from the nucleotide at position 665 to the nucleotide at position 1365, the nucleotide sequence of SEQ ID No: 5 from the nucleotide at position 565 to the nucleotide at position 1365, the nucleotide sequence of SEQ ID No: 5 from the nucleotide at position 465 to the nucleotide at position 1365, the nucleotide sequence of SEQ ID No: 5 from the nucleotide at position 365 to the nucleotide at position 1365, the nucleotide sequence of SEQ ID No: 5 from the nucleotide at position 265 to the nucleotide at position 1365, the nucleotide sequence of SEQ ID No: 5 from the nucleotide at position 165 to the nucleotide at position 1365, the nucleotide sequence of SEQ ID No: 5 from the nucleotide at position 65 to the nucleotide at position 1365, or the nucleotide sequence of SEQ ID No: 5 from the nucleotide at position 1 to the nucleotide at position 1365. A promoter fragment according to the invention may also comprise the nucleotide sequence of SEQ ID No: 6 from the nucleotide at position 958 to the nucleotide at position 1358, the nucleotide sequence of SEQ ID No: 6 from the nucleotide at position 858 to the nucleotide at position 1358, the nucleotide sequence of SEQ ID No: 6 from the nucleotide at position 758 to the nucleotide at position 1358, the nucleotide sequence of SEQ ID No: 6 from the nucleotide at position 658 to the nucleotide at position 1358, the nucleotide sequence of SEQ ID No: 6 from the nucleotide at position 558 to the nucleotide at position 1358, the nucleotide sequence of SEQ ID No: 6 from the nucleotide at position 458 to the nucleotide at position 1358, the nucleotide sequence of SEQ ID No: 6 from the nucleotide at position 358 to the nucleotide at position 1358, the nucleotide sequence of SEQ ID No: 6 from the nucleotide at position 258 to the nucleotide at position 1358, the nucleotide sequence of SEQ ID No: 6 from the nucleotide at position 158 to the nucleotide at position 1358, the nucleotide sequence of SEQ ID No: 6 from the nucleotide at position 58 to the nucleotide at position 1358, or the nucleotide sequence of SEQ ID No: 6 from the nucleotide at position 1 to the nucleotide at position 1358. A promoter fragment according to the invention may also comprise the nucleotide sequence of SEQ ID No: 7 from the nucleotide at position 963 to the nucleotide at position 1363, the nucleotide sequence of SEQ ID No: 7 from the nucleotide at position 863 to the nucleotide at position 1363, the nucleotide sequence of SEQ ID No: 7 from the nucleotide at position 763 to the nucleotide at position 1363, the nucleotide sequence of SEQ ID No: 7 from the nucleotide at position 663 to the nucleotide at position 1363, the nucleotide sequence of SEQ ID No: 7 from the nucleotide at position 563 to the nucleotide at position 1363, the nucleotide sequence of SEQ ID No: 7 from the nucleotide at position 463 to the nucleotide at position 1363, the nucleotide sequence of SEQ ID No: 7 from the nucleotide at position 363 to the nucleotide at position 1363, the nucleotide sequence of SEQ ID No: 7 from the nucleotide at position 263 to the nucleotide at position 1363, the nucleotide sequence of SEQ ID No: 7 from the nucleotide at position 163 to the nucleotide at position 1363, the nucleotide sequence of SEQ ID No: 7 from the nucleotide at position 63 to the nucleotide at position 1363, or the nucleotide sequence of SEQ ID No: 7 from the nucleotide at position 1 to the nucleotide at position 1363.

Such a promoter fragment may be at least about 400 bp, at least about 500 bp, at least about 600 bp, at least about 700 bp, at least about 800 bp, at least about 900 bp, at least about 1000 bp, at least about 1100 bp, at least about 1200 bp, or at least about 1300 bp upstream of the first ATG start codon of the SLP1 transcripts and at least about 300 bp, at least about 400 bp, at least about 500 bp or at least about 600 bp of the intron downstream of the first ATG start codon of the SLP1 gene and have seed- and funiculus-preferential promoter activity. In such promoter fragments the first ATG start codon may optionally be present immediately before the intron fragment. In combination with the above described promoter fragments, a promoter fragment according to the invention may thus comprise the nucleotide sequence of SEQ ID NO: 3 from the nucleotide at position 1014 to the nucleotide at position 1414 and from the nucleotide position 1418 to the nucleotide position 1718, the nucleotide sequence of SEQ ID NO: 3 from the nucleotide at position 914 to the nucleotide at position 1414 and from the nucleotide at position 1418 to the nucleotide at position 1718, the nucleotide sequence of SEQ ID NO: 3 from the nucleotide at position 814 to the nucleotide at position 1414 and from the nucleotide at position 1418 to the nucleotide at position 1718, the nucleotide sequence of SEQ ID NO: 3 from the nucleotide at position 714 to the nucleotide at position 1414 and from the nucleotide at position 1418 to the nucleotide at position 1718, the nucleotide sequence of SEQ ID NO: 3 from the nucleotide at position 614 to the nucleotide at position 1414 and from the nucleotide at position 1418 to the nucleotide at position 1718, the nucleotide sequence of SEQ ID NO: 3 from the nucleotide at position 514 to the nucleotide at position 1414 and from the nucleotide at position 1418 to the nucleotide at position 1718, the nucleotide sequence of SEQ ID NO: 3 from the nucleotide at position 414 to the nucleotide at position 1414 and from the nucleotide at position 1418 to the nucleotide at position 1718, the nucleotide sequence of SEQ ID NO: 3 from the nucleotide at position 314 to the nucleotide at position 1414 and from the nucleotide at position 1418 to the nucleotide at position 1718, the nucleotide sequence of SEQ ID NO: 3 from the nucleotide at position 214 to the nucleotide at position 1414 and from the nucleotide at position 1418 to the nucleotide at the position 1718, the nucleotide sequence of SEQ ID NO: 3 from the nucleotide at position 114 to the nucleotide at position 1414 and from the nucleotide at position 1418 to the nucleotide at position 1718, the nucleotide sequence of SEQ ID NO: 3 from the nucleotide at position 14 to the nucleotide at position 1414 and from the nucleotide at position 1418 to the nucleotide at position 1718, the nucleotide sequence of SEQ ID NO: 3 from the nucleotide at position 1 to the nucleotide at position 1414 and from the nucleotide at position 1418 to the nucleotide at position 1718, the nucleotide sequence of SEQ ID NO: 3 from the nucleotide at position 1014 to the nucleotide at position 1414 and from the nucleotide at position 1418 to the nucleotide at position 1818, the nucleotide sequence of SEQ ID NO: 3 from the nucleotide at position 914 to the nucleotide at position 1414 and from the nucleotide at position 1418 to the nucleotide at position 1818, the nucleotide sequence of SEQ ID NO: 3 from the nucleotide at position 814 to the nucleotide at position 1414 and from the nucleotide at position 1418 to the nucleotide at position 1818, the nucleotide sequence of SEQ ID NO: 3 from the nucleotide at position 714 to the nucleotide at position 1414 and from the nucleotide at position 1418 to the nucleotide at position 1818, the nucleotide sequence of SEQ ID NO: 3 from the nucleotide at position 614 to the nucleotide at position 1414 and from the nucleotide at position 1418 to the nucleotide at position 1818, the nucleotide sequence of SEQ ID NO: 3 from the nucleotide at position 514 to the nucleotide at position 1414 and from the nucleotide at position 1418 to the nucleotide at position 1818, the nucleotide sequence of SEQ ID NO: 3 from the nucleotide at position 414 to the nucleotide at position 1414 and from the nucleotide at position 1418 to the nucleotide at position 1818, the nucleotide sequence of SEQ ID NO: 3 from the nucleotide at position 314 to the nucleotide at position 1414 and from the nucleotide at position 1418 to the nucleotide at position 1818, the nucleotide sequence of SEQ ID NO: 3 from the nucleotide at position 214 to the nucleotide at position 1414 and from the nucleotide at position 1418 to the nucleotide at position 1818, the nucleotide sequence of SEQ ID NO: 3 from the nucleotide at position 114 to the nucleotide at position 1414 and from the nucleotide at position 1418 to the nucleotide at position 1818, the nucleotide sequence of SEQ ID NO: 3 from the nucleotide at position 14 to the nucleotide at position 1414 and from the nucleotide at position 1418 to the nucleotide at position 1818, the nucleotide sequence of SEQ ID NO: 3 from the nucleotide at position 1 to the nucleotide at position 1414 and from the nucleotide at position 1418 to the nucleotide at position 1818, the nucleotide sequence of SEQ ID NO: 3 from the nucleotide at position 1014 to the nucleotide at position 1414 and from the nucleotide at position 1418 to the nucleotide at position 1918, the nucleotide sequence of SEQ ID NO: 3 from the nucleotide at position 914 to the nucleotide at position 1414 and from the nucleotide at position 1418 to the nucleotide at position 1918, the nucleotide sequence of SEQ ID NO: 3 from the nucleotide at position 814 to the nucleotide at position 1414 and from the nucleotide at position 1418 to the nucleotide at position 1918, the nucleotide sequence of SEQ ID NO: 3 from the nucleotide at position 714 to the nucleotide at position 1414 and from the nucleotide at position 1418 to the nucleotide at position 1918, the nucleotide sequence of SEQ ID NO: 3 from the nucleotide at position 614 to the nucleotide at position 1414 and from the nucleotide at position 1418 to the nucleotide at position 1918, the nucleotide sequence of SEQ ID NO: 3 from the nucleotide at position 514 to the nucleotide at position 1414 and from the nucleotide at position 1418 to the nucleotide at position 1918, the nucleotide sequence of SEQ ID NO: 3 from the nucleotide at position 414 to the nucleotide at position 1414 and from the nucleotide at position 1418 to the nucleotide at position 1918, the nucleotide sequence of SEQ ID NO: 3 from the nucleotide at position 314 to the nucleotide at position 1414 and from the nucleotide at position 1418 to the nucleotide at position 1918, the nucleotide sequence of SEQ ID NO: 3 from the nucleotide at position 214 to the nucleotide at position 1414 and from the nucleotide at position 1418 to the nucleotide at position 1918, the nucleotide sequence of SEQ ID NO: 3 from the nucleotide at position 114 to the nucleotide at position 1414 and from the nucleotide at position 1418 to the nucleotide at position 1918, the nucleotide sequence of SEQ ID NO: 3 from the nucleotide at position 14 to the nucleotide at position 1414 and from the nucleotide at position 1418 to the nucleotide at position 1918, the nucleotide sequence of SEQ ID NO: 3 from the nucleotide at position 1 to the nucleotide at position 1414 and from the nucleotide at position 1418 to the nucleotide at position 1918, the nucleotide sequence of SEQ ID NO: 3 from the nucleotide at position 1014 to the nucleotide at position 1414 and from the nucleotide at position 1418 to the nucleotide at position 2018, the nucleotide sequence of SEQ ID NO: 3 from the nucleotide at position 914 to the nucleotide at position 1414 and from the nucleotide at position 1418 to the nucleotide at position 2018, the nucleotide sequence of SEQ ID NO: 3 from the nucleotide at position 814 to the nucleotide at position 1414 and from the nucleotide at position 1418 to the nucleotide at position 2018, the nucleotide sequence of SEQ ID NO: 3 from the nucleotide at position 714 to the nucleotide at position 1414 and from the nucleotide at position 1418 to the nucleotide at position 2018, the nucleotide sequence of SEQ ID NO: 3 from the nucleotide at position 614 to the nucleotide at position 1414 and from the nucleotide at position 1418 to the nucleotide at position 2018, the nucleotide sequence of SEQ ID NO: 3 from the nucleotide at position 514 to the nucleotide at position 1414 and from the nucleotide at position 1418 to the nucleotide at position 2018, the nucleotide sequence of SEQ ID NO: 3 from the nucleotide at position 414 to the nucleotide at position 1414 and from the nucleotide at position 1418 to the nucleotide at position 2018, the nucleotide sequence of SEQ ID NO: 3 from the nucleotide at position 314 to the nucleotide at position 1414 and from the nucleotide at position 1418 to the nucleotide at position 2018, the nucleotide sequence of SEQ ID NO: 3 from the nucleotide at position 214 to the nucleotide at position 1414 and from the nucleotide at position 1418 to the nucleotide at position 2018, the nucleotide sequence of SEQ ID NO: 3 from the nucleotide at position 114 to the nucleotide at position 1414 and from the nucleotide at position 1418 to the nucleotide at position 2018, the nucleotide sequence of SEQ ID NO: 3 from the nucleotide at position 14 to the nucleotide at position 1414 and from the nucleotide at position 1418 to the nucleotide at position 2018, the nucleotide sequence of SEQ ID NO: 3 from the nucleotide at position 1 to the nucleotide at position 1414 and from the nucleotide at position 1418 to the nucleotide at position 2018, the nucleotide sequence of SEQ ID NO: 3 from the nucleotide at position 1014 to the nucleotide at position 1414 and from the nucleotide at position 1418 to the nucleotide at position 2055, the nucleotide sequence of SEQ ID NO: 3 from the nucleotide at position 914 to the nucleotide at position 1414 and from the nucleotide at the position 1418 to the nucleotide at position 2055, the nucleotide sequence of SEQ ID NO: 3 from the nucleotide at position 814 to the nucleotide at position 1414 and from the nucleotide at position 1418 to the nucleotide at position 2055, the nucleotide sequence of SEQ ID NO: 3 from the nucleotide at position 714 to the nucleotide at position 1414 and from the nucleotide at position 1418 to the nucleotide at position 2055, the nucleotide sequence of SEQ ID NO: 3 from the nucleotide at position 614 to the nucleotide at position 1414 and from the nucleotide at position 1418 to the nucleotide at position 2055, the nucleotide sequence of SEQ ID NO: 3 from the nucleotide at position 514 to the nucleotide at position 1414 and from the nucleotide at position 1418 to the nucleotide at position 2055, the nucleotide sequence of SEQ ID NO: 3 from the nucleotide at position 414 to the nucleotide at position 1414 and from the nucleotide at position 1418 to the nucleotide at position 2055, the nucleotide sequence of SEQ ID NO: 3 from the nucleotide at position 314 to the nucleotide at position 1414 and from the nucleotide at position 1418 to the nucleotide at position 2055, the nucleotide sequence of SEQ ID NO: 3 from the nucleotide at position 214 to the nucleotide at position 1414 and from the nucleotide at position 1418 to the nucleotide at position 2055, the nucleotide sequence of SEQ ID NO: 3 from the nucleotide at position 114 to the nucleotide at position 1414 and from the nucleotide at position 1418 to the nucleotide at position 2055, the nucleotide sequence of SEQ ID NO: 3 from the nucleotide at position 14 to the nucleotide at position 1414 and from the nucleotide at position 1418 to the nucleotide at position 2055, or the nucleotide sequence of SEQ ID NO: 3 from the nucleotide at position 1 to the nucleotide at position 1414 and from the nucleotide at position 1418 to the nucleotide at position 2055. A promoter fragment according to the invention may also comprise the nucleotide sequence of SEQ ID No: 4 from the nucleotide at position 964 to the nucleotide at position 1364 and from the nucleotide at position 1368 to the nucleotide at position 1650, the nucleotide sequence of SEQ ID No: 4 from the nucleotide at position 864 to the nucleotide at position 1364 and from the nucleotide at position 1368 to the nucleotide at position 1650, the nucleotide sequence of SEQ ID No: 4 from the nucleotide at position 764 to the nucleotide at position 1364 and from the nucleotide at position 1368 to the nucleotide at position 1650, the nucleotide sequence of SEQ ID No: 4 from the nucleotide at position 664 to the nucleotide at position 1650, the nucleotide sequence of SEQ ID No: 4 from the nucleotide at position 564 to the nucleotide at position 1364 and from the nucleotide at position 1368 to the nucleotide at position 1650, the nucleotide sequence of SEQ ID No: 4 from the nucleotide at position 464 to the nucleotide at position 1364 and from the nucleotide at position 1368 to the nucleotide at position 1650, the nucleotide sequence of SEQ ID No: 4 from the nucleotide at position 364 to the nucleotide at position 1364 and from the nucleotide at position 1368 to the nucleotide at position 1650, the nucleotide sequence of SEQ ID No: 4 from the nucleotide at position 264 to the nucleotide at position 1364 and from the nucleotide at position 1368 to the nucleotide at position 1650, the nucleotide sequence of SEQ ID No: 4 from the nucleotide at position 164 to the nucleotide at position 1364 and from the nucleotide 1368 to the nucleotide at position 1650, the nucleotide sequence of SEQ ID No: 4 from the nucleotide at position 64 to the nucleotide at position 1364 and from the nucleotide at position 1368 to the nucleotide at position 165, or the nucleotide sequence of SEQ ID No: 4 from the nucleotide at position 1 to the nucleotide at position 1364 and from the nucleotide at position 1368 to the nucleotide at position 1650. A promoter fragment according to the invention may also comprise the nucleotide sequence of SEQ ID No: 5 from the nucleotide at position 965 to the nucleotide at position 1365 and from the nucleotide sequence 1369 to the nucleotide position 1669, the nucleotide sequence of SEQ ID No: 5 from the nucleotide at position 865 to the nucleotide at position 1365 and from the nucleotide position 1369 to the nucleotide position 1669, the nucleotide sequence of SEQ ID No: 5 from the nucleotide at position 765 to the nucleotide at position 1365 and from the nucleotide position 1369 to the nucleotide position 1669, the nucleotide sequence of SEQ ID No: 5 from the nucleotide at position 665 to the nucleotide at position 1365 and from the nucleotide at position 1369 to the nucleotide at position 1669, the nucleotide sequence of SEQ ID No: 5 from the nucleotide at position 565 to the nucleotide at position 1365 and from the nucleotide at position1369 to the nucleotide at position 1669, the nucleotide sequence of SEQ ID No: 5 from the nucleotide at position 465 to the nucleotide at position 1365 and from the nucleotide at position 1369 to the nucleotide at position 1669, the nucleotide sequence of SEQ ID No: 5 from the nucleotide at position 365 to the nucleotide at position 1365 and from the nucleotide at position 1369 to the nucleotide at position 1669, the nucleotide sequence of SEQ ID No: 5 from the nucleotide at position 265 to the nucleotide at position 1365 and from the nucleotide at position 1369 to the nucleotide at position 1669, the nucleotide sequence of SEQ ID No: 5 from the nucleotide at position 165 to the nucleotide at position 1365 and from the nucleotide at position 1369 to the nucleotide at position 1669, the nucleotide sequence of SEQ ID No: 5 from the nucleotide at position 65 to the nucleotide at position 1365 and from the nucleotide at position 1369 to the nucleotide at position 1669, the nucleotide sequence of SEQ ID No: 5 from the nucleotide at position 1 to the nucleotide at position 1365 and from the nucleotide at position 1369 to the nucleotide at position 1669, the nucleotide sequence of SEQ ID No: 5 from the nucleotide at position 965 to the nucleotide at position 1365 and from the nucleotide at position 1369 to the nucleotide at position 1769, the nucleotide sequence of SEQ ID No: 5 from the nucleotide at position 865 to the nucleotide at position 1365 and from the nucleotide at position 1369 to the nucleotide at position 1769, the nucleotide sequence of SEQ ID No: 5 from the nucleotide at position 765 to the nucleotide at position 1365 and from the nucleotide at position 1369 to the nucleotide at position 1769, the nucleotide sequence of SEQ ID No: 5 from the nucleotide at position 665 to the nucleotide at position 1365 and from the nucleotide at position 1369 to the nucleotide at position 1769, the nucleotide sequence of SEQ ID No: 5 from the nucleotide at position 565 to the nucleotide at position 1365 and from the nucleotide at position 1369 and to the nucleotide at position 1769, the nucleotide sequence of SEQ ID No: 5 from the nucleotide at position 465 to the nucleotide at position 1365 and from the nucleotide at position 1369 to the nucleotide at position 1769, the nucleotide sequence of SEQ ID No: 5 from the nucleotide at position 365 to the nucleotide at position 1365 and from the nucleotide at position 1369 to the nucleotide at position 1769, the nucleotide sequence of SEQ ID No: 5 from the nucleotide at position 265 to the nucleotide at position 1365 and from the nucleotide at position 1369 to the nucleotide at position 1769, the nucleotide sequence of SEQ ID No: 5 from the nucleotide at position 165 to the nucleotide at position 1365 and from the nucleotide at position 1369 to the nucleotide at position 1769, the nucleotide sequence of SEQ ID No: 5 from the nucleotide at position 65 to the nucleotide at position 1365 and from the nucleotide at position 1369 to the nucleotide at position 1769, the nucleotide sequence of SEQ ID No: 5 from the nucleotide at position 1 to the nucleotide at position 1365 and from the nucleotide at position 1369 to the nucleotide at position 1769, the nucleotide sequence of SEQ ID No: 5 from the nucleotide at position 965 to the nucleotide at position 1365 and from the nucleotide at position 1369 to the nucleotide at position 1869, the nucleotide sequence of SEQ ID No: 5 from the nucleotide at position 865 to the nucleotide at position 1365 and from the nucleotide at position 1369 to the nucleotide at position 1869, the nucleotide sequence of SEQ ID No: 5 from the nucleotide at position 765 to the nucleotide at position 1365 and from the nucleotide at position 1369 to the nucleotide at position 1869, the nucleotide sequence of SEQ ID No: 5 from the nucleotide at position 665 to the nucleotide at position 1365 and from the nucleotide at position 1369 to the nucleotide at position 1869, the nucleotide sequence of SEQ ID No: 5 from the nucleotide at position 565 to the nucleotide at position 1365 and from the nucleotide at position 1369 to the nucleotide at position 1869, the nucleotide sequence of SEQ ID No: 5 from the nucleotide at position 465 to the nucleotide at position 1365 and from the nucleotide at position 1369 to the nucleotide at position 1869, the nucleotide sequence of SEQ ID No: 5 from the nucleotide at position 365 to the nucleotide at position 1365 and from the nucleotide at position 1369 to the nucleotide at position 1869, the nucleotide sequence of SEQ ID No: 5 from the nucleotide at position 265 to the nucleotide at position 1365 and from the nucleotide at position 1369 to the nucleotide at position 1869, the nucleotide sequence of SEQ ID No: 5 from the nucleotide at position 165 to the nucleotide at position 1365 and from the nucleotide at position 1369 to the nucleotide at position 1869, the nucleotide sequence of SEQ ID No: 5 from the nucleotide at position 65 to the nucleotide at position 1365 and from the nucleotide at position 1369 to the nucleotide at position 1869, the nucleotide sequence of SEQ ID No: 5 from the nucleotide at position 1 to the nucleotide at position 1365 and from the nucleotide at position 1369 to the nucleotide at position 1869, the nucleotide sequence of SEQ ID No: 5 from the nucleotide at position 965 to the nucleotide at position 1365 and from the nucleotide at position 1369 to the nucleotide at position 1969, the nucleotide sequence of SEQ ID No: 5 from the nucleotide at position 865 to the nucleotide at position 1365 and from the nucleotide at position 1369 to the nucleotide at position 1969, the nucleotide sequence of SEQ ID No: 5 from the nucleotide at position 765 to the nucleotide at position 1365 and from the nucleotide at position 1369 to the nucleotide at position 1969, the nucleotide sequence of SEQ ID No: 5 from the nucleotide at position 665 to the nucleotide at position 1365 and from the nucleotide at position 1369 to the nucleotide at position 1969, the nucleotide sequence of SEQ ID No: 5 from the nucleotide at position 565 to the nucleotide at position 1365 and from the nucleotide at position 1369 to the nucleotide at position 1969, the nucleotide sequence of SEQ ID No: 5 from the nucleotide at position 465 to the nucleotide at position 1365 and from the nucleotide at position 1369 to the nucleotide at position 1969, the nucleotide sequence of SEQ ID No: 5 from the nucleotide at position 365 to the nucleotide at position 1365 and from the nucleotide at position 1369 to the nucleotide at position 1969, the nucleotide sequence of SEQ ID No: 5 from the nucleotide at position 265 to the nucleotide at position 1365 and from the nucleotide at position 1369 to the nucleotide at position 1969, the nucleotide sequence of SEQ ID No: 5 from the nucleotide at position 165 to the nucleotide at position 1365 and from the nucleotide at position 1369 to the nucleotide at position 1969, the nucleotide sequence of SEQ ID No: 5 from the nucleotide at position 65 to the nucleotide at position 1365 and from the nucleotide at position 1369 to the nucleotide at position 1969, the nucleotide sequence of SEQ ID No: 5 from the nucleotide at position 1 to the nucleotide at position 1365 and from the nucleotide at position 1369 to the nucleotide at position 1969, the nucleotide sequence of SEQ ID No: 5 from the nucleotide at position 965 to the nucleotide at position 1365 and from the nucleotide at position 1369 to the nucleotide at position 2001, the nucleotide sequence of SEQ ID No: 5 from the nucleotide at position 865 to the nucleotide at position 1365 and from the nucleotide at position 1369 to the nucleotide at position 2001, the nucleotide sequence of SEQ ID No: 5 from the nucleotide at position 765 to the nucleotide at position 1365 and from the nucleotide at position 1369 to the nucleotide at position 2001, the nucleotide sequence of SEQ ID No: 5 from the nucleotide at position 665 to the nucleotide at position 1365 and from the nucleotide at position 1369 to the nucleotide at position 2001, the nucleotide sequence of SEQ ID No: 5 from the nucleotide at position 565 to the nucleotide at position 1365 and from the nucleotide at position 1369 to the nucleotide at position 2001, the nucleotide sequence of SEQ ID No: 5 from the nucleotide at position 465 to the nucleotide at position 1365 and from the nucleotide at position 1369 to the nucleotide at position 2001, the nucleotide sequence of SEQ ID No: 5 from the nucleotide at position 365 to the nucleotide at position 1365 and from the nucleotide at position 1369 to the nucleotide at position 2001, the nucleotide sequence of SEQ ID No: 5 from the nucleotide at position 265 to the nucleotide at position 1365 and from the nucleotide at position 1369 to the nucleotide at position 2001, the nucleotide sequence of SEQ ID No: 5 from the nucleotide at position 165 to the nucleotide at position 1365 and from the nucleotide at position 1369 to the nucleotide at position 2001, the nucleotide sequence of SEQ ID No: 5 from the nucleotide at position 65 to the nucleotide at position 1365 and from the nucleotide at position 1369 to the nucleotide at position 2001, or the nucleotide sequence of SEQ ID No: 5 from the nucleotide at position 1 to the nucleotide at position 1365 and from the nucleotide at position 1369 to the nucleotide at position 2001. A promoter fragment according to the invention may also comprise the nucleotide sequence of SEQ ID No: 6 from the nucleotide at position 958 to the nucleotide at position 1358 and from the nucleotide at position 1362 to the nucleotide at position 1647, the nucleotide sequence of SEQ ID No: 6 from the nucleotide at position 858 to the nucleotide at position 1358 and from the nucleotide at position 1362 to the nucleotide at position 1647, the nucleotide sequence of SEQ ID No: 6 from the nucleotide at position 758 to the nucleotide at position 1358 and from the nucleotide at position 1362 to the nucleotide at position 1647, the nucleotide sequence of SEQ ID No: 6 from the nucleotide at position 658 to the nucleotide at position 1358 and from the nucleotide at position 1362 to the nucleotide at position 1647, the nucleotide sequence of SEQ ID No: 6 from the nucleotide at position 558 to the nucleotide at position 1358 and from the nucleotide at position 1362 to the nucleotide at position 1647, the nucleotide sequence of SEQ ID No: 6 from the nucleotide at position 458 to the nucleotide at position 1358 and from the nucleotide at position 1362 to the nucleotide at position 1647, the nucleotide sequence of SEQ ID No: 6 from the nucleotide at position 358 to the nucleotide at position 1358 and from the nucleotide at position 1362 to the nucleotide at position 1647, the nucleotide sequence of SEQ ID No: 6 from the nucleotide at position 258 to the nucleotide at position 1358 and from the nucleotide at position 1362 to the nucleotide at position 1647, the nucleotide sequence of SEQ ID No: 6 from the nucleotide at position 158 to the nucleotide at position 1358 and from the nucleotide at position 1362 to the nucleotide at position 1647, the nucleotide sequence of SEQ ID No: 6 from the nucleotide at position 58 to the nucleotide at position 1358 and from the nucleotide at position 1362 to the nucleotide at position 1647, or the nucleotide sequence of SEQ ID No: 6 from the nucleotide at position 1 to the nucleotide at position 1358 and from the nucleotide at position 1362 to the nucleotide at position 1647. A promoter fragment according to the invention may also comprise the nucleotide sequence of SEQ ID No: 7 from the nucleotide at position 963 to the nucleotide at position 1363 and from the nucleotide at position 1367 to the nucleotide at position 1667, the nucleotide sequence of SEQ ID No: 7 from the nucleotide at position 863 to the nucleotide at position 1363 and from the nucleotide at position 1367 to the nucleotide at position 1667, the nucleotide sequence of SEQ ID No: 7 from the nucleotide at position 763 to the nucleotide at position 1363 and from the nucleotide at position 1367 to the nucleotide at position 1667, the nucleotide sequence of SEQ ID No: 7 from the nucleotide at position 663 to the nucleotide at position 1363 and from the nucleotide at position 1367 to the nucleotide at position 1667, the nucleotide sequence of SEQ ID No: 7 from the nucleotide at position 563 to the nucleotide at position 1363 and from the nucleotide at position 1367 to the nucleotide at position 1667, the nucleotide sequence of SEQ ID No: 7 from the nucleotide at position 463 to the nucleotide at position 1363 and from the nucleotide at position 1367 to the nucleotide at position 1667, the nucleotide sequence of SEQ ID No: 7 from the nucleotide at position 363 to the nucleotide at position 1363 and from the nucleotide at position 1367 to the nucleotide at position 1667, the nucleotide sequence of SEQ ID No: 7 from the nucleotide at position 263 to the nucleotide at position 1363 and from the nucleotide at position 1367 to the nucleotide at position 1667, the nucleotide sequence of SEQ ID No: 7 from the nucleotide at position 163 to the nucleotide at position 1363 and from the nucleotide at position 1367 to the nucleotide at position 1667, the nucleotide sequence of SEQ ID No: 7 from the nucleotide at position 63 to the nucleotide at position 1363 and from the nucleotide at position 1367 to the nucleotide at position 1667, the nucleotide sequence of SEQ ID No: 7 from the nucleotide at position 1 to the nucleotide at position 1363 and from the nucleotide at position 1367 to the nucleotide at position 1667, the nucleotide sequence of SEQ ID No: 7 from the nucleotide at position 963 to the nucleotide at position 1363 and from the nucleotide at position 1367 to the nucleotide at position 1767, the nucleotide sequence of SEQ ID No: 7 from the nucleotide at position 863 to the nucleotide at position 1363 and from the nucleotide at position 1367 to the nucleotide at position 1767, the nucleotide sequence of SEQ ID No: 7 from the nucleotide at position 763 to the nucleotide at position 1363 and from the nucleotide at position 1367 to the nucleotide at position 1767, the nucleotide sequence of SEQ ID No: 7 from the nucleotide at position 663 to the nucleotide at position 1363 and from the nucleotide at position 1367 to the nucleotide at position 1767, the nucleotide sequence of SEQ ID No: 7 from the nucleotide at position 563 to the nucleotide at position 1363 and from the nucleotide at position 1367 to the nucleotide at position 1767, the nucleotide sequence of SEQ ID No: 7 from the nucleotide at position 463 to the nucleotide at position 1363 and from the nucleotide at position 1367 to the nucleotide at position 1767, the nucleotide sequence of SEQ ID No: 7 from the nucleotide at position 363 to the nucleotide at position 1363 and from the nucleotide at position 1367 to the nucleotide at position 1767, the nucleotide sequence of SEQ ID No: 7 from the nucleotide at position 263 to the nucleotide at position 1363 and from the nucleotide at position 1367 to the nucleotide at position 1767, the nucleotide sequence of SEQ ID No: 7 from the nucleotide at position 163 to the nucleotide at position 1363 and from the nucleotide at position 1367 to the nucleotide at position 1767, the nucleotide sequence of SEQ ID No: 7 from the nucleotide at position 63 to the nucleotide at position 1363 and from the nucleotide at position 1367 to the nucleotide at position 1767, the nucleotide sequence of SEQ ID No: 7 from the nucleotide at position 1 to the nucleotide at position 1363 and from the nucleotide at position 1367 to the nucleotide at position 1767, the nucleotide sequence of SEQ ID No: 7 from the nucleotide at position 963 to the nucleotide at position 1363 and from the nucleotide at position 1367 to the nucleotide at position 1867, the nucleotide sequence of SEQ ID No: 7 from the nucleotide at position 863 to the nucleotide at position 1363 and from the nucleotide at position 1367 to the nucleotide at position 1867, the nucleotide sequence of SEQ ID No: 7 from the nucleotide at position 763 to the nucleotide at position 1363 and from the nucleotide at position 1367 to the nucleotide at position 1867, the nucleotide sequence of SEQ ID No: 7 from the nucleotide at position 663 to the nucleotide at position 1363 and from the nucleotide at position 1367 to the nucleotide at position 1867, the nucleotide sequence of SEQ ID No: 7 from the nucleotide at position 563 to the nucleotide at position 1363 and from the nucleotide at position 1367 to the nucleotide at position 1867, the nucleotide sequence of SEQ ID No: 7 from the nucleotide at position 463 to the nucleotide at position 1363 and from the nucleotide at position 1367 to the nucleotide at position 1867, the nucleotide sequence of SEQ ID No: 7 from the nucleotide at position 363 to the nucleotide at position 1363 and from the nucleotide at position 1367 to the nucleotide at position 1867, the nucleotide sequence of SEQ ID No: 7 from the nucleotide at position 263 to the nucleotide at position 1363 and from the nucleotide at position 1367 to the nucleotide at position 1867, the nucleotide sequence of SEQ ID No: 7 from the nucleotide at position 163 to the nucleotide at position 1363 and from the nucleotide at position 1367 to the nucleotide at position 1867, the nucleotide sequence of SEQ ID No: 7 from the nucleotide at position 63 to the nucleotide at position 1363 and from the nucleotide at position 1367 to the nucleotide at position 1867, the nucleotide sequence of SEQ ID No: 7 from the nucleotide at position 1 to the nucleotide at position 1363 and from the nucleotide at position 1367 to the nucleotide at position 1867, the nucleotide sequence of SEQ ID No: 7 from the nucleotide at position 963 to the nucleotide at position 1363 and from the nucleotide at position 1367 to the nucleotide at position 1967, the nucleotide sequence of SEQ ID No: 7 from the nucleotide at position 863 to the nucleotide at position 1363 and from the nucleotide at position 1367 to the nucleotide at position 1967, the nucleotide sequence of SEQ ID No: 7 from the nucleotide at position 763 to the nucleotide at position 1363 and from the nucleotide at position 1367 to the nucleotide at position 1967, the nucleotide sequence of SEQ ID No: 7 from the nucleotide at position 663 to the nucleotide at position 1363 and from the nucleotide at position 1367 to the nucleotide at position 1967, the nucleotide sequence of SEQ ID No: 7 from the nucleotide at position 563 to the nucleotide at position 1363 and from the nucleotide at position 1367 to the nucleotide at position 1967, the nucleotide sequence of SEQ ID No: 7 from the nucleotide at position 463 to the nucleotide at position 1363 and from the nucleotide at position 1367 to the nucleotide at position 1967, the nucleotide sequence of SEQ ID No: 7 from the nucleotide at position 363 to the nucleotide at position 1363 and from the nucleotide at position 1367 to the nucleotide at position 1967, the nucleotide sequence of SEQ ID No: 7 from the nucleotide at position 263 to the nucleotide at position 1363 and from the nucleotide at position 1367 to the nucleotide at position 1967, the nucleotide sequence of SEQ ID No: 7 from the nucleotide at position 163 to the nucleotide at position 1363 and from the nucleotide at position 1367 to the nucleotide at position 1967, the nucleotide sequence of SEQ ID No: 7 from the nucleotide at position 63 to the nucleotide at position 1363 and from the nucleotide at position 1367 to the nucleotide at position 1967, the nucleotide sequence of SEQ ID No: 7 from the nucleotide at position 1 to the nucleotide at position 1363 and from the nucleotide at position 1367 to the nucleotide at position 1967, the nucleotide sequence of SEQ ID No: 7 from the nucleotide at position 963 to the nucleotide at position 1363 and from the nucleotide at position 1367 to the nucleotide at position 2001, the nucleotide sequence of SEQ ID No: 7 from the nucleotide at position 863 to the nucleotide at position 1363 and from the nucleotide at position 1367 to the nucleotide at position 2001, the nucleotide sequence of SEQ ID No: 7 from the nucleotide at position 763 to the nucleotide at position 1363 and from the nucleotide at position 1367 to the nucleotide at position 2001, the nucleotide sequence of SEQ ID No: 7 from the nucleotide at position 663 to the nucleotide at position 1363 and from the nucleotide at position 1367 to the nucleotide at position 2001, the nucleotide sequence of SEQ ID No: 7 from the nucleotide at position 563 to the nucleotide at position 1363 and from the nucleotide at position 1367 to the nucleotide at position 2001, the nucleotide sequence of SEQ ID No: 7 from the nucleotide at position 463 to the nucleotide at position 1363 and from the nucleotide at position 1367 to the nucleotide at position 2001, the nucleotide sequence of SEQ ID No: 7 from the nucleotide at position 363 to the nucleotide at position 1363 and from the nucleotide at position 1367 to the nucleotide at position 2001, the nucleotide sequence of SEQ ID No: 7 from the nucleotide at position 263 to the nucleotide at position 1363 and from the nucleotide at position 1367 to the nucleotide at position 2001, the nucleotide sequence of SEQ ID No: 7 from the nucleotide at position 163 to the nucleotide at position 1363 and from the nucleotide at position 1367 to the nucleotide at position 2001, the nucleotide sequence of SEQ ID No: 7 from the nucleotide at position 63 to the nucleotide at position 1363 and from the nucleotide at position 1367 to the nucleotide at position 2001, or the nucleotide sequence of SEQ ID No: 7 from the nucleotide at position 1 to the nucleotide at position 1363 and from the nucleotide at position 1367 to the nucleotide at position 2001.

Promoter activity for a functional promoter fragment in seeds may be determined by those skilled in the art, for example using analysis of RNA accumulation produced from the nucleic acid which is operably linked to the promoter as described herein, whereby the nucleic acid which is operably linked to the promoter can be the nucleic acid which is naturally linked to the promoter, i.e. the endogenous gene of which expression is driven by the promoter.

The seed- and funiculus-preferential expression capacity of the identified or generated fragments of the promoters of the invention can be conveniently tested by determining levels of the transcript of which expression is naturally driven by the promoter of the invention, i.e. endogenous transcript levels, such as, for example, using the methods as described herein in the Examples. Further, the seed- and funiculus-preferential expression capacity of the identified or generated fragments of the promoters of the invention can be conveniently tested by operably linking such DNA molecules to a nucleotide sequence encoding an easy scorable marker, e.g. a beta-glucuronidase gene, introducing such a chimeric gene into a plant and analyzing the expression pattern of the marker in seeds and in the funiculus as compared with the expression pattern of the marker in other parts of the plant. Other candidates for a marker (or a reporter gene) are chloramphenicol acetyl transferase (CAT) and proteins with fluorescent properties, such as green fluorescent protein (GFP) from *Aequora victoria* or proteins with luminescent properties such as the *Renilla* luciferase or the bacterial *lux* operon. To define a minimal promoter region, a DNA segment representing the promoter region is removed from the 5' region of the gene of interest and operably linked to the coding sequence of a marker (reporter) gene by recombinant DNA techniques well known to the art. The reporter gene is operably linked downstream of the promoter, so that transcripts initiating at the promoter proceed through the reporter gene. Reporter genes generally encode proteins, which are easily measured, including, but not limited to, chloramphenicol acetyl transferase (CAT), beta-glucuronidase (GUS), green fluorescent protein (GFP), beta-galactosidase (beta-GAL), and luciferase. The expression cassette containing the reporter gene under the control of the promoter can be introduced into an appropriate cell type by transfection techniques well known to the art. To assay for the reporter protein, cell lysates are prepared and appropriate assays, which are well known in the art, for the reporter protein are performed. For example, if CAT were the reporter gene of choice, the lysates from cells transfected with constructs containing CAT under the control of a promoter under study are mixed with isotopically labeled chloramphenicol and acetyl-coenzyme A (acetyl-CoA). The CAT enzyme transfers the acetyl group from acetyl-CoA to the 2- or 3-position of chloramphenicol. The reaction is monitored by thin-layer chromatography, which separates acetylated chloramphenicol from unreacted material. The reaction products are then visualized by autoradiography. The level of enzyme activity corresponds to the amount of enzyme that was made, which in turn reveals the level of expression and the seed-preferential functionality from the promoter or promoter fragment of interest. This level of expression can also be compared to other promoters to determine the relative strength of the promoter under study. Once activity and functionality is confirmed, additional mutational and/or deletion analyses may be employed to determine the minimal region and/or sequences required to initiate transcription. Thus, sequences can be deleted at the 5' end of the promoter region and/or at the 3' end of the promoter region, and nucleotide substitutions introduced. These constructs are then again introduced in cells and their activity and/or functionality determined.

The activity or strength of a promoter may be measured in terms of the amount of mRNA or protein accumulation it specifically produces, relative to the total amount of mRNA or protein. The promoter preferably expresses an operably linked nucleic acid sequence at a level greater than about 0.001%, about 0.002%, more preferably greater than about 0.005% of the total mRNA. Alternatively, the activity or strength of a promoter may be expressed relative to a well-characterized promoter (for which transcriptional activity was previously assessed).

It will herein further be clear that equivalent seed- and funiculus-preferential promoters can be isolated from other plants. To this end, equivalent promoters can be isolated using the coding sequences of the genes driven by the promoters of any one of SEQ ID NOs: 3 to 7 to screen a genomic library (e.g. by hybridization or *in silico*) of a crop of interest. When sufficient identity between the coding sequences is obtained (for example, higher than 85% identity) then promoter regions can be isolated upstream of the orthologous genes.

Described herein are nucleic acids comprising seed- and funiculus-preferential promoter activity which comprise a nucleotide sequence having at least 40%, at least 50%, or at least 60%, or at least 70%, or at least 80%, or at least 85%, or at least 90%, or at least 95%, or at least 98% sequence identity to the herein described promoters and promoter regions or functional fragments thereof and are also referred to as variants. The term "variant" with respect to the transcription regulating nucleotide sequences SEQ ID NOs: 3 to 7 of the invention is intended to mean substantially similar sequences. Naturally occurring allelic variants such as these can be identified with the use of well-known molecular biology techniques, as, for example, with polymerase chain reaction (PCR) and hybridization techniques as herein outlined before. Variant nucleotide sequences also include synthetically derived nucleotide sequences, such as those generated, for example, by using site-directed mutagenesis of any one of SEQ ID NOs: 3 to 7. Generally, nucleotide sequence variants of the invention will have at least 40%, 50%, 60%, to 70%, e.g., preferably 71%, 72%, 73%, 74%, 75%, 76%, 77%, 78%, to 79%, generally at least 80%, e.g., 81% to 84%, at least 85%, e.g., 86%, 87%, 88%, 89%, 90%, 91%, 92%, 93%, 94%, 95%, 96%, 97%, to 98% and 99% nucleotide sequence identity to the native (wild type or endogenous) nucleotide sequence or a functional fragment thereof. Derivatives of the DNA molecules disclosed herein may include, but are not limited to, deletions of sequence, single or multiple point mutations, alterations at a particular restriction enzyme site, addition of functional elements, or other means of molecular modification which may enhance, or otherwise alter promoter expression. Techniques for obtaining such derivatives are well-known in the art (see, for example, J. F. Sambrook, D. W. Russell, and N. Irwin (2000) Molecular Cloning: A Laboratory Manual, 3rd edition Volumes 1, 2, and 3. Cold Spring Harbor Laboratory Press). For example, one of ordinary skill in the art may delimit the functional elements within the promoters disclosed herein and delete any non-essential elements. Functional elements may be modified or combined to increase the utility or expression of the sequences of the invention for any particular application. Those of skill in the art are familiar with the standard resource materials that describe specific conditions and procedures for the construction, manipulation, and isolation of macromolecules (e.g., DNA molecules, plasmids, etc.), as well as the generation of recombinant organisms and the screening and isolation of DNA molecules. As used herein, the term "percent sequence identity" refers to the percentage of identical nucleotides between two segments of a window of optimally aligned DNA. Optimal alignment of sequences for aligning a comparison window are well-known to those skilled in the art and may be conducted by tools such as the local homology algorithm of Smith and Waterman (Waterman, M. S. Introduction to Computational Biology: Maps, sequences and genomes. Chapman & Hall. London (1995), the homology alignment algorithm of Needleman and Wunsch (J. Mol. Biol., 48:443-453 (1970), the search for similarity method of Pearson and Lipman (Proc. Natl. Acad. Sci., 85:2444 (1988), and preferably by computerized implementations of these algorithms such as GAP, BESTFIT, FASTA, and TFASTA available as part of the GCG (Registered Trade Mark), Wisconsin Package (Registered Trade Mark from Accelrys Inc., San Diego, Calif.). An "identity fraction" for aligned segments of a test sequence and a reference sequence is the number of identical components that are shared by the two aligned sequences divided by the total number of components in the reference sequence segment, i.e., the entire reference sequence or a smaller defined part of the reference sequence. Percent sequence identity is represented as the identity fraction times 100. The comparison of one or more DNA sequences may be to a full-length DNA sequence or a portion thereof, or to a longer DNA sequence.

A nucleic acid comprising a nucleotide sequence having at least 80% sequence identity to any one of SEQ ID NO: 3 to 7 can thus be a nucleic acid comprising a nucleotide sequence having at least 80%, or at least 85%, or at least 90%, or at least 95%, or at least 98%, or 100% sequence identity to any one of SEQ ID NOs: 3 to 7.

A "functional fragment" of a nucleic acid comprising seed-preferential promoter denotes a nucleic acid comprising a stretch of the nucleic acid sequences of any one of SEQ ID NOs: 3 to 7, or of the nucleic acid having at least 80% sequence identity to any one of SEQ ID NOs: 3 to 7 which still exerts the desired function, i.e. which has seed-preferential promoter activity. Assays for determining seed-preferential promoter activity are provided herein. Preferably, the functional fragment of the seed-preferential promoter contains the conserved promoter motifs, such as, for example, conserved promoter motifs as described in DoOP (doop.abc.hu, databases of Orthologous Promoters, Barta E. et al (2005) Nucleic Acids Research Vol. 33, D86-D90). A functional fragment may be a fragment of at least about 400 bp, at least about 500 bp, at least about 600 bp, at least about 700 bp, at least about 800 bp, at least about 900 bp, at least about 1000 bp, at least about 1100 bp; at least about 1200 bp, or at least about 1300 bp upstream of the first ATG start codon of the SLP1 transcripts and at least about 300 bp, at least about 400 bp, at least about 500 bp or at least about 600 bp downstream of the first ATG start codon of the SLP1 gene and have seed- and funiculus-preferential promoter activity.

A nucleic acid comprising the nucleotide sequence of any one of SEQ ID NO: 3 to 7 which further comprises insertion, deletion, substitution of at least 1 nucleotide up to 20 nucleotides, at least 1 nucleotide up to 15 nucleotides, at least 1 nucleotide up to 10 nucleotides, at least 1 nucleotide up to 5 nucleotides, at least 1 nucleotide up to 4 nucleotides, at least 1 nucleotide up to 3 nucleotides, or even at least 1 nucleotide up to 2 nucleotides may cover at least about 300 bp, at least about 400 bp, at least about 500 bp, at least about 600 bp, at least about 700 bp, at least about 800 bp, at least about 900 bp, at least about 1000 bp, at least about 1100 bp; at least about 1200 bp, or at least about 1300 bp from the translation start site.

A number of highly conserved regions (consensus sequences) were identified on the promoter sequence disclosed herein.

Variants of the promoter described herein include those which comprise the identified consensus sequences - promoter consensus sequence (SEQ ID NO: 18), UTR consensus sequence 1 (SEQ ID NO: 19), UTR consensus sequence 2 (SEQ ID NO: 20), UTR consensus sequence 3 (SEQ ID NO: 21), intron consensus sequence 1 (SEQ ID NO: 22), intron consensus sequence 2 (SEQ ID NO: 23) and/or intron consensus sequence 3 (SEQ ID NO: 24)-, but have otherwise been modified to delete nucleotide stretches within the sequence which are not needed for the promoter to be functional in seed- and funiculus-preferential manner. For example, any nucleotide stretch located between the consensus sequences and /or between the transcriptional start and the first consensus sequence may be at least partially deleted to result in a shorter nucleotide sequence than the about 2 kb sequence of SEQ ID NOs: 3, 5 and 7, or than the about 1.7 kb of SEQ ID NOs: 4 and 6.

"Isolated nucleic acid", used interchangeably with "isolated DNA" as used herein refers to a nucleic acid not occurring in its natural genomic context, irrespective of its length and sequence. Isolated DNA can, for example, refer to DNA which is physically separated from the genomic context, such as a fragment of genomic DNA. Isolated DNA can also be an artificially produced DNA, such as a chemically synthesized DNA, or such as DNA produced via amplification reactions, such as polymerase chain reaction (PCR) well-known in the art. Isolated DNA can further refer to DNA present in a context of DNA in which it does not occur naturally. For example, isolated DNA can refer to a piece of DNA present in a plasmid. Further, the isolated DNA can refer to a piece of DNA present in another chromosomal context than the context in which it occurs naturally, such as for example at another position in the genome than the natural position, in the genome of another species than the species in which it occurs naturally, or in an artificial chromosome.

A further embodiment provides a recombinant gene comprising the nucleic acid according to the invention operably linked to a heterologous nucleic acid sequence encoding an expression product of interest, and optionally a transcription termination and polyadenylation sequence, preferably a transcription termination and polyadenylation region functional in plant cells. In a further embodiment, said expression product of interest an RNA capable of modulating the expression of a gene or is a protein.

The term "expression product" refers to a product of transcription. Said expression product can be the transcribed RNA. It is understood that the RNA which is produced is a biologically active RNA. Said expression product can also be a peptide, a polypeptide, or a protein, when said biologically active RNA is an mRNA and said protein is produced by translation of said mRNA.

Alternatively, the heterologous nucleic acid, operably linked to the promoters of the invention, may also code for an RNA capable of modulating the expression of a gene. Said RNA capable of modulating the expression of a gene can be an RNA which reduces expression of a gene. Said RNA can reduce the expression of a gene for example through the mechanism of RNA-mediated gene silencing.

Said RNA capable of modulating the expression of a gene can be a silencing RNA down-regulating expression of a target gene. As used herein, "silencing RNA" or "silencing RNA molecule" refers to any RNA molecule, which upon introduction into a plant cell, reduces the expression of a target gene. Such silencing RNA may e.g. be so-called "antisense RNA", whereby the RNA molecule comprises a sequence of at least 20 consecutive nucleotides having 95% sequence identity to the complement of the sequence of the target nucleic acid, preferably the coding sequence of the target gene. However, antisense RNA may also be directed to regulatory sequences of target genes, including the promoter sequences and transcription termination and polyadenylation signals. Silencing RNA further includes so-called "sense RNA" whereby the RNA molecule comprises a sequence of at least 20 consecutive nucleotides having 95% sequence identity to the sequence of the target nucleic acid. Other silencing RNA may be "unpolyadenylated RNA" comprising at least 20 consecutive nucleotides having 95% sequence identity to the complement of the sequence of the target nucleic acid, such as described in WO01/12824 or US6423885 (both documents herein incorporated by reference). Yet another type of silencing RNA is an RNA molecule as described in WO03/076619 (herein incorporated by reference) comprising at least 20 consecutive nucleotides having 95% sequence identity to the sequence of the target nucleic acid or the complement thereof, and further comprising a largely-double stranded region as described in WO03/076619 (including largely double stranded regions comprising a nuclear localization signal from a viroid of the Potato spindle tuber viroid-type or comprising CUG trinucleotide repeats). Silencing RNA may also be double stranded RNA comprising a sense and antisense strand as herein defined, wherein the sense and antisense strand are capable of base-pairing with each other to form a double stranded RNA region (preferably the said at least 20 consecutive nucleotides of the sense and antisense RNA are complementary to each other). The sense and antisense region may also be present within one RNA molecule such that a hairpin RNA (hpRNA) can be formed when the sense and antisense region form a double stranded RNA region. hpRNA is well-known within the art (see e.g WO99/53050, herein incorporated by reference). The hpRNA may be classified as long hpRNA, having long, sense and antisense regions which can be largely complementary, but need not be entirely complementary (typically larger than about 200 bp, ranging between 200 and 1000 bp). hpRNA can also be rather small ranging in size from about 30 to about 42 bp, but not much longer than 94 bp (see WO04/073390, herein incorporated by reference). Silencing RNA may also be artificial micro-RNA molecules as described e.g. in WO2005/052170, WO2005/047505 or US 2005/0144667, or ta-siRNAs as described in WO2006/074400. Said RNA capable of modulating the expression of a gene can also be an RNA ribozyme.

Said RNA capable of modulating the expression of a gene can modulate, preferably down-regulate, the expression of other genes (i.e. target genes) comprised within the seeds or funiculus or even of genes present within a pathogen or pest that feeds upon the seeds of the transgenic plant such as a virus, fungus, insect, bacteria.

The nucleic acid sequence heterologous to the promoters according to the invention may generally be any nucleic acid sequence effecting increased, altered (e.g. in a different organ) or reduced level of transcription of a gene for which such expression modulation is desired. The nucleic acid sequence can for example encode a protein of interest. Exemplary genes for which an increased or reduced level of transcription may be desired in the seeds are e.g. nucleic acids that can provide an agriculturally or industrially important feature in seeds. Suitable heterologous nucleic acid sequences of interest include nucleic acids modulating expression of genes conferring resistance to diseases, stress tolerance genes, genes involved in at different stages of fatty acid biosynthesis or degradation, in acyl editing, in storage compound storage or breakdown, genes encoding epoxidases, hydroxylases, cytochrome P450 mono-oxygenases, desaturases, tocopherol biosynthetic enzymes, carotenoid biosynthesis enzymes, amino acid biosynthetic enzymes, steroid pathway enzymes, starch branching enzymes, genes encoding proteins involved in starch synthesis, glycolysis, carbon metabolism, oxidative pentose phosphate cycle, protein synthesis, organelle organization and biogenesis, DNA metabolism, DNA replication, cell cycle, cell organization and biogenesis, cell proliferation, chromosome organization and biogenesis, microtubule-based processes, microtubule-based movement, cytoskeleton-dependent intracellular transport, cytoskeleton organization and biogenesis, chromatin assembly or disassembly, DNA-dependent DNA replication, chromosome organization and biogenesis, DNA packaging, establishment and/or maintenance of chromatin architecture, regulation of progression through the cell cycle, regulation of the cell cycle, nucleobase, nucleoside, nucleotide and nucleic acid metabolism, chromatin assembly, macromolecule biosynthesis, intracellular transport, establishment of cellular localization, cellular localization, nucleosome assembly, macromolecule metabolism, or M-phase; genes involved in secondary metabolism or genes involved in seed and/or seed coat architecture.

Genes involved in the fatty acid biosynthesis or degradation include but are not limited to genes encoding an acyl-CoA synthetase, a glycerol-phosphate acyltransferase, an O-acyltransferase, a lyso-phosphatidic acid acyltransferase, a phosphatidic acid phosphatase, a diacylglycerol acyltransferase, an oleate desaturases, a linoleate desaturases, an acyl-CoA hydroxylase, an acyl-lipid hydroxylase, a fatty acid epoxidase, a phospholipid:sterol acyltransferase, a phospholipid:diacylglycerol acyltransferase, a diacylglycerol transacylase, a lysophosphatidylcholine acyltransferase, a phosphatidylcholine:diacylglycerol cholinephosphotransferase, an acyl-CoA elongase, an acyl-lipid elongase, a phosphatidylglycerol-phosphate synthetase, a phosphatidylglycerol-phosphate phosphatase, a CDP-diacylglycerol synthetase, a phosphatidylinositol synthase, a phosphatidylserine synthase, a choline kinase, an ethanolamine kinase, a CDP-choline synthetase, a CDP-ethanolamine synthetase, a phosphatidylserine decarboxylase, a lipoxygenase, a phospholipase, a lipase, a carboxylesterase, a fatty alcohol reductase, a wax ester synthase, a bifunctional acyltranferases/wax synthase, a ketoacyl-CoA synthase, a ketoacyl-CoA reductase, a hydroxylacyl-CoA dehydrase, an enoyl-CoA reductase, an alcohol-forming fatty acyl-CoA reductase, an aldehyde-forming fatty acyl-CoA reductase, an aldehyde decarbonylase, a wax ester hydrolase, a glycerol-3-P-dehydrogenase, a CDP-choline:1,2-diacylglycerol cholinephosphotransferase, an oxidase, a ketosphinganine reductase, a ceramide synthase, an acylglycerophosphorylcholine acyltransferase, an acylglycerol-phosphate acyltransferase, a phosphoethanolamine N-methyltransferase, a ceramide sphingobase desaturase, a glucosylceramide synthase, a acyl-ceramide synthase, a triacylglycerol lipase, a monoacylglycerol lipase, an acyl-CoA oxidase, an hydroxyacyl-CoA dehydrogenase, a dienoyl-CoA reductase, a fatty acid omega-alcohol oxidase, a monoacylglycerol lipase, an acyl-CoA oxidase, a hydroxyacyl-CoA dehydrogenase, a dienoyl-CoA reductase, a fatty acid omega-alcohol oxidase, a fatty acid/acyl-CoA transporter, a acyl-CoA dehydrogenase, a diacylglycerol-phosphate kinase, a lysophosphatidic acic phosphatase, a peroxygenase; a Δ4-desaturase; a Δ5-desaturase, a Δ6-desaturase; a Δ9-desaturase, a Δ12-desaturase or a A15-desaturase.

Genes involved in cell proliferation include but are not limited to genes encoding Da1 (Li et al., 2008, Genes Dev 22:1331, WO2015/067943), Da2, EOD1 or EOD3 (WO2015/022192, PCT/GB/2013/050072).

A "transcription termination and polyadenylation region" as used herein is a sequence that drives the cleavage of the nascent RNA, whereafter a poly(A) tail is added at the resulting RNA 3' end, functional in plant cells. Transcription termination and polyadenylation signals functional in plant cells include, but are not limited to, 3'nos, 3'35S, 3'his and 3'g7.

The term "protein" interchangeably used with the term "polypeptide" as used herein describes a group of molecules consisting of more than 30 amino acids, whereas the term "peptide" describes molecules consisting of up to 30 amino acids. Proteins and peptides may further form dimers, trimers and higher oligomers, i.e. consisting of more than one (poly)peptide molecule. Protein or peptide molecules forming such dimers, trimers etc. may be identical or non-identical. The corresponding higher order structures are, consequently, termed homo- or heterodimers, homo- or heterotrimers etc. The terms "protein" and "peptide" also refer to naturally modified proteins or peptides wherein the modification is effected e.g. by glycosylation, acetylation, phosphorylation and the like. Such modifications are well known in the art.

The term "heterologous" refers to the relationship between two or more nucleic acid or protein sequences that are derived from different sources. For example, a promoter is heterologous with respect to an operably linked DNA region, such as a coding sequence if such a combination is not normally found in nature. In addition, a particular sequence may be "heterologous" with respect to a cell or organism into which it is inserted (i.e. does not naturally occur in that particular cell or organism).

The term "recombinant gene" refers to any gene that contains: a) DNA sequences, including regulatory and coding sequences that are not found together in nature, or b) sequences encoding parts of proteins not naturally adjoined, or c) parts of promoters that are not naturally adjoined. Accordingly, a recombinant gene may comprise regulatory sequences and coding sequences that are derived from different sources, or comprise regulatory sequences, and coding sequences derived from the same source, but arranged in a manner different from that found in nature.

Any of the promoters and heterologous nucleic acid sequences described above may be provided in a recombinant vector. A recombinant vector typically comprises, in a 5' to 3' orientation: a promoter to direct the transcription of a nucleic acid sequence and a nucleic acid sequence. The recombinant vector may further comprise a 3' transcriptional terminator, a 3' polyadenylation signal, other untranslated nucleic acid sequences, transit and targeting nucleic acid sequences, selectable markers, enhancers, and operators, as desired. The wording "5' UTR" refers to the untranslated region of DNA upstream, or 5' of the coding region of a gene and "3' UTR" refers to the untranslated region of DNA downstream, or 3' of the coding region of a gene. Means for preparing recombinant vectors are well known in the art. Methods for making recombinant vectors particularly suited to plant transformation are described in US4971908, US4940835, US4769061 and US4757011. Typical vectors useful for expression of nucleic acids in higher plants are well known in the art and include vectors derived from the tumor-inducing (Ti) plasmid of *Agrobacterium tumefaciens.* One or more additional promoters may also be provided in the recombinant vector. These promoters may be operably linked, for example, without limitation, to any of the nucleic acid sequences described above. Alternatively, the promoters may be operably linked to other nucleic acid sequences, such as those encoding transit peptides, selectable marker proteins, or antisense sequences. These additional promoters may be selected on the basis of the cell type into which the vector will be inserted. Also, promoters which function in bacteria, yeast, and plants are all well taught in the art. The additional promoters may also be selected on the basis of their regulatory features. Examples of such features include enhancement of transcriptional activity, inducibility, tissue specificity, and developmental stage-specificity.

The recombinant vector may also contain one or more additional nucleic acid sequences. These additional nucleic acid sequences may generally be any sequences suitable for use in a recombinant vector. Such nucleic acid sequences include, without limitation, any of the nucleic acid sequences, and modified forms thereof, described above. The additional structural nucleic acid sequences may also be operably linked to any of the above described promoters. The one or more structural nucleic acid sequences may each be operably linked to separate promoters. Alternatively, the structural nucleic acid sequences may be operably linked to a single promoter (i.e., a single operon).

Yet another embodiment provides a host cell, such as an E. coli cell, an Agrobacterium cell, a yeast cell, an algal cell, or a plant cell, comprising the isolated nucleic acid according to the invention, or the recombinant gene according to the invention.

Other nucleic acid sequences may also be introduced into the host cell along with the promoter and structural nucleic acid sequence, e. g. also in connection with the vector of the invention. These other sequences may include 3' transcriptional terminators, 3' polyadenylation signals, other untranslated nucleic acid sequences, transit or targeting sequences, selectable markers, enhancers, and operators. Preferred nucleic acid sequences, including recombinant vectors, structural nucleic acid sequences, promoters, and other regulatory elements, are described above.

In further embodiments, a plant and a plant cell are provided comprising the recombinant gene according to the invention. Also described are seeds obtainable from the plant according to the invention. the plants or seeds according to the invention may be seed crop plants or seeds.

The plant cell or plant comprising the recombinant gene according to the invention can be a plant cell or a plant comprising a recombinant gene of which either the promoter, or the heterologous nucleic acid sequence operably linked to said promoter, are heterologous with respect to the plant cell. Such plant cells or plants may be transgenic plant in which the recombinant gene is introduced via transformation. Alternatively, the plant cell of plant may comprise the promoter according to the invention derived from the same species operably linked to a nucleic acid which is also derived from the same species, i.e. neither the promoter nor the operably linked nucleic acid is heterologous with respect to the plant cell, but the promoter is operably linked to a nucleic acid to which it is not linked in nature. A recombinant gene can be introduced in the plant or plant cell via transformation, such that both the promoter and the operably linked nucleotide are at a position in the genome in which they do not occur naturally. Alternatively, the promoter according to the invention can be integrated in a targeted manner in the genome of the plant or plant cell upstream of an endogenous nucleic acid encoding an expression product of interest, i.e. to modulate the expression pattern of an endogenous gene. The promoter that is integrated in a targeted manner upstream of an endogenous nucleic acid can be integrated in cells of a plant species from which it is originally derived, or in cells of a heterologous plant species. Alternatively, a heterologous nucleic acid can be integrated in a targeted manner in the genome of the plant or plant cell downstream of the promoter according to the invention, such that said heterologous nucleic acid is expressed seed-preferentially. Said heterologous nucleic acid is a nucleic acid which is heterologous with respect to the promoter, i.e. the combination of the promoter with said heterologous nucleic acid is not normally found in nature. Said heterologous nucleic acid may be a nucleic acid which is heterologous to said plant species in which it is inserted, but it may also naturally occur in said plant species at a different location in the plant genome. Said promoter or said heterologous nucleic acid can be integrated in a targeted manner in the plant genome via targeted sequence insertion, using, for example, the methods as described in WO2005/049842.

Plants comprising at least two recombinant genes according to the invention wherein the nucleic acid comprising seed- and funiculus-preferential promoter activity is different in each recombinant gene are, for example, plants comprising a first recombinant gene comprising a nucleotide sequence having at least 95% sequence identity to SEQ ID NO: 3 or a functional fragment thereof, and a second recombinant gene comprising a nucleotide sequence having at least 95% sequence identity to any one of SEQ ID NO: 4 to SEQ ID NO: 7 or a functional fragment thereof. It will be clear that, when the first recombinant gene comprises a nucleotide sequence having at least 95% sequence identity to SEQ ID NO: x or a functional fragment thereof, wherein SEQ ID NO: x is selected from any one of SEQ ID NO: 3 to SEQ ID NO: 7, the second recombinant gene may comprise a nucleotide sequence having at least 95% sequence identity to any one of the sequences according to the invention or a functional fragment thereof, except to SEQ ID NO: x. Said plants are suitable to express different genes with the same tissue-specificity, however without the negative features associated with the repeated use of one promoter, such as gene silencing or recombination of a vector comprising the recombinant genes. The at least two recombinant genes according to the invention may be present at one locus in the genome of said plant, and may be derived from the same transforming DNA molecule.

Plants according to the invention may comprise one or more recombinant genes according to the invention, but may in addition contain a recombinant gene comprising a nucleic acid comprising promoter activity which is preferential or specific to other plant tissues, such as apical meristem, flower buds, cotyledons, flowers, pods, roots, and leaves, operably linked to a nucleic acid sequence encoding an expression product of interest. The recombinant gene according to the invention and the recombinant gene comprising a nucleic acid comprising another promoter activity may be present at one locus and may be derived from the same transforming DNA molecule.

Yet another embodiment provides a method of producing a transgenic plant comprising the steps of (a) introducing or providing the recombinant gene according to the invention to a plant cell to create transgenic cells; and (b) regenerating transgenic plants from said transgenic cell.

"Introducing" in connection with the present application relates to the placing of genetic information in a plant cell or plant by artificial means. This can be effected by any method known in the art for introducing RNA or DNA into plant cells, protoplasts, calli, roots, tubers, seeds, stems, leaves, seedlings, embryos, pollen and microspores, other plant tissues, or whole plants. "Introducing" also comprises stably integrating into the plant's genome. Introducing the recombinant gene can be performed by transformation.

The term "transformation" herein refers to the introduction (or transfer) of nucleic acid into a recipient host such as a plant or any plant parts or tissues including plant cells, protoplasts, calli, roots, tubers, seeds, stems, leaves, seedlings, embryos and pollen. Plants containing the transformed nucleic acid sequence are referred to as "transgenic plants". Transformed, transgenic and recombinant refer to a host organism such as a plant into which a heterologous nucleic acid molecule (e.g. an expression cassette or a recombinant vector) has been introduced. The nucleic acid can be stably integrated into the genome of the plant.

As used herein, the phrase "transgenic plant" refers to a plant having an introduced nucleic acid stably introduced into a genome of the plant, for example, the nuclear or plastid genomes. In other words, plants containing transformed nucleic acid sequence are referred to as "transgenic plants". Transgenic and recombinant refer to a host organism such as a plant into which a heterologous nucleic acid molecule (e.g. the promoter, the chimeric gene or the vector as described herein) has been introduced. The nucleic acid can be stably integrated into the genome of the plant.

Transformation methods are well known in the art and include *Agrobacterium-*mediated transformation. Agrobacterium-mediated transformation of cotton has been described e.g. in US patent 5,004,863, in US patent 6,483,013 and WO2000/71733. Plants may also be transformed by particle bombardment: Particles of gold or tungsten are coated with DNA and then shot into young plant cells or plant embryos. This method also allows transformation of plant plastids. Viral transformation (transduction) may be used for transient or stable expression of a gene, depending on the nature of the virus genome. The desired genetic material is packaged into a suitable plant virus and the modified virus is allowed to infect the plant. The progeny of the infected plants is virus free and also free of the inserted gene. Suitable methods for viral transformation are described or further detailed e. g. in WO 90/12107, WO 03/052108 or WO 2005/098004. Further suitable methods well-known in the art are microinjection, electroporation of intact cells, polyethyleneglycol-mediated protoplast transformation, electroporation of protoplasts, liposome-mediated transformation, silicon-whiskers mediated transformation etc. Said transgene may be stably integrated into the genome of said plant cell, resulting in a transformed plant cell. The transformed plant cells obtained in this way may then be regenerated into mature fertile transformed plants.

Further provided is a method of effecting seed- and funiculus-preferential expression of a nucleic acid comprising introducing the recombinant gene according to the invention into the genome of a plant, or providing the plant according to the invention. Also provided is a method for altering seed properties of a plant or to produce a commercially relevant product in a plant, comprising introducing the recombinant gene according to the invention into the genome of a plant, or providing the plant according to the invention. In another embodiment, said plant is a seed crop plant.

"Seed properties" as used herein are properties of the seed. Seed properties can, for example, be seed yield, seed storage compound production, seed compound accumulation, seed nutrient accumulation; seed micronutrient accumulation; seed storage compound quality, seed compound composition, seed quality, biotic stress tolerance such as disease tolerance, abiotic stress tolerance, herbicide tolerance, seed dormancy, seed imbibition, seed germination, seed vigor. Seed storage compounds can, for example, be, seed oil, seed starch, or seed protein.

Seed properties may be modulated by modulating metabolic pathways, such as starch metabolism, sugar metabolism, inositol phosphate metabolism, glycolysis, amino acid biosynthesis, carbon metabolism, nucleotide metabolism, oxidative pentose phosphate cycle, fatty acid biosynthesis, protein synthesis, or phytate metabolism, and modulating secondary metabolism pathways. Another example is the methyl recycling metabolic activity impacting chromatin remodeling, phospholipid biosynthesis and cell wall lignification. Such metabolic pathways can be modulated by, for example, overexpressing or down-regulating a gene involved in one or more of the metabolic pathways using the seed- and funiculus-preferential promoter according to the invention.

Yield as used herein can comprise yield of the plant or plant part which is harvested, such as seed, including seed oil content, seed protein content, seed weight, seed number. Increased yield can be increased yield per plant, and increased yield per surface unit of cultivated land, such as yield per hectare. Yield can be increased by modulating, for example, by increasing seed size or oil content or indirectly by increasing the tolerance to biotic and abiotic stress conditions and decreasing seed abortion.

Quality as used herein can comprise quality of the seed or grain such as beneficial carbohydrate composition or level, beneficial amino acid composition or level, beneficial fatty acid composition or level, nutritional value, seed and fiber content.

Abiotic stress tolerance as used herein can comprise resistance to environmental stress factors such as drought, extreme (high or low) temperatures.

Biotic stress tolerance as used herein can comprise pest resistance, such as resistance or fungal, bacterial, bacterial or viral pathogens or insects.

Also provided is the use of the isolated nucleic acid according to the invention to regulate expression of an operably linked nucleic acid in a plant, and the use of the isolated nucleic acid according to the invention, or the recombinant gene according to the invention to alter seed properties of a plant or to produce a commercially relevant product in a plant.

Also provided is the use of the isolated nucleic acid according to the invention to identify other nucleic acids comprising seed- and funiculus-preferential promoter activity.

The promoters according to the invention can further be used to create hybrid promoters, i.e. promoters containing (parts of) one or more of the promoters(s) of the current invention and (parts of) other promoter which can be newly identified or known in the art. Such hybrid promoters may have optimized tissue specificity or expression level.

Yet another embodiment provides a method of producing food, feed, or an industrial product comprising (a) obtaining the plant or a part thereof, according to the invention; and (b) preparing the food, feed or industrial product from the plant or part thereof. Said food or feed is oil, meal, grain, starch, flour or protein, or said industrial product is biofuel, fiber, industrial chemicals, a pharmaceutical or a nutraceutical.

A "seed crop" or "seed crop plant" as used herein is a crop grown for its seeds or material derived from the seeds. Examples of seed crops are rice, maize, wheat, barley, millet, rye, oats, camelina, crambe, *Linum*, castor bean, calendula, safflower, sunflower, soybean, cotton, or *Brassica* species, such as *Brassica napus, Brassica juncea, Brassica carinata, Brassica rapa, Brassica oleracea,* and *Brassica nigra.*

"Brassicaceae" or "Brassicaceae plant" as used herein refers to plants belonging to the family of Brassicaceae plants, also called Cruciferae or mustard family. Examples of Brassicaceae are, but are not limited to, *Brassica* species, such as *Brassica napus*, *Brassica oleracea, Brassica rapa, Brassica carinata*, *Brassica nigra*, and *Brassica juncea*; *Raphanus* species, such as *Raphanus caudatus, Raphanus raphanistrum,* and *Raphanus sativus*; *Matthiola* species; *Cheiranthus* species; *Camelina* species, such as *Camelina sativa; Crambe* species, such as *Crambe abyssinica* and *Crambe hispanica*; *Eruca* species, such as *Eruca vesicaria*; *Sinapis* species such as *Sinapis alba*; *Diplotaxis* species; *Lepidium* species; *Nasturtium* species; *Orychophragmus* species; *Armoracia* species, *Eutrema* species; *Lepidium* species; and *Arabidopsis* species.

Said Brassicaceae plant can be a *Brassica* plant. "*Brassica* plant" refers to allotetraploid or amphidiploid *Brassica napus* (AACC, 2n=38), *Brassica juncea* (AABB, 2n=36), *Brassica carinata* (BBCC, 2n=34), or to diploid *Brassica rapa* (syn. *B. campestris*) (AA, 2n=20), *Brassica oleracea* (CC, 2n=18) or *Brassica nigra* (BB, 2n=16).

Crop plants of the *Brassica* species are, for example, *Brassica napus*, *Brassica juncea*, *Brassica carinata*, *Brassica rapa* (syn. *B. campestris*), *Brassica oleracea* or *Brassica nigra.*

The plants according to the invention may additionally contain an endogenous or a transgene, which confers herbicide resistance, such as the bar or pat gene, which confer resistance to glufosinate ammonium (Liberty®, Basta® or Ignite®) [EP 0 242 236 and EP 0 242 246]; or any modified EPSPS gene, such as the 2mEPSPS gene from maize [EP0 508 909 and EP 0 507 698 incorporated by reference], or glyphosate acetyltransferase, or glyphosate oxidoreductase, which confer resistance to glyphosate (RoundupReady®), or bromoxynitril nitrilase to confer bromoxynitril tolerance, or any modified AHAS gene, which confers tolerance to sulfonylureas, imidazolinones, sulfonylaminocarbonyltriazolinones, triazolopyrimidines or pyrimidyl(oxy/thio)benzoates, such as oilseed rape imidazolinone-tolerant mutants PM1 and PM2, currently marketed as Clearfield® canola. Further, the plants according to the invention may additionally contain an endogenous or a transgene which confers increased oil content or improved oil composition, such as a 12:0 ACP thioesteraseincrease to obtain high laureate, which confers pollination control, such as such as barnase under control of an anther-specific promoter to obtain male sterility, or barstar under control of an anther-specific promoter to confer restoration of male sterility, or such as the Ogura cytoplasmic male sterility and nuclear restorer of fertility.

The plants or seeds of the plants according to the invention may be further treated with a chemical compound, such as a chemical compound selected from the following lists: Herbicides: Clethodim, Clopyralid, Diclofop, Ethametsulfuron, Fluazifop, Glufosinate, Glyphosate, Metazachlor, Quinmerac, Quizalofop, Tepraloxydim, Trifluralin. Fungicides / PGRs: Azoxystrobin, N-[9-(dichloromethylene)-1,2,3,4-tetrahydro-1,4-methanonaphthalen-5-yl]-3-(difiuoromethyl)-1-methyl-1H-pyrazole-4-carboxamide (Benzovindiflupyr, Benzodiflupyr), Bixafen, Boscalid, Carbendazim, Carboxin, Chlormequat-chloride, Coniothryrium minitans, Cyproconazole, Cyprodinil, Difenoconazole, Dimethomorph, Dimoxystrobin, Epoxiconazole, Famoxadone, Fluazinam, Fludioxonil, Fluopicolide, Fluopyram, Fluoxastrobin, Fluquinconazole, Flusilazole, Fluthianil, Flutriafol, Fluxapyroxad, Iprodione, Isopyrazam, Mefenoxam, Mepiquat-chloride, Metalaxyl, Metconazole, Metominostrobin, Paclobutrazole, Penflufen, Penthiopyrad, Picoxystrobin, Prochloraz, Prothioconazole, Pyraclostrobin, Sedaxane, Tebuconazole, Tetraconazole, Thiophanate-methyl, Thiram, Triadimenol, Trifloxystrobin, Bacillus firmus, Bacillus firmus strain I-1582, Bacillus subtilis, Bacillus subtilis strain GB03, Bacillus subtilis strain QST 713, Bacillus pumulis, Bacillus. pumulis strain GB34. Insecticides: Acetamiprid, Aldicarb, Azadirachtin, Carbofuran, Chlorantraniliprole (Rynaxypyr), Clothianidin, Cyantraniliprole (Cyazypyr), (beta-)Cyfluthrin, gamma-Cyhalothrin, lambda-Cyhalothrin, Cypermethrin, Deltamethrin, Dimethoate, Dinetofuran, Ethiprole, Flonicamid, Flubendiamide, Fluensulfone, Fluopyram,Flupyradifurone, tau-Fluvalinate, Imicyafos, Imidacloprid, Metaflumizone, Methiocarb, Pymetrozine, Pyrifluquinazon, Spinetoram, Spinosad, Spirotetramate, Sulfoxaflor, Thiacloprid, Thiamethoxam, 1-(3-chloropyridin-2-yl)-N-[4-cyano-2-methyl-6-(methylcarbamoyl)phenyl]-3-{[5-(trifluoromethyl)-2H-tetrazol-2-yl]methyl}-1H-pyrazole-5-carboxamide, 1-(3-chloropyridin-2-yl)-N-[4-cyano-2-methyl-6-(methylcarbamoyl)phenyl]-3-{[5-(trifluoromethyl)-1H-tetrazol-1-yl]methyl}-1H-pyrazole-5-carboxamide, 1-{2-fluoro-4-methyl-5-[(2,2,2-trifluorethyl)sulfinyl]phenyl}-3-(trifluoromethyl)-1H-1,2,4-triazol-5-amine, (1E)-N-[(6-chloropyridin-3-yl)methyl]-N'-cyano-N-(2,2-difluoroethyl)ethanimidamide, Bacillus firmus, Bacillus firmus strain I-1582, Bacillus subtilis, Bacillus subtilis strain GB03, Bacillus subtilis strain QST 713, Metarhizium anisopliae F52.

Whenever reference to a "plant" or "plants" according to the invention is made, it is understood that also plant parts (cells, tissues or organs, seed pods, seeds, severed parts such as roots, leaves, flowers, pollen, etc.), progeny of the plants which retain the distinguishing characteristics of the parents, such as seed obtained by selfing or crossing, e.g. hybrid seed (obtained by crossing two inbred parental lines), hybrid plants and plant parts derived there from are encompassed herein, unless otherwise indicated.

The plant cells of the invention as well as plant cells generated according to the methods of the invention, may be non-propagating cells.

The obtained plants according to the invention can be used in a conventional breeding scheme to produce more plants with the same characteristics or to introduce the same characteristic in other varieties of the same or related plant species, or in hybrid plants. The obtained plants can further be used for creating propagating material. Plants according to the invention can further be used to produce gametes, seeds (including crushed seeds and seed cakes), seed oil, embryos, either zygotic or somatic, progeny or hybrids of plants obtained by methods of the invention. Seeds obtained from the plants according to the invention are also described herein.

"Creating propagating material", as used herein, relates to any means know in the art to produce further plants, plant parts or seeds and includes inter alia vegetative reproduction methods (e.g. air or ground layering, division, (bud) grafting, micropropagation, stolons or runners, storage organs such as bulbs, corms, tubers and rhizomes, striking or cutting, twin-scaling), sexual reproduction (crossing with another plant) and asexual reproduction (e.g. apomixis, somatic hybridization).

As used herein "comprising" is to be interpreted as specifying the presence of the stated features, integers, steps or components as referred to, but does not preclude the presence or addition of one or more features, integers, steps or components, or groups thereof. Thus, e.g., a nucleic acid or protein comprising a sequence of nucleotides or amino acids, may comprise more nucleotides or amino acids than the actually cited ones, i.e., be embedded in a larger nucleic acid or protein. A chimeric gene comprising a nucleic acid which is functionally or structurally defined, may comprise additional DNA regions etc.

Furthermore, the disclosed invention is expected to yield similar results in other seed crop plant species. Particularly, it is expected to drive seed- and funiculus-preferential expression in soybean. It is also expected to drive seed- and funiculus-preferential expression in wheat. The disclosed promoter may lead to a seed- and funiculus-preferential expression in cotton.

The sequence listing contained in the file named "BCS16-2003_ST25.txt", which is 45 kilobytes (size as measured in Microsoft Windows®), contains 24 sequences SEQ ID NO: 1 through SEQ ID NO: 24 is filed herewith by electronic submission and is incorporated by reference herein.

In the description and examples, reference is made to the following sequences:

### SEQUENCES

SEQ ID NO: 1: nucleotide sequence of the T-DNA Pslp1 BnA::GUS.
SEQ ID NO: 2: nucleotide sequence of the T-DNA Pslp1 intron BnA::GUS.
SEQ ID NO: 3: nucleotide sequence of the promoter region Pslp1 intron BnA.
SEQ ID NO: 4: nucleotide sequence of the promoter region Pslp1 intron BnC.
SEQ ID NO: 5: nucleotide sequence of the promoter region Pslp1 intron Br.
SEQ ID NO: 6: nucleotide sequence of the promoter region Pslp1 intron Bo.
SEQ ID NO: 7: nucleotide sequence of the promoter region Pslp1 intron BjA.
SEQ ID NO: 8: amino acid sequence of SLP1 BnA.
SEQ ID NO: 9: amino acid sequence of SLP1 BnC.
SEQ ID NO: 10: amino acid sequence of SLP1 Br.
SEQ ID NO: 11: amino acid sequence of SLP1 Bo.
SEQ ID NO: 12: amino acid sequence of SLP1 BjA.
SEQ ID NO: 13: nucleotide sequence of the coding sequence of SLP1 BnA.
SEQ ID NO: 14: nucleotide sequence of the coding sequence of SLP1 BnC.
SEQ ID NO: 15: nucleotide sequence of the coding sequence of SLP1 Br.
SEQ ID NO: 16: nucleotide sequence of the coding sequence of SLP1 Bo.
SEQ ID NO: 17: nucleotide sequence of the coding sequence of SLP1 BjA.
SEQ ID NO: 18: Promoter consensus sequence.
SEQ ID NO: 19: UTR consensus sequence 1.
SEQ ID NO: 20: UTR consensus sequence 2.
SEQ ID NO: 21: UTR consensus sequence 3.
SEQ ID NO: 22: Intron consensus sequence 1.
SEQ ID NO: 23: Intron consensus sequence 2.
SEQ ID NO: 24: Intron consensus sequence 3.

### EXAMPLES

Unless stated otherwise in the Examples, all recombinant DNA techniques are carried out according to standard protocols as described in Sambrook and Russell (2001) Molecular Cloning: A Laboratory Manual, Third Edition, Cold Spring Harbor Laboratory Press, NY, in Volumes 1 and 2 of Ausubel et al. (1994) Current Protocols in Molecular Biology, Current Protocols, USA and in Volumes I and II of Brown (1998) Molecular Biology LabFax, Second Edition, Academic Press (UK). Standard materials and methods for plant molecular work are described in Plant Molecular Biology Labfax (1993) by R.D.D. Croy, jointly published by BIOS Scientific Publications Ltd (UK) and Blackwell Scientific Publications, UK. Standard materials and methods for polymerase chain reactions can be found in Dieffenbach and Dveksler (1995) PCR Primer: A Laboratory Manual, Cold Spring Harbor Laboratory Press, and in McPherson at al. (2000) PCR - Basics: From Background to Bench, First Edition, Springer Verlag, Germany.

### Example 1 - Generation of expression constructs with the PsIp1BnA promoter fragments of Brassica napus operably linked to the GUS reporter gene (Pslp1 BnA:GUS and Pslp1 intron BnA::GUS)

Because the SLP1 gene structure contains an intron immediately after the translation start (Figure 1), it was hypothesized that this intron may be required for the promoter activity. Therefore expression constructs with or without this first intron were generated.

The promoter sequence of the *Brassica napus* Pslp1 BnA promoter (position 1 to 1414 of SEQ ID NO: 3 or 5' to 3' position 139 to 1552 of SEQ ID NO:1) isolated from an *in house* developed *Brassica napus* line, the GUS gene (β-glucuronidase) with intron (5' to 3' position 1553 to 3553 of SEQ ID NO: 1) and a fragment of the 3' untranslated region (UTR) of the TL-DNA gene 7 of Agrobacterium tumefaciens octopine (5' to 3' position 3610 to 3813 of SEQ ID NO: 1) were assembled in a vector which contains the bar selectable marker cassette (position 3894 to 6404 of SEQ ID NO: 1) to result in the T-DNA Pslp1 BnA::GUS (SEQ ID NO: 1).

The promoter sequence of the *Brassica napus* slp1BnA promoter plus the first intron (SEQ ID NO: 3 or 5' to 3' position 139 to 2193 of SEQ ID NO: 2) isolated from an *in house* developed *Brassica napus* line, the GUS gene (β-glucuronidase) with intron (5' to 3' position 2194 to 4191 of SEQ ID NO: 2) and a fragment of the 3' untranslated region (UTR) of the TL-DNA gene 7 of Agrobacterium tumefaciens octopine (5' to 3' position 4248 to 4451 of SEQ ID NO: 2) were assembled in a vector which contains the bar selectable marker cassette (position 4532 to 7042 of SEQ ID NO: 2) to result in the T-DNA Pslp1 intron BnA::GUS (SEQ ID NO: 2).

### Example 2 - Generation of transgenic plants comprising the Pslp1 BnA::GUS or Pslp1 intron BnA::GUS

In a next step the recombinant vectors comprising the expression cassettes of example 1, i. e. Pslp1 BnA::GUS and Pslp1 intron BnA::GUS, were used to stably transform *Brassica napus.*

### Example 3 - In planta expression pattern of Pslp1BnA::GUS and Pslp1 BnAintron::GUS in Brassica napus

The *in planta* expression pattern of Pslp1 BnA::GUS and Pslp1 intron BnA::GUS in the different tissues of *Brassica napus* seeds was monitored according to the method of Jasik et al. 2011.

Figure 2 provides the semi quantitative assessment of the GUS labelling in the seed coat and the embryo of transgenic lines carrying either the Pslp1 BnA::GUS or Pslp1 intron BnA::GUS T-DNAs. For both constructs, the GUS staining is detected in both embryos and seed coat. For both constructs, the intensity of the staining in the embryo increases as the embryo progresses through the developmental stages, in other words, the expression level is higher at late stage than at early stage. In contrast, in the seed coat, the labelling is the strongest at early stages and then decreases as it progresses towards maturation. Though both constructs confer the same expression pattern, lines carrying the T-DNA Pslp1 intron BnA::GUS have a moderate expression level while the lines carrying the T-DNA Pslp1BnA::GUS have a weak expression level. The presence of the intron after the translation start ATG increases the expression level by at least 3 fold, up to more than 10 fold.

Figure 3 shows the GUS labelling of the reporter gene in the endosperm and seed coat at early (panels A and B) and late (panel C) seed developmental stages. At early stage, both endosperm and seed coat are labelled, with about the same intensity. The close-up view shows that the outer integument of the seed coat is more intensely stained than the inner integument. The activity of the Pslp1 intron BnA promoter fragment is moderate in the endosperm and seed coat at early stage. The close up view on the seed coat and endosperm at late stage show that the GUS labelling occurs in the outer integument but not in the inner integument, nor in the endosperm residues. Figure 3 further shows the GUS labelling of the reporter gene in the funiculus. The same result was obtained with the lines carrying the Pslp1 BnA::GUS T-DNA though with a weaker intensity.

Some GUS activity was also detected in the assessed non-seed tissues, namely stem, young pod, flowers (receptacle and peduncle) and leaves, though to a lower level, at least 2 fold weaker than in the seed and the funiculus tissues, thereby confirming the seed- and funiculus-preference of the promoter Pslp1 intron BnA.

### Example 4 - Identification of the second Brassica napus copy of SLP1 and of the orthologues of SLP1 in Brassica rapa, Brassica oleracea and Brassica juncea

The sequences of the second *Brassica napus* copy of SLP1 as well as the orthologues in *Brassica rapa, Brassica oleracea* and *Brassica juncea* were obtained by blasting the coding sequence of the SLP1 BnA against an *in-house* database of *Brassica napus, Brassica rapa, Brassica oleracea* and *Brassica juncea* sequences.

The nucleotide sequences obtained in this way are given in SEQ ID NO: 13 to SEQ ID NO: 17. These nucleotide sequences were translated into amino acid sequences, given respectively in SEQ ID NO: 8 to SEQ ID NO: 12.

In *Brassica rapa,* the result indicated that 2 copies of SLP1 are present in the genome, on two different chromosomes. As the nucleotide sequences of the two genomic fragments comprising the promoter and the genes are 100% identical, the nucleotide and amino acid sequences are provided only once. In *Brassica juncea,* no start codon was identified for the B copy of SLP1. This copy was therefore considered a pseudogene and was not further studied.

Figure 6 shows the alignment of the retrieved amino acid sequence. Any two of these sequences share at least 97% sequence identity.

### Examples 5 - RNA isolation from different Brassica tissues

The following tissues were isolated from *Brassica napus*:
a. Apical meristem 33 days after sowing (DAS) (including smallest leaves) (AM33)
b. Big flower buds (> 5 mm) 42 DAS (BFB42)
c. Cotyledons (with hypocotyl) 10 DAS (CTYL10)
d. Open flowers 52 DAS (OF52)
e. Pods 14-20 DAS (Pod2)
f. Pods 21-25 DAS (Pod3)
g. Roots 14 DAS (Ro2w)
h. Small flower buds ≤ 5 mm 42 DAS (SFB42)
i. Seeds 14-20 days after flowering (DAF) (Seed2)
j. Seeds 21-25 DAF (Seed3)
k. Seeds 26-30 DAF (Seed4)
l. Seeds 31-35 DAF (Seed5)
m. Seeds 42 DAF (Seed6)
n. Seeds 49 DAF (Seed7)
o. Stem 14 DAS (St2w)
p. Stem 33 DAS (St5w)
q. Young leaf 33 DAS (≤ 3 cm leaf next to apical meristem) (YL33)

The following tissues were isolated from *Brassica juncea*:
a. Apical meristem 22 days after sowing (DAS) (including smallest leaves) (AM22)
b. Big flower buds (> 5 mm) 35 DAS (BFB35)
c. Cotyledons (with hypocotyl) 8 DAS (CTYL8)
d. Open flowers 35 DAS (OF35)
e. Pods 14-20 DAS (Pod2)
f. Pods 21-25 DAS (Pod3)
g. Pods 26-30 DAS (Pod4)
h. Pods 31-35 DAS (Pod5)
i. Roots 14 DAS (Ro2w)
j. Small flower buds ≤ 5 mm 35 DAS (SFB35)
k. Seeds 14-20 days after flowering (DAF) (Seed2)
l. Seeds 21-25 DAF (Seed3)
m. Seeds 26-30 DAF (Seed4)
n. Seeds 31-35 DAF (Seed5)
o. Seeds 42 DAF (Seed6)
p. Seeds 49 DAF (Seed7)
q. Stem 14 DAS (St2w)
r. Stem 22 DAS (St3w)
s. Young leaf 22 DAS (≤ 3 cm leaf next to apical meristem) (YL22)
t. Old leaf 22 DAS (OL22)

Total RNA from the different tissues was isolated according to standard methods.

In our growth conditions, the correspondence between embryo developmental stages and the selected time points is as follows:
a. Between 10 and 13 DAF: torpedo stage
b. Seed2 or between 14 and 20 DAF: "walking stick" cotyledon stage
c. Seed3 or between 21 and 25 DAF: curled cotyledon stage
d. Seed4 and Seed5 or between 26 and 35 DAF: green cotyledon stage
e. Seed6 and Seed7 or after 36 DAF: mature embryo

### Example 6 - In silico expression analyses of the different copies of SLP1 of Brassica napus and their orthologues in Brassica rapa, Brassica oleracea and Brassica juncea

Figure 5 shows the relative expression levels of the endogenous transcripts of the different *Brassica napus* (A and B), *Brassica oleracea* (C), *Brassica rapa* (D) and *Brassica juncea* (E) copies of SLP1 in different tissues, as isolated in Example 5.

The SLP1 BnA transcript (panel A) is clearly detected in the seed tissues (Seed2 to Seed7) and only barely detectable in other tissues. This result confirms, as determined *in planta*, that Pslp1 intron BnA has seed-preferential promoter activity.

The SLP1 BnC transcript (panel B) is clearly detected in the seed tissues (Seed2 to Seed7) and only barely detectable in the other tissues. This result confirms, as determined *in planta*, that Pslp1 intron BnC has seed-preferential promoter activity.

The SLP1 Br transcript (panel C) is clearly detected in the seed tissues (Seed2 to Seed7) and only barely detectable in the other tissues. This result confirms, as determined *in planta*, that Pslp1 intron Br has seed-preferential promoter activity.

The SLP1 Bo transcript (panel D) is clearly detected in the seed tissues (Seed2 to Seed7) and only barely detectable in the other tissues. This result confirms, as determined *in planta*, that Pslp1 intron Bo has seed-preferential promoter activity.

The SLP1 BjA transcript (panel E) is clearly detected in the seed tissues (Seed2, Seed4, Seed6 and Seed7) and only barely detectable in the other tissues. This result confirms, as determined *in planta*, that Pslp1 intron BjA has seed-preferential promoter activity.

### Example 7 - Sequence analysis of the promoters and first intron of the SLP1 genes from Brassica rapa, Brassica juncea, Brassica oleracea and Brassica napus

For the different SLP1 gene identified, the genomic DNA sequence upstream of the translation start and including the first intron was retrieved from an *in-house* database of *Brassica napus, Brassica rapa, Brassica oleracea* and *Brassica juncea* sequences. The nucleotide sequences obtained in this way are given in SEQ ID NO: 4 to SEQ ID NO: 7.

Figure 6 shows the alignment of the about 400 bp sequence upstream of the translation start of the promoter sequences (SEQ ID NO: 3 to SEQ ID NO: 7). These promoters share a surprisingly high level of conservation in this region. Four consensus sequences were identified. The promoters described herein each comprise the following consensus sequence:
a. Promoter consensus sequence is given in SEQ ID NO: 18;
b. UTR consensus sequence 1 is given in SEQ ID NO: 19;
c. UTR consensus sequence 2 is given in SEQ ID NO: 20;
d. UTR consensus sequence 3 is given in SEQ ID NO: 21.

The presence of these consensus sequences in all analyzed promoter sequences described herein indicate that these consensus sequences are required for the observed seed- and funiculus-preferential expression pattern.

Consequently, as Pslp1 BnC (position 1 to 1364 of SEQ ID NO: 4) sequence comprises the promoter consensus sequence and the 3 UTR consensus sequences, it can be concluded that it has seed- and funiculus-preferential promoter activity. As Pslp1 intron BnC (SEQ ID NO: 4) sequence comprises the promoter consensus sequence and the 3 UTR consensus sequences, it can also be concluded that it has seed- and funiculus-preferential promoter activity. As Pslp1 Br (position 1 to 1365 of SEQ ID NO: 5) sequence comprises the promoter consensus sequence and the 3 UTR consensus sequences, it can also be concluded that it has seed- and funiculus-preferential promoter activity. As Pslp1 intron Br (SEQ ID NO: 5) sequence comprises the promoter consensus sequence and the 3 UTR consensus sequences, it can also be concluded that it has seed- and funiculus-preferential promoter activity. As Pslp1 Bo (position 1 to 1358 of SEQ ID NO: 6) sequence comprises the promoter consensus sequence and the 3 UTR consensus sequences, it can be concluded that it has seed- and funiculus-preferential promoter activity. As Pslp1 intron Bo (SEQ ID NO: 6) sequence comprises the promoter consensus sequence and the 3 UTR consensus sequences, it can be concluded that it has seed- and funiculus-preferential promoter activity. As Pslp1 BjA (position 1 to 1363 of SEQ ID NO: 7) sequence comprises the promoter consensus sequence and the 3 UTR consensus sequences, it can be concluded that it has seed- and funiculus-preferential promoter activityAs Pslp1 intron BjA (SEQ ID NO: 7) sequence comprises the promoter consensus sequence and the 3 UTR consensus sequences, it can be concluded that it has seed- and funiculus-preferential promoter activity.

More generally, these results indicate that a *Brassica* promoter comprising the promoter consensus sequence and the 3 UTR consensus sequences would have seed- and funiculus-preferential promoter activity.

Figure 7 shows the alignment of the first about 300 bp of the intron sequence of the promoter sequences (SEQ ID NO: 3 to SEQ ID NO: 7). This intron shares a surprisingly high level of conservation in this region. Three consensus sequences were identified in this first intron. The promoters described herein each comprise the following consensus sequence:
a. Intron consensus sequence 1 is given in SEQ ID NO: 22;
b. Intron consensus sequence 2 is given in SEQ ID NO: 23;
c. Intron consensus sequence 3 is given in SEQ ID NO: 24.

The presence of these consensus sequences in all analyzed intron sequences described herein indicates that these consensus sequences may be required for the observed increased expression level compared to the expression level achieved without the first intron.

Consequently, as the intron in Pslp1 intron BnC (position 1368 to 1650 of SEQ ID NO: 4) comprises the intron consensus sequences 1 to 3, it can be concluded that Pslp1 intron BnC has higher (at least 3 fold, up to 10 fold) seed- and funiculus-preferential promoter activity than Pslp1 BnC (position 1 to 1364 of SEQ ID NO: 4). As the intron in Pslp1 intron Br (position 1369 to 2001 of SEQ ID NO: 5) comprises the intron consensus sequences 1 to 3, it can be concluded that Pslp1 intron Br (SEQ ID NO: 5) has higher (at least 3 fold, up to 10 fold) seed- and funiculus-preferential promoter activity than Pslp1 Br (position 1 to 1365 of SEQ ID NO: 5). As the intron in Pslp1 intron Bo (position 1362 to 1647 of SEQ ID NO: 6) comprises the intron consensus sequences 1 to 3, it can be concluded that Pslp1 intron Bo (SEQ ID NO: 6) has higher (at least 3 fold, up to 10 forld) seed- and funiculus-preferential promoter activity than Pslp1 Bo (position 1 to 1358 of SEQ ID NO: 6). As the intron in Pslp1 intron BjA (position 1367 to 2001 of SEQ ID NO: 7) comprises the intron consensus sequences 1 to 3, it can be concluded that Pslp1 intron BjA (SEQ ID NO: 7) has higher (at least 3 fold, up to 10 fold) seed- and funiculus-preferential promoter activity than Pslp1 BjA (position 1 to 1363 of SEQ ID NO: 7).

More generally, these results indicate that a *Brassica* promoter comprising a intron comprising the intron consensus sequences 1 to 3 would have higher (at least 3 fold, up to 10 fold) promoter activity than the same *Brassica* promoter without such intron.

## Claims

1. An isolated nucleic acid having seed- and funiculus-preferential promoter activity selected from the group consisting of:
a. a nucleic acid comprising the nucleotide sequence selected from the group:
i. the nucleotide sequence from position 1 to position 1414 of SEQ ID NO: 3 or a functional fragment thereof comprising at least the nucleotide sequence from position 1014 to position 1414 of SEQ ID NO: 3,
ii. the nucleotide sequence from position 1 to position 1364 of SEQ ID NO: 4 or a functional fragment thereof comprising at least the nucleotide sequence from position 964 to position 1364 of SEQ ID NO: 4,
iii. the nucleotide sequence from position 1 to position 1365 of SEQ ID NO: 5 or a functional fragment thereof comprising at least the nucleotide sequence from position 965 to position 1365 of SEQ ID NO: 5,
iv. the nucleotide sequence from position 1 to position 1358 of SEQ ID NO: 6 or a functional fragment thereof comprising at least the nucleotide sequence from position 958 to position 1358 of SEQ ID NO: 6, and
v. the nucleotide sequence from position 1 to position 1363 of SEQ ID NO: 7 or a functional fragment thereof comprising at least the nucleotide sequence from position 963 to position 1363 of SEQ ID NO: 7,
and
b. a nucleic acid comprising a nucleotide sequence having at least 80% sequence identity to any one of a.

2. The nucleic acid according to claim 1 comprising:
a. the nucleotide sequence of SEQ ID NO: 18;
b. the nucleotide sequence of SEQ ID NO: 19;
c. the nucleotide sequence of SEQ ID NO: 20; and
d. the nucleotide sequence of SEQ ID NO: 21.

3. The nucleic acid according to claim 1 or 2 comprising an intron, the nucleotide sequence of which is selected from the group of:
a. a nucleotide sequence selected from the group:
i. the nucleotide sequence from position 1418 to position 2055 of SEQ ID NO: 3,
ii. the nucleotide sequence from position 1368 to position 1650 of SEQ ID NO: 4,
iii. the nucleotide sequence from position 1369 to position 2001 of SEQ ID NO: 5,
iv. the nucleotide sequence from position 1362 to position 1647 of SEQ ID NO: 6, and
v. the nucleotide sequence from position 1367 to position 2001 of SEQ ID NO: 7,
or a functional fragment thereof; and
b. a nucleic acid comprising a nucleotide sequence having at least 80% sequence identity to any one of a., or a functional fragment thereof.

4. The nucleic acid according to claim 3 comprising:
a. the nucleotide sequence of SEQ ID NO: 22;
b. the nucleotide sequence of SEQ ID NO: 23; and
c. the nucleotide sequence of SEQ ID NO: 24
in the intron sequence.

5. The nucleic acid according to claim 3 or 4 having higher seed- and funiculus-preferential promoter activity than the nucleic acid according to claim 1 or 2.

6. The isolated nucleic acid according to any one of claims 1 to 5 selected from the group:
a. a nucleic acid comprising any one of SEQ ID NOs: 3 to 7, or a functional fragment thereof, and
b. a nucleic acid comprising a nucleotide sequence having at least 80% sequence identity to any one of SEQ ID NOs: 3 to 7, or a functional fragment thereof.

7. A recombinant gene comprising the nucleic acid according to any one of claims 1 to 6 operably linked to a heterologous nucleic acid sequence encoding an expression product of interest, and optionally a transcription termination and polyadenylation sequence, preferably a transcription termination and polyadenylation region functional in plants.

8. The recombinant gene according to claim 7, wherein the expression product of interest is an RNA molecule capable of modulating the expression of a gene or is a protein.

9. A plant, a seed or a host cell comprising the recombinant gene of claim 7 or 8.

10. The plant or the seed according to claim 9, comprising at least two recombinant genes according to claim 7, wherein the nucleic acid according to any one of claims 1 to 6, and, optionally, the heterologous nucleic acid sequence, is different in each recombinant gene.

11. Method of producing a transgenic plant comprising the steps of:
a. introducing or providing the recombinant gene according to claim 7 or 8 to a plant cell to create transgenic cells; and
b. regenerating transgenic plants from said transgenic cell.

12. Method of effecting seed- and funiculus-preferential expression of a nucleic acid comprising introducing the recombinant gene according to claim 7 or 8 into the genome of a plant, or providing the plant according to claim 9 or 10.

13. Method for altering seed properties of a plant or to produce a commercially relevant product in a plant, said method comprising introducing the recombinant gene according to claim 7 or 8 into the genome of a plant, or providing the plant according to claim 9 or 10.

14. Use of the isolated nucleic acid according to any one of claims 1 to 6 to regulate expression of an operably linked nucleic acid in a plant or to identify other nucleic acids comprising seed- and funiculus-preferential promoter activity.

15. Use of the isolated nucleic acid according to any one of claims 1 to 6, or the recombinant gene according to claim 7 or 8 to alter seed properties of a plant or to produce a commercially relevant product in a plant.

16. A method of producing food, feed, or an industrial product comprising
a) obtaining the plant or a part thereof, according to claim 9 or 10; and
b) preparing the food, feed or industrial product from the plant or part thereof
wherein
a. the food or feed is oil, meal, grain, starch, flour or protein; or
b. the industrial product is biofuel, fiber, industrial chemicals, a pharmaceutical or a nutraceutical.

## Patentansprüche

1. Isolierte Nukleinsäure mit in Samen und Funiculus bevorzugter Promotoraktivität, ausgewählt aus der Gruppe bestehend aus:
a. einer Nukleinsäure, die die aus der folgenden Gruppe ausgewählte Nukleotidsequenz umfasst:
i. die Nukleotidsequenz von Position 1 bis Position 1414 von SEQ ID NO: 3 oder ein funktionelles Fragment davon, das wenigstens die Nukleotidsequenz von Position 1014 bis Position 1414 von SEQ ID NO: 3 umfasst,
ii. die Nukleotidsequenz von Position 1 bis Position 1364 von SEQ ID NO: 4 oder ein funktionelles Fragment davon, das wenigstens die Nukleotidsequenz von Position 964 bis Position 1364 von SEQ ID NO: 4 umfasst,
iii. die Nukleotidsequenz von Position 1 bis Position 1365 von SEQ ID NO: 5 oder ein funktionelles Fragment davon, das wenigstens die Nukleotidsequenz von Position 965 bis Position 1365 von SEQ ID NO: 5 umfasst,
iv. die Nukleotidsequenz von Position 1 bis Position 1358 von SEQ ID NO: 6 oder ein funktionelles Fragment davon, das wenigstens die Nukleotidsequenz von Position 958 bis Position 1358 von SEQ ID NO: 6 umfasst, und
v. die Nukleotidsequenz von Position 1 bis Position 1363 von SEQ ID NO: 7 oder ein funktionelles Fragment davon, das wenigstens die Nukleotidsequenz von Position 963 bis Position 1363 von SEQ ID NO: 7 umfasst,
und
b. einer Nukleinsäure, die eine Nukleotidsequenz mit wenigstens 80% Sequenzidentität mit einer unter a. umfasst.

2. Nukleinsäure nach Anspruch 1, umfassend:
a. die Nukleotidsequenz unter SEQ ID NO: 18;
b. die Nukleotidsequenz unter SEQ ID NO: 19;
c. die Nukleotidsequenz unter SEQ ID NO: 20; und
d. die Nukleotidsequenz unter SEQ ID NO: 21.

3. Nukleinsäure nach Anspruch 1 oder 2, umfassend ein Intron, dessen Nukleotidsequenz ausgewählt ist aus der Gruppe:
a. einer aus der folgenden Gruppe ausgewählten Nukleotidsequenz:
i. die Nukleotidsequenz von Position 1418 bis Position 2055 von SEQ ID NO: 3,
ii. die Nukleotidsequenz von Position 1368 bis Position 1650 von SEQ ID NO: 4,
iii. die Nukleotidsequenz von Position 1369 bis Position 2001 von SEQ ID NO: 5,
iv. die Nukleotidsequenz von Position 1362 bis Position 1647 von SEQ ID NO: 6 und
v. die Nukleotidsequenz von Position 1367 bis Position 2001 von SEQ ID NO: 7,
oder einem funktionellen Fragment davon; und
b. einer Nukleinsäure, die eine Nukleotidsequenz mit wenigstens 80% Sequenzidentität mit einer unter a. oder ein funktionelles Fragment davon umfasst.

4. Nukleinsäure nach Anspruch 3, umfassend:
a. die Nukleotidsequenz unter SEQ ID NO: 22;
b. die Nukleotidsequenz unter SEQ ID NO: 23; und
c. die Nukleotidsequenz unter SEQ ID NO: 24 in der Intronsequenz.

5. Nukleinsäure nach Anspruch 3 oder 4, mit höherer in Samen und Funiculus bevorzugter Promotoraktivität als die Nukleinsäure nach Anspruch 1 oder 2.

6. Isolierte Nukleinsäure nach einem der Ansprüche 1 bis 5, ausgewählt aus der folgenden Gruppe:
a. eine Nukleinsäure, die eine von SEQ ID NO: 3 bis 7 oder ein funktionelles Fragment davon umfasst, und
b. eine Nukleinsäure, die eine Nukleotidsequenz mit wenigstens 80% Sequenzidentität mit einer von SEQ ID NO: 3 bis 7 oder ein funktionelles Fragment davon umfasst.

7. Rekombinantes Gen, umfassend die Nukleinsäure nach einem der Ansprüche 1 bis 6 in operativer Verknüpfung mit einer heterologen Nukleinsäuresequenz, die ein Expressionsprodukt von Interesse codiert, und gegebenenfalls einer Transkriptionsterminations- und Polyadenylierungssequenz, bevorzugt einer in Pflanzen funktionsfähigen Transkriptionsterminations- und Polyadenylierungsregion.

8. Rekombinantes Gen nach Anspruch 7, wobei es sich bei dem Expressionsprodukt von Interesse um ein RNA-Molekül mit der Fähigkeit zur Modulation der Expression eines Gens oder um ein Protein handelt.

9. Pflanze, Samen oder Wirtszelle, umfassend das rekombinante Gen gemäß Anspruch 7 oder 8.

10. Pflanze oder Samen nach Anspruch 9, umfassend wenigstens zwei rekombinante Gene nach Anspruch 7, wobei die Nukleinsäure nach einem der Ansprüche 1 bis 6, und gegebenenfalls die heterologe Nukleinsäuresequenz in jedem rekombinanten Gen unterschiedlich ist.

11. Verfahren zur Erzeugung einer transgenen Pflanze, umfassend die Schritte:
a. Einführen oder Bereitstellen des rekombinanten Gens nach Anspruch 7 oder 8 inbeziehungsweise an eine Pflanzenzelle, so dass transgene Zellen entstehen; und
b. Regenerieren transgener Pflanzen aus der transgenen Zelle.

12. Verfahren zur Bewirkung in Samen und Funiculus bevorzugter Expression einer Nukleinsäure, umfassend Einführen des rekombinanten Gens nach Anspruch 7 oder 8 in das Genom einer Pflanze oder Bereitstellen der Pflanze nach Anspruch 9 oder 10.

13. Verfahren zur Veränderung von Sameneigenschaften einer Pflanze oder zum Erzeugen eines kommerziell relevanten Produkts in einer Pflanze, wobei das Verfahren Einführen des rekombinanten Gens nach Anspruch 7 oder 8 in das Genom einer Pflanze oder Bereitstellen der Pflanze nach Anspruch 9 oder 10 umfasst.

14. Verwendung der isolierten Nukleinsäure nach einem der Ansprüche 1 bis 6 zur Regulierung der Expression einer in operativer Verknüpfung stehenden Nukleinsäure in einer Pflanze oder zur Identifizierung anderer Nukleinsäuren, die in Samen und Funiculus bevorzugte Promotoraktivität umfassen.

15. Verwendung der isolierten Nukleinsäure nach einem der Ansprüche 1 bis 6 oder des rekombinanten Gens nach Anspruch 7 oder 8 zum Verändern von Sameneigenschaften einer Pflanze oder zum Erzeugen eines kommerziell relevanten Produkts in einer Pflanze.

16. Verfahren zur Erzeugung eines Lebensmittels, Futtermittels oder Industrieprodukts, umfassend
a) Gewinnen der Pflanze oder eines Teils davon nach Anspruch 9 oder 10; und
b) Herstellen des Lebensmittel-, Futtermittel- oder Industrieprodukts aus der Pflanze bzw. dem Teil davon,
wobei
a. es sich bei dem Lebensmittel oder Futtermittel um Öl, Schrotmehl, Korn, Stärke, Mehl oder Protein handelt; oder
b. es sich bei dem Industrieprodukt um Biokraftstoff, Faser, Industriechemikalien, ein Pharmazeutikum oder ein Nutrazeutikum handelt.

## Revendications

1. Acide nucléique isolé ayant une activité de promoteur préférentielle dans la graine et le funicule choisi dans le groupe constitué par :
a. un acide nucléique comprenant la séquence nucléotidique choisie dans le groupe suivant :
i. la séquence nucléotidique de la position 1 jusqu'à la position 1414 de la SEQ ID n° : 3 ou un fragment fonctionnel de celle-ci comprenant au moins la séquence nucléotidique de la position 1014 jusqu'à la position 1414 de la SEQ ID n° : 3,
ii. la séquence nucléotidique de la position 1 jusqu'à la position 1364 de la SEQ ID n° : 4 ou un fragment fonctionnel de celle-ci comprenant au moins la séquence nucléotidique de la position 964 jusqu'à la position 1364 de la SEQ ID n° : 4,
iii. la séquence nucléotidique de la position 1 jusqu'à la position 1365 de la SEQ ID n° : 5 ou un fragment fonctionnel de celle-ci comprenant au moins la séquence nucléotidique de la position 965 jusqu'à la position 1365 de la SEQ ID n° : 5,
iv. la séquence nucléotidique de la position 1 jusqu'à la position 1358 de la SEQ ID n° : 6 ou un fragment fonctionnel de celle-ci comprenant au moins la séquence nucléotidique de la position 958 jusqu'à la position 1358 de la SEQ ID n° : 6, et
v. la séquence nucléotidique de la position 1 jusqu'à la position 1363 de la SEQ ID n° : 7 ou un fragment fonctionnel de celle-ci comprenant au moins la séquence nucléotidique de la position 963 jusqu'à la position 1363 de la SEQ ID n° : 7,
et
b. un acide nucléique comprenant une séquence nucléotidique ayant une identité de séquence d'au moins 80 % avec un quelconque élément du a.

2. Acide nucléique, selon la revendication 1, comprenant :
a. la séquence nucléotidique de SEQ ID n° : 18 ;
b. la séquence nucléotidique de SEQ ID n° : 19 ;
c. la séquence nucléotidique de SEQ ID n° : 20 ; et
d. la séquence nucléotidique de SEQ ID n° : 21.

3. Acide nucléique, selon la revendication 1 ou 2, comprenant un intron dont la séquence nucléotidique est choisie dans le groupe de :
a. une séquence nucléotidique choisie dans le groupe suivant :
i. la séquence nucléotidique de la position 1418 jusqu'à la position 2055 de la SEQ ID n° : 3,
ii. la séquence nucléotidique de la position 1368 jusqu'à la position 1650 de la SEQ ID n° : 4,
iii. la séquence nucléotidique de la position 1369 jusqu'à la position 2001 de la SEQ ID n° : 5,
iv. la séquence nucléotidique de la position 1362 jusqu'à la position 1647 de la SEQ ID n° : 6, et
v. la séquence nucléotidique de la position 1367
jusqu'à la position 2001 de la SEQ ID n° : 7, ou un fragment fonctionnel de celle-ci ; et
b. un acide nucléique comprenant une séquence nucléotidique ayant une identité de séquence d'au moins 80 % avec un quelconque élément du a. ou un fragment fonctionnel de celle-ci.

4. Acide nucléique, selon la revendication 3, comprenant :
a. la séquence nucléotidique de SEQ ID n° : 22 ;
b. la séquence nucléotidique de SEQ ID n° : 23 ; et
c. la séquence nucléotidique de SEQ ID n° : 24 dans la séquence de l'intron.

5. Acide nucléique, selon la revendication 3 ou 4, ayant une activité de promoteur préférentielle dans la graine et le funicule supérieure à l'acide nucléique selon la revendication 1 ou 2.

6. Acide nucléique isolé, selon l'une quelconque des revendications 1 à 5, choisi dans le groupe suivant :
a. un acide nucléique comprenant l'une quelconque des SEQ ID n° : 3 à 7, ou un fragment fonctionnel de celle-ci, et
b. un acide nucléique comprenant une séquence nucléotidique ayant une identité de séquence d'au moins 80 % avec l'une quelconque des SEQ ID n° : 3 à 7 ou un fragment fonctionnel de celle-ci.

7. Gène recombinant comprenant l'acide nucléique, selon l'une quelconque des revendications 1 à 6, lié de manière opérationnelle à une séquence d'acide nucléique hétérologue codant pour un produit d'expression d'intérêt et, en option, une séquence de terminaison de la transcription et de polyadénylation, de préférence une région de terminaison de la transcription et de polyadénylation fonctionnelle dans des plantes.

8. Gène recombinant selon la revendication 7, dans lequel le produit d'expression d'intérêt est une molécule d'ARN, capable de moduler l'expression d'un gène, ou est une protéine.

9. Plante, graine ou cellule hôte comprenant le gène recombinant de la revendication 7 ou 8.

10. Plante ou graine, selon la revendication 9, comprenant au moins deux gènes recombinants, selon la revendication 7, dans laquelle l'acide nucléique, selon l'une quelconque des revendications 1 à 6, et en option la séquence d'acide nucléique hétérologue, est/sont différent(s) dans chaque gène recombinant.

11. Procédé de production d'une plante transgénique comprenant les étapes :
a. d'introduction ou de fourniture du gène recombinant, selon la revendication 7 ou 8, dans/à une cellule de plante pour créer des cellules transgéniques ; et
b. de régénération de plantes transgéniques à partir de ladite cellule transgénique.

12. Procédé de réalisation de l'expression préférentielle dans la graine et le funicule d'un acide nucléique comprenant l'étape d'introduction du gène recombinant, selon la revendication 7 ou 8, dans le génome d'une plante ou de fourniture de la plante selon la revendication 9 ou 10.

13. Procédé destiné à altérer des propriétés des graines d'une plante ou à produire un produit commercialement intéressant dans une plante, ledit procédé comprenant l'étape d'introduction du gène recombinant, selon la revendication 7 ou 8, dans le génome d'une plante ou de fourniture de la plante selon la revendication 9 ou 10.

14. Utilisation de l'acide nucléique isolé, selon l'une quelconque des revendications 1 à 6, pour réguler l'expression d'un acide nucléique lié de manière opérationnelle dans une plante ou pour identifier d'autres acides nucléiques comprenant une activité de promoteur préférentielle dans la graine et le funicule.

15. Utilisation de l'acide nucléique isolé, selon l'une quelconque des revendications 1 à 6, ou du gène recombinant, selon la revendication 7 ou 8, pour altérer des propriétés des graines d'une plante ou pour produire un produit commercialement intéressant dans une plante.

16. Procédé de production d'un aliment, d'un aliment pour animaux ou d'un produit industriel comprenant les étapes
a) d'obtention de la plante ou d'une partie de celle-ci selon la revendication 9 ou 10 ; et
b) de préparation de l'aliment, de l'aliment pour animaux ou du produit industriel à partir de la plante ou d'une partie de celle-ci
dans lequel
a. l'aliment ou l'aliment pour animaux est de l'huile, un plat, une céréale, de l'amidon, de la farine ou une protéine ; ou
b. le produit industriel est un biocarburant, une fibre, des produits chimiques industriels, une substance pharmaceutique ou une substance nutraceutique.


### SEQUENCE LISTING

<110> Bayer CropScience NV
<120> Seed- and Funiculus-preferential promoters and uses thereof
<130> BCS16-2003
<160> 24
<170> PatentIn version 3.5
<210> 1
   <211> 6611
   <212> DNA
   <213> Artificial Sequence
<220>
   <223> T-DNA Pslp1 BnA::GUS
<400> 1
   aattacaacg gtatatatcc tgccagtact ttgtggcgct ctatcatagc tataaaccta      60
   ttcagcacaa tatcgattaa gggccccctc gagggcgatc gctacgtacc tgcaggcccg     120
   ggttaattaa gcggccgctt tggctcgtat atcctctgga aagaatttaa tatctttcgg     180
   aatgtaataa tatcttttgg aatgtaaaca aaatggattt aatatctttt ggaatgtaaa     240
   agatattatt acattcacac cattttgttt cttttcgcta cgatgaaaga tattaaatcc     300
   aatttggtta ccagtcacac cattttgttt cttttcgcta cgatgaaata attttgaaaa     360
   agtaacttct cttattttga ttatgttcgt ttcgatgttc atgtaacctg cgatcgcgac     420
   cttaaataaa atattgttct tttgtttgtt gaaagaacaa ttgtttttta agttgtttaa     480
   attgtcagca aaatgaaatt ttgagtatga ttggtcaacg aagaccaatt ggctgaattt     540
   atccatgcat atatatatgt tgagaatcga cgccttcgat tgactttaaa ctcaatcata     600
   gaacacactt cgatttagtc cggtacatgt attaaaatcg gtacatcggt taggacctgt     660
   cggtccctaa gtttcgatag aagacgttgg aaaacacctt catgacccta aaatacatac     720
   gtaatagtaa agccatgttc gtttcgttat cgccgattcc agcggcagcg ttcaaaaata     780
   taaggcaaca gagacaaaaa aaaacagaat caaaggtaaa agcaatcgct catcaactgc     840
   cagagacact agaaaacgag acgcagtgac gctgaaattt tccgcgtcgt ttagttcatt     900
   gttaactgcc gcagcgtctg gaaaacaagt cattaatgcg tcaccggtgc tgtacgatgc     960
   catgatgaaa ggcttaatta tctgacgctg acgctgcgtc cagcgttgat aaaacgaaca    1020
   ggccttaagt cggtgctgaa gtttaattgc ctctttactt tgtaaccttc ttctttaaat    1080
   aggatgttta ccaatcttct agtggattgt acttttcaat gcacttccaa agagtttgaa    1140
   attttgtgaa ccagagagtg ttacacaaga acaattgagg tgctagagtc cgttatgaga    1200
   ctgtgatgtg gcatctatcc attggtggaa gaagatttgc tttgctgaca actaacaaga    1260
   tttggtgtta tcacaacaaa ttggagtcgg ctatatataa cgctctagag aaggctcaag 1320
   tatcagtcta ataactggtt caggatccgg tttagagaaa accgggagag tgtgacgtgt 1380
   ggaagtagcg ctctggattc tttgagtcat catagctcaa agtggacaaa aaacaatatt 1440
   ttgtaacgta tcaaagaatt tcctcttcga tatttttact tagagagaga gagagagaga 1500
   tcttctcttt caagttctag agctttctcc gccgatttca tctcctttag ccatggtccg 1560
   tcctgtagaa accccaaccc gtgaaatcaa aaaactcgac ggcctgtggg cattcagtct 1620
   ggatcgcgaa aactgtggaa ttgatcagcg ttggtgggaa agcgcgttac aagaaagccg 1680
   ggcaattgct gtgccaggca gttttaacga tcagttcgcc gatgcagata ttcgtaatta 1740
   tgcgggcaac gtctggtatc agcgcgaagt ctttataccg aaaggttggg caggccagcg 1800
   tatcgtgctg cgtttcgatg cggtcactca ttacggcaaa gtgtgggtca ataatcagga 1860
   agtgatggag catcagggcg gctatacgcc atttgaagcc gatgtcacgc cgtatgttat 1920
   tgccgggaaa agtgtacgta agtttctgct tctacctttg atatatatat aataattatc 1980
   attaattagt agtaatataa tatttcaaat atttttttca aaataaaaga atgtagtata 2040
   tagcaattgc ttttctgtag tttataagtg tgtatatttt aatttataac ttttctaata 2100
   tatgaccaaa atttgttgat gtgcaggtat caccgtttgt gtgaacaacg aactgaactg 2160
   gcagactatc ccgccgggaa tggtgattac cgacgaaaac ggcaagaaaa agcagtctta 2220
   cttccatgat ttctttaact atgccggaat ccatcgcagc gtaatgctct acaccacgcc 2280
   gaacacctgg gtggacgata tcaccgtggt gacgcatgtc gcgcaagact gtaaccacgc 2340
   gtctgttgac tggcaggtgg tggccaatgg tgatgtcagc gttgaactgc gtgatgcgga 2400
   tcaacaggtg gttgcaactg gacaaggcac tagcgggact ttgcaagtgg tgaatccgca 2460
   cctctggcaa ccgggtgaag gttatctcta tgaactgtgc gtcacagcca aaagccagac 2520
   agagtgtgat atctacccgc ttcgcgtcgg catccggtca gtggcagtga agggcgaaca 2580
   gttcctgatt aaccacaaac cgttctactt tactggcttt ggtcgtcatg aagatgcgga 2640
   cttgcgtggc aaaggattcg ataacgtgct gatggtgcac gaccacgcat taatggactg 2700
   gattggggcc aactcctacc gtacctcgca ttacccttac gctgaagaga tgctcgactg 2760
   ggcagatgaa catggcatcg tggtgattga tgaaactgct gctgtcggct ttaacctctc 2820
   tttaggcatt ggtttcgaag cgggcaacaa gccgaaagaa ctgtacagcg aagaggcagt 2880
   caacggggaa actcagcaag cgcacttaca ggcgattaaa gagctgatag cgcgtgacaa 2940
   aaaccaccca agcgtggtga tgtggagtat tgccaacgaa ccggataccc gtccgcaagg 3000
   tgcacgggaa tatttcgcgc cactggcgga agcaacgcgt aaactcgacc cgacgcgtcc 3060
   gatcacctgc gtcaatgtaa tgttctgcga cgctcacacc gataccatca gcgatctctt 3120
   tgatgtgctg tgcctgaacc gttattacgg atggtatgtc caaagcggcg atttggaaac 3180
   ggcagagaag gtactggaaa aagaacttct ggcctggcag gagaaactgc atcagccgat 3240
   tatcatcacc gaatacggcg tggatacgtt agccgggctg cactcaatgt acaccgacat 3300
   gtggagtgaa gagtatcagt gtgcatggct ggatatgtat caccgcgtct ttgatcgcgt 3360
   cagcgccgtc gtcggtgaac aggtatggaa tttcgccgat tttgcgacct cgcaaggcat 3420
   attgcgcgtt ggcggtaaca agaaagggat cttcactcgc gaccgcaaac cgaagtcggc 3480
   ggcttttctg ctgcaaaaac gctggactgg catgaacttc ggtgaaaaac cgcagcaggg 3540
   aggcaaacaa tgaatcaaca actctcctgg cgcaccatcg tcggctacag cctcggcgcg 3600
   tggcgcgcct aagctagcta tatcatcaat ttatgtatta cacataatat cgcactcagt 3660
   ctttcatcta cggcaatgta ccagctgata taatcagtta ttgaaatatt tctgaattta 3720
   aacttgcatc aataaattta tgtttttgct tggactataa tacctgactt gttattttat 3780
   caataaatat ttaaactata tttctttcaa gatgaattcg atatcattac cctgttatcc 3840
   ctaaagctta ttaatataac ttcgtatagc atacattata cgaagttatg tttcaaattt 3900
   attatgtgtt ttttttccgt ggtcgagatt gtgtattatt ctttagttat tacaagactt 3960
   ttagctaaaa tttgaaagaa tttactttaa gaaaatctta acatctgaga taatttcagc 4020
   aatagattat atttttcatt actctagcag tatttttgca gatcaatcgc aacatatatg 4080
   gttgttagaa aaaatgcact atatatatat atattatttt ttcaattaaa agtgcatgat 4140
   atataatata tatatatata tatatatgtg tgtgtgtata tggtcaaaga aattcttata 4200
   caaatataca cgaacacata tatttgacaa aatcaaagta ttacactaaa caatgagttg 4260
   gtgcatggcc aaaacaaata tgtagattaa aaattccagc ctccaaaaaa aaatccaagt 4320
   gttgtaaagc attatatata tatagtagat cccaaatttt tgtacaattc cacactgatc 4380
   gaatttttaa agttgaatat ctgacgtagg atttttttaa tgtcttacct gaccatttac 4440
   taataacatt catacgtttt catttgaaat atcctctata attatattga atttggcaca 4500
   taataagaaa cctaattggt gatttatttt actagtaaat ttctggtgat gggctttcta 4560
   ctagaaagct ctcggaaaat cttggaccaa atccatattc catgacttcg attgttaacc 4620
   ctattagttt tcacaaacat actatcaata tcattgcaac ggaaaaggta caagtaaaac 4680
   attcaatccg atagggaagt gatgtaggag gttgggaaga caggcccaga aagagattta 4740
   tctgacttgt tttgtgtata gttttcaatg ttcataaagg aagatggaga cttgagaagt 4800
   tttttttgga ctttgtttag ctttgttggg cgtttttttt ttttgatcaa taactttgtt 4860
   gggcttatga tttgtaatat tttcgtggac tctttagttt atttagacgt gctaactttg 4920
   ttgggcttat gacttgttgt aacatattgt aacagatgac ttgatgtgcg actaatcttt 4980
   acacattaaa catagttctg ttttttgaaa gttcttattt tcatttttat ttgaatgtta 5040
   tatatttttc tatatttata attctagtaa aaggcaaatt ttgcttttaa atgaaaaaaa 5100
   tatatattcc acagtttcac ctaatcttat gcatttagca gtacaaattc aaaaatttcc 5160
   catttttatt catgaatcat accattatat attaactaaa tccaaggtaa aaaaaaggta 5220
   tgaaagctct atagtaagta aaatataaat tccccataag gaaagggcca agtccaccag 5280
   gcaagtaaaa tgagcaagca ccactccacc atcacacaat ttcactcata gataacgata 5340
   agattcatgg aattatcttc cacgtggcat tattccagcg gttcaagccg ataagggtct 5400
   caacacctct ccttaggcct ttgtggccgt taccaagtaa aattaacctc acacatatcc 5460
   acactcaaaa tccaacggtg tagatcctag tccacttgaa tctcatgtat cctagaccct 5520
   ccgatcactc caaagcttgt tctcattgtt gttatcatta tatatagatg accaaagcac 5580
   tagaccaaac ctcagtcaca caaagagtaa agaagaacaa tggacccaga acgacgcccg 5640
   gccgacatcc gccgtgccac cgaggcggac atgccggcgg tctgcaccat cgtcaaccac 5700
   tacatcgaga caagcacggt caacttccgt accgagccgc aggaaccgca ggagtggacg 5760
   gacgacctcg tccgtctgcg ggagcgctat ccctggctcg tcgccgaggt ggacggcgag 5820
   gtcgccggca tcgcctacgc gggcccctgg aaggcacgca acgcctacga ctggacggcc 5880
   gagtcgaccg tgtacgtctc cccccgccac cagcggacgg gactgggctc cacgctctac 5940
   acccacctgc tgaagtccct ggaggcacag ggcttcaaga gcgtggtcgc tgtcatcggg 6000
   ctgcccaacg acccgagcgt gcgcatgcac gaggcgctcg gatatgcccc ccgcggcatg 6060
   ctgcgggcgg ccggcttcaa gcacgggaac tggcatgacg tgggtttctg gcagctggac 6120
   ttcagcctgc cggtaccgcc ccgtccggtc ctgcccgtca ccgagatctg atctcacccg 6180
   tctaggatcc cccgatgagc taagctagct atatcatcaa tttatgtatt acacataata 6240
   tcgcactcag tctttcatct acggcaatgt accagctgat ataatcagtt attgaaatat 6300
   ttctgaattt aaacttgcat caataaattt atgtttttgc ttggactata atacctgact 6360
   tgttatttta tcaataaata tttaaactat atttctttca agataaacat aacttcgtat 6420
   agcatacatt atacgaagtt atcaaaacgt cgtgagacag tttggttaac tataacggtc 6480
   ctaaggtagc gatcgaggca ttacggcatt acggcactcg cgagggtccg aatctatgtc 6540
   gggtgcggag aaagaggtaa tgaaatggca attcgagcat ggagccattt acaattgaat 6600
   atatcctgcc g 6611
<210> 2
   <211> 7249
   <212> DNA
   <213> Artificial Sequence
<220>
   <223> T-DNA Pslplintron BnA::GUS
<400> 2
   aattacaacg gtatatatcc tgccagtact ttgtggcgct ctatcatagc tataaaccta      60
   ttcagcacaa tatcgattaa gggccccctc gagggcgatc gctacgtacc tgcaggcccg     120
   ggttaattaa gcggccgctt tggctcgtat atcctctgga aagaatttaa tatctttcgg     180
   aatgtaataa tatcttttgg aatgtaaaca aaatggattt aatatctttt ggaatgtaaa     240
   agatattatt acattcacac cattttgttt cttttcgcta cgatgaaaga tattaaatcc     300
   aatttggtta ccagtcacac cattttgttt cttttcgcta cgatgaaata attttgaaaa     360
   agtaacttct cttattttga ttatgttcgt ttcgatgttc atgtaacctg cgatcgcgac     420
   cttaaataaa atattgttct tttgtttgtt gaaagaacaa ttgtttttta agttgtttaa     480
   attgtcagca aaatgaaatt ttgagtatga ttggtcaacg aagaccaatt ggctgaattt     540
   atccatgcat atatatatgt tgagaatcga cgccttcgat tgactttaaa ctcaatcata     600
   gaacacactt cgatttagtc cggtacatgt attaaaatcg gtacatcggt taggacctgt     660
   cggtccctaa gtttcgatag aagacgttgg aaaacacctt catgacccta aaatacatac     720
   gtaatagtaa agccatgttc gtttcgttat cgccgattcc agcggcagcg ttcaaaaata     780
   taaggcaaca gagacaaaaa aaaacagaat caaaggtaaa agcaatcgct catcaactgc     840
   cagagacact agaaaacgag acgcagtgac gctgaaattt tccgcgtcgt ttagttcatt     900
   gttaactgcc gcagcgtctg gaaaacaagt cattaatgcg tcaccggtgc tgtacgatgc     960
   catgatgaaa ggcttaatta tctgacgctg acgctgcgtc cagcgttgat aaaacgaaca    1020
   ggccttaagt cggtgctgaa gtttaattgc ctctttactt tgtaaccttc ttctttaaat    1080
   aggatgttta ccaatcttct agtggattgt acttttcaat gcacttccaa agagtttgaa    1140
   attttgtgaa ccagagagtg ttacacaaga acaattgagg tgctagagtc cgttatgaga    1200
   ctgtgatgtg gcatctatcc attggtggaa gaagatttgc tttgctgaca actaacaaga    1260
   tttggtgtta tcacaacaaa ttggagtcgg ctatatataa cgctctagag aaggctcaag    1320
   tatcagtcta ataactggtt caggatccgg tttagagaaa accgggagag tgtgacgtgt    1380
   ggaagtagcg ctctggattc tttgagtcat catagctcaa agtggacaaa aaacaatatt    1440
   ttgtaacgta tcaaagaatt tcctcttcga tatttttact tagagagaga gagagagaga    1500
   tcttctcttt caagttctag agctttctcc gccgatttca tctcctttag ccatggtaat    1560
   taaaaaaaaa aactattttc agtctttctt gccttcatat ttcacgtctc tctggattct    1620
   ctgctaattt tggattgtgg tatggctttt acgttgtcta ggaaaatcga tggaaacaaa    1680
   agaaaactga aatgcatgct atatgttaga ttttgtaatt gtttaaaatt aataagaaat    1740
   tagcagtagt ttcaagtaag tggttaaaac caaatagatt tatgtaaaac ttgcatgtga    1800
   aattccaact ttaaagtttg ttcatgatga tcttgatagc gtgaaaatca agaattttag    1860
   ctttaaaagt cattggagga ggtgctagtg tgagttggtt gaatgttgtt aaactgatca 1920
   caaagaagaa ttttttgttt tcattgattt tattttttaa gttagtttgt atttgtagta 1980
   aggttggaat agcatgtggt gaagatgttg tttagcagtt tgacttccct ggatccatag 2040
   gaaaaaggat aattaggcct tctcttaatg taaaaactga gactttgaag ttattctcaa 2100
   acgttgttct atcattagat catatatcct tttatgattt gcaatatctc acaatcttta 2160
   agctttcttg tgttttattt gttcacatag ggcgtccgtc ctgtagaaac cccaacccgt 2220
   gaaatcaaaa aactcgacgg cctgtgggca ttcagtctgg atcgcgaaaa ctgtggaatt 2280
   gatcagcgtt ggtgggaaag cgcgttacaa gaaagccggg caattgctgt gccaggcagt 2340
   tttaacgatc agttcgccga tgcagatatt cgtaattatg cgggcaacgt ctggtatcag 2400
   cgcgaagtct ttataccgaa aggttgggca ggccagcgta tcgtgctgcg tttcgatgcg 2460
   gtcactcatt acggcaaagt gtgggtcaat aatcaggaag tgatggagca tcagggcggc 2520
   tatacgccat ttgaagccga tgtcacgccg tatgttattg ccgggaaaag tgtacgtaag 2580
   tttctgcttc tacctttgat atatatataa taattatcat taattagtag taatataata 2640
   tttcaaatat ttttttcaaa ataaaagaat gtagtatata gcaattgctt ttctgtagtt 2700
   tataagtgtg tatattttaa tttataactt ttctaatata tgaccaaaat ttgttgatgt 2760
   gcaggtatca ccgtttgtgt gaacaacgaa ctgaactggc agactatccc gccgggaatg 2820
   gtgattaccg acgaaaacgg caagaaaaag cagtcttact tccatgattt ctttaactat 2880
   gccggaatcc atcgcagcgt aatgctctac accacgccga acacctgggt ggacgatatc 2940
   accgtggtga cgcatgtcgc gcaagactgt aaccacgcgt ctgttgactg gcaggtggtg 3000
   gccaatggtg atgtcagcgt tgaactgcgt gatgcggatc aacaggtggt tgcaactgga 3060
   caaggcacta gcgggacttt gcaagtggtg aatccgcacc tctggcaacc gggtgaaggt 3120
   tatctctatg aactgtgcgt cacagccaaa agccagacag agtgtgatat ctacccgctt 3180
   cgcgtcggca tccggtcagt ggcagtgaag ggcgaacagt tcctgattaa ccacaaaccg 3240
   ttctacttta ctggctttgg tcgtcatgaa gatgcggact tgcgtggcaa aggattcgat 3300
   aacgtgctga tggtgcacga ccacgcatta atggactgga ttggggccaa ctcctaccgt 3360
   acctcgcatt acccttacgc tgaagagatg ctcgactggg cagatgaaca tggcatcgtg 3420
   gtgattgatg aaactgctgc tgtcggcttt aacctctctt taggcattgg tttcgaagcg 3480
   ggcaacaagc cgaaagaact gtacagcgaa gaggcagtca acggggaaac tcagcaagcg 3540
   cacttacagg cgattaaaga gctgatagcg cgtgacaaaa accacccaag cgtggtgatg 3600
   tggagtattg ccaacgaacc ggatacccgt ccgcaaggtg cacgggaata tttcgcgcca 3660
   ctggcggaag caacgcgtaa actcgacccg acgcgtccga tcacctgcgt caatgtaatg 3720
   ttctgcgacg ctcacaccga taccatcagc gatctctttg atgtgctgtg cctgaaccgt 3780
   tattacggat ggtatgtcca aagcggcgat ttggaaacgg cagagaaggt actggaaaaa 3840
   gaacttctgg cctggcagga gaaactgcat cagccgatta tcatcaccga atacggcgtg 3900
   gatacgttag ccgggctgca ctcaatgtac accgacatgt ggagtgaaga gtatcagtgt 3960
   gcatggctgg atatgtatca ccgcgtcttt gatcgcgtca gcgccgtcgt cggtgaacag 4020
   gtatggaatt tcgccgattt tgcgacctcg caaggcatat tgcgcgttgg cggtaacaag 4080
   aaagggatct tcactcgcga ccgcaaaccg aagtcggcgg cttttctgct gcaaaaacgc 4140
   tggactggca tgaacttcgg tgaaaaaccg cagcagggag gcaaacaatg aatcaacaac 4200
   tctcctggcg caccatcgtc ggctacagcc tcggcgcgtg gcgcgcctaa gctagctata 4260
   tcatcaattt atgtattaca cataatatcg cactcagtct ttcatctacg gcaatgtacc 4320
   agctgatata atcagttatt gaaatatttc tgaatttaaa cttgcatcaa taaatttatg 4380
   tttttgcttg gactataata cctgacttgt tattttatca ataaatattt aaactatatt 4440
   tctttcaaga tgaattcgat atcattaccc tgttatccct aaagcttatt aatataactt 4500
   cgtatagcat acattatacg aagttatgtt tcaaatttat tatgtgtttt ttttccgtgg 4560
   tcgagattgt gtattattct ttagttatta caagactttt agctaaaatt tgaaagaatt 4620
   tactttaaga aaatcttaac atctgagata atttcagcaa tagattatat ttttcattac 4680
   tctagcagta tttttgcaga tcaatcgcaa catatatggt tgttagaaaa aatgcactat 4740
   atatatatat attatttttt caattaaaag tgcatgatat ataatatata tatatatata 4800
   tatatgtgtg tgtgtatatg gtcaaagaaa ttcttataca aatatacacg aacacatata 4860
   tttgacaaaa tcaaagtatt acactaaaca atgagttggt gcatggccaa aacaaatatg 4920
   tagattaaaa attccagcct ccaaaaaaaa atccaagtgt tgtaaagcat tatatatata 4980
   tagtagatcc caaatttttg tacaattcca cactgatcga atttttaaag ttgaatatct 5040
   gacgtaggat ttttttaatg tcttacctga ccatttacta ataacattca tacgttttca 5100
   tttgaaatat cctctataat tatattgaat ttggcacata ataagaaacc taattggtga 5160
   tttattttac tagtaaattt ctggtgatgg gctttctact agaaagctct cggaaaatct 5220
   tggaccaaat ccatattcca tgacttcgat tgttaaccct attagttttc acaaacatac 5280
   tatcaatatc attgcaacgg aaaaggtaca agtaaaacat tcaatccgat agggaagtga 5340
   tgtaggaggt tgggaagaca ggcccagaaa gagatttatc tgacttgttt tgtgtatagt 5400
   tttcaatgtt cataaaggaa gatggagact tgagaagttt tttttggact ttgtttagct 5460
   ttgttgggcg tttttttttt ttgatcaata actttgttgg gcttatgatt tgtaatattt 5520
   tcgtggactc tttagtttat ttagacgtgc taactttgtt gggcttatga cttgttgtaa 5580
   catattgtaa cagatgactt gatgtgcgac taatctttac acattaaaca tagttctgtt 5640
   ttttgaaagt tcttattttc atttttattt gaatgttata tatttttcta tatttataat 5700
   tctagtaaaa ggcaaatttt gcttttaaat gaaaaaaata tatattccac agtttcacct 5760
   aatcttatgc atttagcagt acaaattcaa aaatttccca tttttattca tgaatcatac 5820
   cattatatat taactaaatc caaggtaaaa aaaaggtatg aaagctctat agtaagtaaa 5880
   atataaattc cccataagga aagggccaag tccaccaggc aagtaaaatg agcaagcacc 5940
   actccaccat cacacaattt cactcataga taacgataag attcatggaa ttatcttcca 6000
   cgtggcatta ttccagcggt tcaagccgat aagggtctca acacctctcc ttaggccttt 6060
   gtggccgtta ccaagtaaaa ttaacctcac acatatccac actcaaaatc caacggtgta 6120
   gatcctagtc cacttgaatc tcatgtatcc tagaccctcc gatcactcca aagcttgttc 6180
   tcattgttgt tatcattata tatagatgac caaagcacta gaccaaacct cagtcacaca 6240
   aagagtaaag aagaacaatg gacccagaac gacgcccggc cgacatccgc cgtgccaccg 6300
   aggcggacat gccggcggtc tgcaccatcg tcaaccacta catcgagaca agcacggtca 6360
   acttccgtac cgagccgcag gaaccgcagg agtggacgga cgacctcgtc cgtctgcggg 6420
   agcgctatcc ctggctcgtc gccgaggtgg acggcgaggt cgccggcatc gcctacgcgg 6480
   gcccctggaa ggcacgcaac gcctacgact ggacggccga gtcgaccgtg tacgtctccc 6540
   cccgccacca gcggacggga ctgggctcca cgctctacac ccacctgctg aagtccctgg 6600
   aggcacaggg cttcaagagc gtggtcgctg tcatcgggct gcccaacgac ccgagcgtgc 6660
   gcatgcacga ggcgctcgga tatgcccccc gcggcatgct gcgggcggcc ggcttcaagc 6720
   acgggaactg gcatgacgtg ggtttctggc agctggactt cagcctgccg gtaccgcccc 6780
   gtccggtcct gcccgtcacc gagatctgat ctcacccgtc taggatcccc cgatgagcta 6840
   agctagctat atcatcaatt tatgtattac acataatatc gcactcagtc tttcatctac 6900
   ggcaatgtac cagctgatat aatcagttat tgaaatattt ctgaatttaa acttgcatca 6960
   ataaatttat gtttttgctt ggactataat acctgacttg ttattttatc aataaatatt 7020
   taaactatat ttctttcaag ataaacataa cttcgtatag catacattat acgaagttat 7080
   caaaacgtcg tgagacagtt tggttaacta taacggtcct aaggtagcga tcgaggcatt 7140
   acggcattac ggcactcgcg agggtccgaa tctatgtcgg gtgcggagaa agaggtaatg 7200
   aaatggcaat tcgagcatgg agccatttac aattgaatat atcctgccg 7249
<210> 3
   <211> 2055
   <212> DNA
   <213> Brassica napus
<400> 3
   tttggctcgt atatcctctg gaaagaattt aatatctttc ggaatgtaat aatatctttt 60
   ggaatgtaaa caaaatggat ttaatatctt ttggaatgta aaagatatta ttacattcac 120
   accattttgt ttcttttcgc tacgatgaaa gatattaaat ccaatttggt taccagtcac 180
   accattttgt ttcttttcgc tacgatgaaa taattttgaa aaagtaactt ctcttatttt 240
   gattatgttc gtttcgatgt tcatgtaacc tgcgatcgcg accttaaata aaatattgtt 300
   cttttgtttg ttgaaagaac aattgttttt taagttgttt aaattgtcag caaaatgaaa 360
   ttttgagtat gattggtcaa cgaagaccaa ttggctgaat ttatccatgc atatatatat 420
   gttgagaatc gacgccttcg attgacttta aactcaatca tagaacacac ttcgatttag 480
   tccggtacat gtattaaaat cggtacatcg gttaggacct gtcggtccct aagtttcgat 540
   agaagacgtt ggaaaacacc ttcatgaccc taaaatacat acgtaatagt aaagccatgt 600
   tcgtttcgtt atcgccgatt ccagcggcag cgttcaaaaa tataaggcaa cagagacaaa 660
   aaaaaacaga atcaaaggta aaagcaatcg ctcatcaact gccagagaca ctagaaaacg 720
   agacgcagtg acgctgaaat tttccgcgtc gtttagttca ttgttaactg ccgcagcgtc 780
   tggaaaacaa gtcattaatg cgtcaccggt gctgtacgat gccatgatga aaggcttaat 840
   tatctgacgc tgacgctgcg tccagcgttg ataaaacgaa caggccttaa gtcggtgctg 900
   aagtttaatt gcctctttac tttgtaacct tcttctttaa ataggatgtt taccaatctt 960
   ctagtggatt gtacttttca atgcacttcc aaagagtttg aaattttgtg aaccagagag 1020
   tgttacacaa gaacaattga ggtgctagag tccgttatga gactgtgatg tggcatctat 1080
   ccattggtgg aagaagattt gctttgctga caactaacaa gatttggtgt tatcacaaca 1140
   aattggagtc ggctatatat aacgctctag agaaggctca agtatcagtc taataactgg 1200
   ttcaggatcc ggtttagaga aaaccgggag agtgtgacgt gtggaagtag cgctctggat 1260
   tctttgagtc atcatagctc aaagtggaca aaaaacaata ttttgtaacg tatcaaagaa 1320
   tttcctcttc gatattttta cttagagaga gagagagaga gatcttctct ttcaagttct 1380
   agagctttct ccgccgattt catctccttt agccatggta attaaaaaaa aaaactattt 1440
   tcagtctttc ttgccttcat atttcacgtc tctctggatt ctctgctaat tttggattgt 1500
   ggtatggctt ttacgttgtc taggaaaatc gatggaaaca aaagaaaact gaaatgcatg 1560
   ctatatgtta gattttgtaa ttgtttaaaa ttaataagaa attagcagta gtttcaagta 1620
   agtggttaaa accaaataga tttatgtaaa acttgcatgt gaaattccaa ctttaaagtt 1680
   tgttcatgat gatcttgata gcgtgaaaat caagaatttt agctttaaaa gtcattggag 1740
   gaggtgctag tgtgagttgg ttgaatgttg ttaaactgat cacaaagaag aattttttgt 1800
   tttcattgat tttatttttt aagttagttt gtatttgtag taaggttgga atagcatgtg 1860
   gtgaagatgt tgtttagcag tttgacttcc ctggatccat aggaaaaagg ataattaggc 1920
   cttctcttaa tgtaaaaact gagactttga agttattctc aaacgttgtt ctatcattag 1980
   atcatatatc cttttatgat ttgcaatatc tcacaatctt taagctttct tgtgttttat 2040
   ttgttcacat agggc 2055
<210> 4
   <211> 1650
   <212> DNA
   <213> Brassica napus
<400> 4
   aacatttttt ctgtcaaaac caaaaaaatg atttttttcg ctaaaaccgt aaaaaaaaaa 60
   attatctcac agaaaccaaa atatgcgttt ttccgccgaa acggaaaaac cgagttttct 120
   cgccaaaaca ataaaaccaa gttttcccgt caaaaccgca aaaccgagtg ttctcgccaa 180
   aaccgcaaaa ccaaaatttc ctgccaaaac cccaaaaccg cgaaaccgag tttttccgac 240
   aaaaccttaa aattgagttt tccgactgaa aaccgcaaaa tcgagttttc ccgccaaaac 300
   cgcaaaaccg agttttctca ccaaaaccgt taaaacaagt ttttccgcca aaaccgcaaa 360
   acccaatttt tccgccaaaa ccgcaaaacg agttttccag ccgaaaccaa aaaaccgagt 420
   ttcccgccaa aactgaaaaa ccaagttttc ccgccaaaac cgcaaaaccg agttttctcg 480
   ccaaaaccgc aaagcaaatt ttgttcgcca aaaccgcaaa accgaatttt cccgccaaaa 540
   ccacaaagag tttcttcgcc gaaaccagaa aaccgagttt ttccgccaaa atcgcaaaac 600
   cgagtttttc cgtcaaaatc agaaaaatga gttttcccgc caaaaccgta aaatcgagtt 660
   ttcctgccga aaccgcaaaa ccgagttttt ccgccaaaat cgaaaacaga atttcccgcc 720
   aaaatcgtaa acgagttttt gcgctaaaac atgtttttct ataaaattgc gaaatcttgt 780
   ttatatatta aagctcacat taatgatatt aatattttac atgatctttt tagaataaat 840
   aagataatat ttttggtatc tatgggtaaa catataccgt atttgggttt ggatttcaac 900
   cttgatgttt ctgggttttt gcaggtcggg tataaactgg gttaggttgt ctgttggttg 960
   gatttgggtg ggttattata ccctacgtta acatccctag ttagagtccg ttatgagatt 1020
   gtgatgtggc gtctatccgt tggtggaaga atatttgctt tgctgacaac taacaagatt 1080
   tggtgttatc acaacaaatt ggagtcggat atatataacg ctctagagaa ggcccaagta 1140
   tcagtctaat aactggttca ggatccggtt tagagaaaac cgggagagtg tgacgtgtgg 1200
   aagtagcact ctggattctt tgagtcatca tagctcaaag tggacaaaaa acaatatttt 1260
   gtaacgtatc aaagaatttc ctcttcgata tttttactta gagagagaga gatcttctct 1320
   ttcaagttct agagctttct ccgccgattt catctccttt agccatggta attaaaaaaa 1380
   aaaatctgtt ttcagtcttt cttgccttca tatttcacgt ttctctggat tctctgctaa 1440
   ttttggattg tggtatggct tttacgttgt ctaggaaaat cgatggaaac aaaagaaaac 1500
   tgaaatgcat gcgatatgtt agattttgta attgtttata attaaaaaaa aattagcagt 1560
   agtttcaagt aagtggttaa aaccaaatag atttatgtaa aacttgcatg tgaaattcca 1620
   acttcaaagt ttgttcatta tcttgatagc 1650
<210> 5
   <211> 2001
   <212> DNA
   <213> Brassica rapa
<400> 5
   tgaattttct gaatgactag gatgtactag attttttggc tcgtatatct tcgggaaaga      60
   atttaatatc tttcggaatg taataatatc ttttggaatg taaacaaaag ggatttaata     120
   tcttttggaa tgtaaaagat attattacat tcacaccatt ttgtttcttt tcgctacgat     180
   gaaagatatt aaatccaatt tggttaccag tcacaccatt ttgtttcttt tcgctacgat     240
   gaaataattt tgaaaaagta acttctctta ttttgattat gttcgtttcg atgttcatgt     300
   aacctgcgat cgcgacctta aataaaatat tgttcttttg tttgtttaaa gaacaattgt     360
   ttttatgttg tttaaattgt cagcaaaatg aaatattgag tatgattggt caacgaagac     420
   caattggctg aatttaacca tgcatatata tatatatata tatatattat atatgttgag     480
   aatcgacgcc ttcgattgac tttaaactca atcatagaac acacttcgat ttagtccggt     540
   acatgtatta aaatcggtac atcggttagg acctgtcggt ccctaagttt cgatagaaga     600
   cgttggaaaa cacctcttaa gaagaagaag aagaagaaga agaagaattc acaaagaaga     660
   agaagaattc acaaagaaga gaagagaaca tcaagtcaaa gtcatgactc taaaatacat     720
   acgtaatagt atcaagtcat cacgtcagaa tagattctct ctcagattaa tactcttctg     780
   gttttgttga cgacgaggga aacgaagtct aagtcggtgc tgaagtttca ttgccttctg     840
   tgacacatga atatcactaa aacctcttta ctttgtaacc ttcttcttta tataggatgt     900
   ttaccaatct tctagtggat tgtacttttc aatgcacttc caaagagttt gaaattttgt     960
   gaaccagaga gtgttacaca agaaaaattg aggtgctaga gtccgttatg agactgtgat    1020
   gtggcatcta tccattggtg gaagaagatt tgctttgctg acaactaaca agatttggtg    1080
   ttatcacaac aaattggagt cggctatata taacgctcta gagaaggctc aagtatcagt    1140
   ctaataactg gttcaggatc cggtttagag aaaaccggga gagtgtgacg tgtggaagta    1200
   gcactctgga ttctttgagt catcatagct caaagtggac aaaaaacaat attttgtaac    1260
   gtatcaaaga atttcctctt cgatattttt acttagagag agagagagag agatcttctc    1320
   tttcaagttc tagagctttc tccgccgatt tcatctcctt tagccatggt aattaaaaaa    1380
   aaactatttt cagtctttct tgccttcata tttcacgtct ctctggattc tctgctaatt    1440
   ttggattgtg gtatggcttt tacgttctct aggaaaatcg atggaaacaa aagaaaactg    1500
   aaatgcatgc tatatgttag attttgtaat tgtttaaaat taataagaaa ttagcagtag    1560
   tttcaagtaa gtggttaaaa ccaaatagat ttatgtaaaa cttgcatgtg aaattccaac      1620
   tttaaagttt gttcatgatg atcttgatag cgtgaaaatc aagaatttta gctttaaaag      1680
   tcattggagg aggtgctagt gtgagttggt tgaatgttgt taaactgatc acaaagaaga      1740
   attttttgtt ttcattgatt ttatttttta agttagtttg tatttgtagt aaggttggaa       1800
   tagcatgtgg tgaagatgtt gtttagcagt ttgacttccc tggatccata ggaaaaagga      1860
   taattaggcc ttctcttaat gtaaaaactg agactttgaa gttattctca aacgttgttc      1920
   tatcattaga tcatatatcc ttttatgatt tgcaatatct cacaatcttt aagctttctt      1980
   gtgttttatt tgttcacata g                                                2001
<210> 6
   <211> 1647
   <212> DNA
   <213> Brassica oleracea
<400> 6
   aaaaatgatt tttttcgcta aaaccgtaaa aaaataaatt atctcacaga aaccaaaata      60
   tgagttttcc cgccgaaacc gaaaaaccgg gtttttctgc caaaaccata aaaccaagtt     120
   ttcccgtcaa aaccgcaaaa ccgagtgttc tcgccaaaac cgcaaaacca aaatttcccg     180
   ccaaaacccc aaaaccgcga aaccgagttt ttccgacaaa accttaaaat tgagtttttc     240
   cgactgaaaa ccgcaaaacc gagtttttcc gccaaaagcg cgaaaccgag ttttctcacc     300
   aaaaccgtta aaataagttt tccgccaaaa ccgcaaaacc caattttttc gccaaaaccg      360
   caaaacgagt tttcctgccg aaaccgaaaa atcgagtttt cccgccaaaa ccgaaaaatc     420
   gagttttccc gccaaaaccg caaaaccgag ttttctagcc aaaaccgcaa agcaaatttt     480
   gttcgccaaa accgcaaaac cgaattttcc cgccaaaacc acaaagagtt tcttcgccga     540
   aaccagaaaa ccgagttttc ccgccaaaat cgcaaaaccg agtttttccg tcgaaaccac     600
   aaaaaggagt tttcccgcca aaaccgtaaa atcgagtttt tctgccgaaa ccgcaaaacc     660
   gagttttccc gccaaaatcg aaaacggaat ttcccgccaa aatcgtaaac gagtttttgc     720
   gctaaaacaa gtttttctat aaaattacga aatcttgttt atatattaaa gctcacatta     780
   atgatattaa tattttacat gatattttta gaataaataa gataatattt tgggtatcga     840
   tgggtaaacc tataccgtat ttgggttatt ttttgggttt ggatttcaac cttgatgttt     900
   ctgggttttt gcatggttgg gtataaactg ggttaggtta tatgttggtt gggctggggt     960
   gggttatttt accctacgtt aacatcccta gttagagtcc gttatgagat tgtgatgtgg    1020
   cgtctatccg ttggtggaag aatatttgct ttgctgacaa ctaacaagat tcggtgttat    1080
   cacaacaaat tggagtcgga tatatataac gctctagaga aggcccaagt atcagtctaa    1140
   taactggttc aggatccggt ttagagaaaa ccgagagagt gtgacgtgtg aaagtagcac    1200
   tctggattct ttgagtcatc atagcttaaa gtggacaaaa aacaatattt tgtaacgtat    1260
   caaagaattt cctcttcgat atttttactt aagagagaga gagagatctt ctgtttcaag    1320
   ttctagagct ttctccgccg atttcatctc ctttagccat ggtaattaaa aaaaaaaaaa    1380
   atctattttc agtctttctt gccttcattt ttcacgtttc tctggattct ctgctaattt    1440
   tggattgtgg tatggctttt acgttgtcta ggaaaatcga tggaaacaaa agaaaactga    1500
   aatgcatgcg atatgttaga ttttgtaatt gtttataatt aaaaagaaat tagcagtagt    1560
   ttcaagtaag tggttaaaac caaatagatt tatgtaaaac ttgcatgtga aattccaact    1620
   tcaaagtttg ttcattatct tgatagc                                         1647
<210> 7
   <211> 2001
   <212> DNA
   <213> Brassica juncea
<400> 7
   aatgtggaca tgttctgttt tataaaaaaa gaaacaaaat gaattttctg aatgactacg      60
   atttactaga ttttttggct cgtatatctt ctggaaagaa tttaatatct ttcggaatgt     120
   aataatatct tttggaatgt aaacaaaagg gatttaatat cttttggaat gtaaaagata     180
   ttattattca caccattttg tttcttttcg ctacgatgaa agatattaaa tccaatttgg     240
   ttaccagtca caccattttg tttcttttcg ctacgatgaa ataattttga aaaagtaact     300
   tctcttattt tgattatgtt cgtttcgatg ttcatgtaac ctgcgatcgc gaccttaaat     360
   aaaatattgt tcttttgttt gtttaaagaa caattgtttt ttaaattgtt taaattgtca     420
   gcaaaatgaa attttgagta tgattggtca acgaagacca attggctgaa tttatccatg     480
   catatatata tgttgagaat cgacgccttc gattgacttt aaactcaatc atagaacaca     540
   cttcgattta gtccggtaca tgtattaaaa tcggtacatc ggttaggacc cctaagtttc     600
   gatagaagac gttggaaaac acctcttaag aagaagaaga agaagaattc acaaagaaga     660
   gaagagaaca ccaagtcaaa gtcatgactc taaaatacat acgtaatagt atcaagtcat     720
   cacgtcagaa tagattctct ctcagattaa tactcttctg gttttgttga cgacgaggga     780
   aacgaagtct aagtcggtgc tgaagtttca ttgccttctt ctcatgtgac acatgaatat     840
   cactaaaacg tctttacttt gtaaccttct tctttaaata ggatgtttac caatcttcta     900
   gtggattgta cttttcaatg cacttccaaa gagtttgaaa ttttgtgagc cagagagtgt     960
   tacacaagaa aaattgaggt gttagagtcc gttatgagat tgtgatgtgg catctatcca    1020
   ttgatggaag aagatttgct ttgctgacaa ctaacaagat ttggtgttat cacaacaaat    1080
   tggagtcgaa tatatataac gctctaggga aggcccaagt atcagtctaa taactggttc    1140
   aggatccggt ttagagaaaa ccgggagagt gtgacgtgtg gaagtagcac tctggattct    1200
   ttgagtcatc atagctcaaa gtggacaaaa aacaatattt tgtaacttca atattttgta      1260
   acgtatcaaa gaatttcctc ttcgatattt ttacttagag agagagagag atcttctctt      1320
   tcaagttcta gagctttctc cgccgatttc atctccttta gccatggtaa ttaaaaagaa      1380
   aatctatttt cagtctttct tgccttcata tttcacgttt ctctggattc tctgctaatt      1440
   ttggattgtg gtatggcttt tacgttgtct aggaaaatcg atggaaacaa aagaaaaatt      1500
   aaatgcatgc gatatgttag attttgtaat tgtttataat taaaaagaaa ttagcagtag      1560
   tttcaagtaa gtggttaaaa ccaaatagat ttatgtaaaa cttgcatgtg aaattccaac      1620
   ttcaaagttt gttcatgatg atcttgatag cgtggaaaat caagaatttt agctttaaaa      1680
   gtcattggag gaggtgctag tgtgagttgg ttgaatgttg ttaaactgat cacaaagaag      1740
   aatttttgtt ttcattgatt ttattttgta agttagtttg tatttgtagt aaggttggaa      1800
   tagcatgtgg tgaagatgtt gtttagcagt ttaacttccc tggatccata ggaaaaagga      1860
   taattaggcc ttctcttaat gtaaaaactg agactttgaa gttattctca aacgttgttc      1920
   tatcattgga tcatatatcc ttttatgata tgcaatatct cacaatcttt aagctttctt      1980
   gtgttttatt tgttcacata g                                                2001
<210> 8
   <211> 177
   <212> PRT
   <213> Brassica napus
<400> 8
   Met Glu Leu Pro Pro Lys Val Lys Asn Ile Leu Leu Leu Asp Ser Glu
   1               5                   10                  15
   Gly Lys Arg Ile Ala Ala Lys Tyr Tyr Ser Gly Asp Trp Pro Thr Asn
               20                  25                  30
   Thr Ala Lys Glu Ala Phe Glu Lys Ala Val Phe Ser Lys Thr Gln Lys
           35                  40                  45
   Thr Asn Ala Arg Thr Glu Val Glu Val Thr Ala Leu Glu Asn Asn Ile
       50                  55                  60
   Ile Val Tyr Lys Phe Val Gln Asp Leu His Phe Phe Val Thr Gly Gly
   65                  70                  75                  80
   Glu Glu Glu Asn Glu Leu Ile Leu Ala Ser Val Leu Gln Gly Leu Phe
                   85                  90                  95
   Asp Ala Val Asn Leu Leu Leu Arg Gly Asn Ala Asp Lys Arg Glu Ala
               100                 105                 110
   Leu Asp Asn Leu Asp Leu Ile Phe Leu Cys Phe Asp Glu Ile Ile Asp
           115                 120                 125
   Gly Gly Ile Val Leu Glu Thr Asp Ala Asn Val Ile Ala Glu Lys Ala
       130                 135                 140
   Gly Ile Asn Ser Ile Asp Pro Asn Ala Pro Leu Ser Glu Gln Thr Ile
   145                 150                 155                 160
   Ser Gln Ala Leu Ala Thr Ala Arg Glu His Leu Thr Arg Ser Leu Met
                   165                 170                 175
   Lys
<210> 9
   <211> 175
   <212> PRT
   <213> Brassica napus
<400> 9
   Met Glu Leu Pro Pro Lys Val Lys Asn Ile Leu Leu Leu Asp Ser Glu
   1               5                   10                  15
   Gly Lys Arg Ile Ala Ala Lys Tyr Tyr Ser Gly Asp Trp Pro Thr Asn
               20                  25                  30
   Thr Ala Lys Glu Ala Phe Glu Lys Ala Val Phe Ser Lys Thr Gln Lys
           35                  40                  45
   Thr Asn Ala Arg Thr Glu Val Glu Val Thr Ala Leu Glu Asn Asn Ile
       50                  55                  60
   Ile Val Tyr Lys Phe Val Gln Asp Leu His Phe Phe Val Thr Gly Gly
   65                  70                  75                  80
   Glu Glu Glu Asn Glu Leu Val Leu Ala Ser Leu Leu Leu Phe Asp Ala
                   85                  90                  95
   Val Asn Leu Leu Leu Arg Gly Asn Ala Asp Lys Arg Glu Ala Leu Asp
               100                 105                 110
   Asn Leu Asp Leu Ile Phe Leu Cys Phe Asp Glu Ile Ile Asp Gly Gly
           115                 120                 125
   Ile Val Leu Glu Thr Asp Ala Asn Val Ile Ala Glu Lys Ala Gly Ile
       130                 135                 140
   Asn Ser Ile Asp Pro Asn Ala Pro Leu Ser Glu Gln Thr Ile Ser Gln
   145                 150                 155                 160
   Ala Leu Ala Thr Ala Arg Glu His Leu Thr Arg Ser Leu Met Lys
                   165                 170                 175
<210> 10
   <211> 177
   <212> PRT
   <213> Brassica rapa
<400> 10
   Met Glu Leu Pro Pro Lys Val Lys Asn Ile Leu Leu Leu Asp Ser Glu
   1               5                   10                  15
   Gly Lys Arg Ile Ala Ala Lys Tyr Tyr Ser Gly Asp Trp Pro Thr Asn
               20                  25                  30
   Thr Ala Lys Glu Ala Phe Glu Lys Ala Val Phe Ser Lys Thr Gln Lys
           35                  40                  45
   Thr Asn Ala Arg Thr Glu Val Glu Val Thr Ala Leu Glu Asn Asn Ile
       50                  55                  60
   Ile Val Tyr Lys Phe Val Gln Asp Leu His Phe Phe Val Thr Gly Gly
   65                  70                  75                  80
   Glu Glu Glu Asn Glu Leu Val Leu Ala Ser Val Leu Gln Gly Leu Phe
                   85                  90                  95
   Asp Ala Val Asn Leu Leu Leu Arg Gly Asn Val Asp Lys Arg Glu Ala
               100                 105                 110
   Leu Asp Asn Leu Asp Leu Ile Phe Leu Cys Phe Asp Glu Ile Ile Asp
           115                 120                 125
   Gly Gly Ile Val Leu Glu Thr Asp Ala Asn Val Ile Ala Glu Lys Ala
       130                 135                 140
   Gly Ile Asn Ser Ile Asp Pro Asn Ala Pro Leu Ser Glu Gln Thr Ile
   145                 150                 155                 160
   Ser Gln Ala Leu Ala Thr Ala Arg Glu His Leu Thr Arg Ser Leu Met
                   165                 170                 175
   Lys
<210> 11
   <211> 177
   <212> PRT
   <213> Brassica oleracea
<400> 11
   Met Glu Leu Pro Pro Lys Val Lys Asn Ile Leu Leu Leu Asp Ser Glu
   1 5 10 15
   Gly Lys Arg Ile Ala Ala Lys Tyr Tyr Ser Gly Asp Trp Pro Thr Asn
   20 25 30
   Thr Ala Lys Glu Ala Phe Glu Lys Ala Val Phe Ser Lys Thr Gln Lys
   35 40 45
   Thr Asn Ala His Thr Glu Val Glu Val Thr Ala Leu Glu Asn Asn Ile
   50 55 60
   Ile Val Tyr Lys Phe Val Gln Asp Leu His Phe Phe Val Ser Gly Gly
   65 70 75 80
   Glu Glu Glu Asn Glu Leu Val Leu Ala Ser Val Leu Gln Gly Leu Phe
   85 90 95
   Asp Ala Val Asn Leu Leu Leu Arg Gly Asn Ala Asp Lys Arg Glu Ala
   100 105 110
   Leu Asp Asn Leu Asp Leu Ile Phe Leu Cys Phe Asp Glu Ile Ile Asp
   115 120 125
   Gly Gly Ile Val Leu Glu Thr Asp Ala Asn Val Ile Ala Glu Lys Ala
   130 135 140
   Gly Ile Asn Ser Ile Asp Pro Asn Ala Pro Leu Ser Glu Gln Thr Ile
   145 150 155 160
   Ser Gln Ala Leu Ala Thr Ala Arg Glu His Leu Thr Arg Ser Leu Met
   165 170 175
   Lys
<210> 12
   <211> 177
   <212> PRT
   <213> Brassica juncea
<400> 12
   Met Glu Leu Pro Pro Lys Val Lys Asn Ile Leu Leu Leu Asp Ser Glu
   1               5                   10                  15
   Gly Lys Arg Ile Ala Ala Lys Tyr Tyr Ser Gly Asp Trp Pro Thr Asn
               20                  25                  30
   Thr Ala Lys Glu Ala Phe Glu Lys Ala Val Phe Ser Lys Thr Gln Lys
           35                  40                  45
   Thr Asn Ala Arg Thr Glu Val Glu Val Thr Ala Leu Glu Asn Asn Ile
       50                  55                  60
   Ile Val Tyr Lys Phe Val Gln Asp Leu His Phe Phe Val Thr Gly Gly
   65                  70                  75                  80
   Glu Glu Glu Asn Glu Leu Val Leu Ala Ser Val Leu Gln Gly Leu Phe
                   85                  90                  95
   Asp Ala Val Asn Leu Leu Leu Arg Gly Asn Val Asp Lys Arg Glu Ala
               100                 105                 110
   Leu Asp Asn Leu Asp Leu Ile Phe Leu Cys Phe Asp Glu Ile Ile Asp
           115                 120                 125
   Gly Gly Ile Val Leu Glu Thr Asp Ala Asn Val Ile Ala Glu Lys Ala
       130                 135                 140
   Gly Ile Asn Ser Ile Asp Pro Asn Ala Pro Leu Ser Glu Gln Thr Ile
   145                 150                 155                 160
   Ser Gln Ala Leu Ala Thr Ala Arg Glu His Leu Thr Arg Ser Leu Met
                   165                 170                 175
   Lys
<210> 13
   <211> 534
   <212> DNA
   <213> Brassica napus
<400> 13
   atggagttac ccccaaaagt gaagaacatt ctgcttcttg attctgaagg aaagcgcata      60
   gccgctaagt actactctgg cgactggcca acaaatacgg ctaaagaagc attcgaaaaa     120
   gctgtcttct caaagactca aaaaacaaac gctcgcaccg aagttgaagt gacggcactg     180
   gagaacaaca tcattgtgta caaatttgtg caagatcttc atttctttgt tacgggaggt     240
   gaagaagaaa acgagcttat cttagccagt gtgcttcagg gtctcttcga tgcagtgaat      300
   ctcctcctca gaggcaatgc tgataagaga gaggcattgg acaatcttga tctcatcttt      360
   ctatgctttg acgaaatcat cgatggcggt attgtcctgg agacggatgc aaatgtgata      420
   gcagaaaagg caggaatcaa tagcattgac cctaacgctc ctctatccga gcagacgata      480
   agtcaggcac tggcgacggc aagagagcat ttgacaaggt cacttatgaa atga            534
<210> 14
   <211> 528
   <212> DNA
   <213> Brassica napus
<400> 14
   atggagttac ccccaaaagt gaagaacatt ctgcttcttg attctgaagg aaagcgcata       60
   gccgctaagt actactctgg cgactggcca acaaatacgg ctaaagaagc attcgaaaaa      120
   gctgtcttct caaagactca gaaaacaaac gctcgcaccg aagttgaagt gacggcactg      180
   gagaacaaca tcattgtgta caaatttgtg caagatcttc atttctttgt tacgggaggt      240
   gaagaagaaa acgagcttgt cttagccagt ttgcttctct tcgatgcagt gaatctcctc      300
   ctcagaggca atgctgataa gagagaggca ttggacaatc ttgatctcat ctttctatgc      360
   tttgacgaaa tcatcgatgg cggtattgtc ctggagacgg atgcaaatgt gatagcagaa      420
   aaggcaggaa tcaatagcat tgaccctaac gctcctctat ccgagcagac gataagtcag      480
   gcactggcaa cggcaagaga gcatttgaca aggtcactta tgaaatga                   528
<210> 15
   <211> 534
   <212> DNA
   <213> Brassica rapa
<400> 15
   atggagttac ccccaaaagt gaagaacatt ctgcttcttg attctgaagg aaagcgcata 60
   gccgctaagt actactctgg cgactggcca acaaatacgg ctaaagaagc attcgaaaaa 120
   gctgtcttct caaagactca aaaaacaaac gctcgcaccg aagttgaagt gacggcactg 180
   gagaacaaca tcattgtgta caaatttgtg caagatcttc atttctttgt tacgggaggt 240
   gaagaagaaa acgagcttgt cttagccagt gtgcttcagg gtctcttcga tgcagtgaat 300
   ctcctcctca gaggcaatgt tgataagaga gaggcattgg acaatcttga tctcatcttt 360
   ctatgctttg acgaaatcat cgatggcggt attgtcctgg agacggatgc aaatgtgata 420
   gcagaaaagg caggaatcaa tagcattgac cctaacgctc ctctatccga gcagacgata 480
   agtcaggcac tggcgacggc aagagagcat ttgacaaggt cacttatgaa atga 534
<210> 16
   <211> 534
   <212> DNA
   <213> Brassica oleracea
<400> 16
   atggagttac ccccaaaagt gaagaacatt ctgcttcttg attctgaagg aaagcgcata 60
   gccgctaagt actactctgg cgactggcca acaaatacgg ctaaagaagc attcgaaaaa 120
   gctgtcttct caaagactca gaaaacaaac gctcacaccg aagttgaagt gacggcactg 180
   gagaacaaca tcattgtgta caaatttgtg caagatcttc atttctttgt ttcgggaggt 240
   gaagaagaaa acgagcttgt cttagccagt gtgcttcagg gtctcttcga tgcagtgaat 300
   ctcctcctca gaggcaatgc tgataagaga gaggcattgg acaatcttga tctcatcttt 360
   ctatgctttg acgaaatcat tgatggcggt attgtcctgg agacggatgc aaatgtgata 420
   gcagaaaagg caggaatcaa tagcattgac cctaacgctc ctctatccga gcagacgata 480
   agtcaggcac tggcaacggc aagagagcat ttgacaaggt cacttatgaa atga 534
<210> 17
   <211> 534
   <212> DNA
   <213> Brassica juncea
<400> 17
   atggagttac ccccaaaagt gaagaacatt ctgcttcttg attctgaagg aaagcgcata 60
   gccgctaagt actactctgg cgactggcca acaaatacgg ctaaagaagc attcgaaaaa 120
   gctgtcttct caaagactca aaaaacaaac gctcgcaccg aagttgaagt gacggcactg 180
   gagaacaaca tcattgtgta caaatttgtg caagatcttc atttctttgt tacgggaggt 240
   gaagaagaaa acgagcttgt cttagccagt gtgcttcagg gtctcttcga tgcagtgaat 300
   ctcctcctca gaggcaatgt tgataagaga gaggcattgg acaatcttga tctcatcttt 360
   ctatgctttg acgaaatcat cgatggcggt attgtcctgg agacggatgc aaatgtgata 420
   gcagaaaagg caggaatcaa tagcattgac cctaacgctc ctctatccga gcagacgata 480
   agtcaggcac tggcgacggc aagagagcat ttgacaaggt cacttatgaa atga 534
<210> 18
   <211> 143
   <212> DNA
   <213> Artificial Sequence
<220>
   <223> Promoter consensus sequence
<400> 18
   gytagagtcc gttatgagay tgtgatgtgg crtctatccr ttgrtggaag aakatttgct 60
   ttgctgacaa ctaacaagat tyggtgttat cacaacaaat tggagtcgrm tatatataac 120
   gctctagrga aggcycaagt atc 143
<210> 19
   <211> 123
   <212> DNA
   <213> Artificial Sequence
<220>
   <223> UTR consensus sequence 1
<400> 19
   agtctaataa ctggttcagg atccggttta gagaaaaccg rgagagtgtg acgtgtgraa 60
   gtagcrctct ggattctttg agtcatcata gctyaaagtg gacaaaaaac aatattttgt 120
   aac 123
<210> 20
   <211> 34
   <212> DNA
   <213> Artificial Sequence
<220>
   <223> UTR consensus sequence 2
<400> 20
   gtatcaaaga atttcctctt cgatattttt actt 34
<210> 21
   <211> 65
   <212> DNA
   <213> Artificial Sequence
<220>
   <223> UTR consensus sequence 3
<400> 21
   agagagagag agatcttcts tttcaagttc tagagctttc tccgccgatt tcatctcctt 60
   tagcc 65
<210> 22
   <211> 11
   <212> DNA
   <213> Artificial Sequence
<220>
   <223> Intron consensus sequence 1
<400> 22
   gtaattaaaa a 11
<210> 23
   <211> 253
   <212> DNA
   <213> Artificial Sequence
<220>
   <223> Intron consensus sequence 2
<400> 23
   ctrttttcag tctttcttgc cttcatwttt cacgtytctc tggattctct gctaattttg 60
   gattgtggta tggcttttac gttstctagg aaaatcgatg gaaacaaaag aaaamtkaaa 120
   tgcatgckat atgttagatt ttgtaattgt ttawaattaa waaraaatta gcagtagttt 180
   caagtaagtg gttaaaacca aatagattta tgtaaaactt gcatgtgaaa ttccaactty 240
   aaagtttgtt cat 253
<210> 24
   <211> 11
   <212> DNA
   <213> Artificial Sequence
<220>
   <223> Intron consensus sequence 3
<400> 24
   atcttgatag c 11